# EUROPEAN PATENT SPECIFICATION

(11) **EP 3 353 552 B1**
(45) Date of publication and mention of the grant of the patent: **01.01.2020**
(21) Application number: 16774639.5
(22) Date of filing: 22.09.2016
(51) Int. Cl.: G01N 33/574

(54) **METHOD AND ARRAY FOR DIAGNOSING PANCREATIC CANCER IN AN INDIVIDUAL**
VERFAHREN, ANORDNUNG UND VERWENDUNG DAVON
MÉTHODE, ENSEMBLE ET LEUR UTILISATION

(30) Priority: 22.09.2015 GB 201516801
(43) Date of publication of application: 01.08.2018
(73) Proprietor: Immunovia AB, 223 81 Lund (SE)
(72) Inventor: BORREBAECK, Carl Arne Krister, S-223 63 Lund (SE); WINGREN, Christer Lars Bertil, SE-247 32 Södra (SE)
(74) Representative: Potter Clarkson
(86) International application number: PCT/EP2016/072617
(87) International publication number: WO 2017/050939

(56) References cited:
- WO-A1-2014/141683
- WO-A2-2008/117067
- WO-A2-2012/120288
- WO-A2-2015/067969
- DATABASE WPI Week 201518 Thomson Scientific, London, GB; AN 2015-17722Q XP002765169, -& JP 2015 033381 A (UNIV TOKYO) 19 February 2015 (2015-02-19)
- Qing-Cai Meng ET AL: "Original Article Overexpression of NDC80 is correlated with prognosis of pancreatic cancer and regulates cell proliferation", , 1 May 2015 (2015-05-01), XP055328778, Retrieved from the Internet: URL:https://www.ncbi.nlm.nih.gov/pmc/artic les/PMC4497439/pdf/ajcr0005-1730.pdf [retrieved on 2016-12-13]

### SEQUENCE LISTING

<110> Immunovia AB
<120> METHODS AND ARRAYS FOR USE IN THE SAME
<140>
   <141>
<150> GB1516801.6
   <151> 2015-09-22
<160> 2
<170> SeqWin99
<210> 1
   <211> 242
   <212> PRT
   <213> Homo sapiens
<400> 1
   Met Ala Glu Val Gln Leu Leu Glu Ser Gly Gly Gly Leu Val Gln Pro
   1               5                   10                  15
   Gly Gly Ser Leu Arg Leu Ser Cys Ala Ala Ser Gly Phe Thr Phe Ser
               20                  25                  30
   Ser Tyr Gly Phe His Trp Val Arg Gln Ala Pro Gly Lys Gly Leu Glu
           35                  40                  45
   Trp Val Ser Leu Ile Ser Trp Asp Gly Gly Ser Thr Tyr Tyr Ala Asp
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Gly Thr Trp Phe Asp Pro Trp Gly Gln Gly Thr Leu
               100                 105                 110
   Val Thr Val Ser Ser Gly Gly Gly Gly Ser Gly Gly Gly Gly Ser Gly
           115                 120                 125
   Gly Gly Gly Ser Gln Ser Val Leu Thr Gln Pro Pro Ser Ala Ser Gly
       130                 135                 140
   Thr Pro Gly Gln Arg Val Thr Ile Ser Cys Ser Gly Ser Ser Ser Asn
   145                 150                 155                 160
   Ile Gly Asn Asn Ala Val Asn Trp Tyr Gln Gln Leu Pro Gly Thr Ala
                   165                 170                 175
   Pro Lys Leu Leu Ile Tyr Arg Asn Asn Gln Arg Pro Ser Gly Val Pro
               180                 185                 190
   Asp Arg Phe Ser Gly Ser Lys Ser Gly Thr Ser Ala Ser Leu Ala Ile
           195                 200                 205
   Ser Gly Leu Arg Ser Glu Asp Glu Ala Asp Tyr Tyr Cys Ala Ala Trp
       210                 215                 220
   Asp Asp Ser Leu Ser Trp Val Phe Gly Gly Gly Thr Lys Leu Thr Val
   225                 230                 235                 240
   Leu Gly
<210> 2
   <211> 31
   <212> PRT
   <213> Artificial Sequence
<220>
   <223> 6 x histidine affinity tag
<400> 2
   Asp Tyr Lys Asp His Asp Gly Asp Tyr Lys Asp His Asp Ile Asp Tyr
   1               5                   10                  15
   Lys Asp Asp Asp Asp Lys Ala Ala Ala His His His His His His
               20                  25                  30

## Description

### Field of Invention

The present invention relates to methods for detecting pancreatic cancer, and biomarkers and arrays for use in the same.

### Background

Pancreatic ductal adenocarcinoma (PDAC) is the 4th most common cancer-related cause of death (Siegel *et al,* 2012). Multiple factors account for its poor prognosis and early diagnosis provides today the only possibility for cure. PDAC is often detected at late stages with 80% of patients not eligible for surgery due to either locally advanced or metastatic disease (Hidalgo, 2010; Porta *et al*, 2005; Siegel *et al*, 2012).

The biological diversity of tumours due to its localization in pancreatic cancer has been previously demonstrated. Tumours in the body/tail of pancreas are rarer than tumour in the head of pancreas (77% of PDAC). Because of differences in e.g., blood supply, and lymphatic and venous backflow, there are also differences in the disease presentation with body/tail tumours causing less jaundice, more pain, higher albumin and CEA levels and lower CA19-9 levels.

Body/tail tumours are more often detected at a later stage than head tumours and have a higher rate of metastasis. As the biological differences can result in different treatment efficiency, biomarkers that can discriminate between tumour localization would be of clinical relevance and could pave the way for personalized treatment strategies. However, few differences have been found on a genetic level, with no significant variation in the overall number of mutations, deletions and amplifications, or in K-ras point mutations.

Accordingly, there is a continuing need to provide methods for determining biomarkers that can determine the locality and/or presence of pancreatic cancer tumours.

JP 2015 033381 describes a molecular marker for detecting a cancer stem cell from a subject by expression of a PRDM14 gene, the molecular marker preferably including a PRDM14 gene product or fragments thereof, and/or humoral factors induced by expression of the PRDM14 gene. WO 2015/067969 describes a method for determining the presence of pancreatic cancer in an individual comprising or consisting of the steps of: (a) providing a sample to be tested from the individual, and (b) determining a biomarker signature of the test sample by measuring the expression in the test sample of one or more selected biomarkers, wherein the expression in the test sample of one or more selected biomarkers is indicative of the individual having pancreatic cancer, as well as arrays and kits of parts for use in the method. Meng QC et al. (2015) American Journal of Cancer Research 5(5):1730-1740 describes overexpression of NDC80 being correlated with prognosis of pancreatic cancer and regulating cell proliferation.

### Summary of the Invention

A major problem with tumours of the body/tail in comparison with pancreatic head cancer is distant metastasis, especially in the liver, and resection of the tumour does not increase postoperative survival in metastatic disease. On the other hand, patients with local-stage body/tail tumours had higher survival rates compared with local-stage pancreatic head cancer.

Several antibodies identified markers that showed on differential protein expression levels between head and body/tail tumours. A condensed signatures differentiating the groups could be defined. Consequently, these results are encouraging for a future development of a blood protein biomarker signature discriminating body/tail and head tumours at an early disease stage.

Taken together, we provide information that serum protein markers associated with different tumour locations in the pancreas could be identified. Serum protein markers associated with tumour localization were identified.

The invention is defined by the claims.

A first aspect of the invention provides a method for determining the location of a tumour in the pancreas of an individual with pancreatic cancer, the method comprising or consisting of the steps of:
a) providing a sample to be tested from the individual;
b) determining a biomarker signature of the test sample by measuring the expression in the test sample of two or more biomarkers selected from the group defined in Table A (i), (ii) or (iii);
wherein the two or more biomarkers selected from the group defined in Table A (i), (ii) or (iii) include PR domain zinc finger protein 14 (PRD14) and kinetochore protein NDC80 homolog (HsHec1); and
wherein the expression in the test sample of the two or more biomarkers selected from the group defined in Table A (i), (ii) or (iii) is indicative of the location of the tumour in the pancreas of the individual.

By "sample to be tested", "test sample" or "control sample" we include a tissue or fluid sample taken or derived from an individual. Preferably the sample to be tested is provided from a mammal. The mammal may be any domestic or farm animal. Preferably, the mammal is a rat, mouse, guinea pig, cat, dog, horse or a primate. Most preferably, the mammal is human. Preferably the sample is a cell or tissue sample (or derivative thereof) comprising or consisting of plasma, plasma cells, serum, tissue cells or equally preferred, protein or nucleic acid derived from a cell or tissue sample. Preferably test and control samples are derived from the same species.

In an alternative or additional embodiment the tissue sample is pancreatic tissue. In an alternative or additional embodiment, the cell sample is a sample of pancreatic cells.

By "expression" we mean the level or amount (relative and/or absolute) of a gene product such as ctDNA (circulating DNA), mRNA or protein. Expression may be used to define clusters associated with disease states of interest. Alternatively or additionally, "expression" excludes the measurement of ctDNA.

Methods of detecting and/or measuring the concentration of protein and/or nucleic acid are well known to those skilled in the art, see for example Sambrook and Russell, 2001, Cold Spring Harbor Laboratory Press.

By "biomarker" we mean a naturally-occurring biological molecule, or component or fragment thereof, the measurement of which can provide information useful in determining the locality and/or presence of pancreatic cancer. For example, the biomarker may be a naturally-occurring nucleic acid, protein or carbohydrate moiety, or an antigenic component or fragment thereof.

By *'determining the locality of pancreatic cancer,' 'indicative of the pancreatic cancer locality'* and the like we include determining (or providing indication of) whether the pancreatic cancer is located in and/or originated from (a) the head of the pancreas; or (b) the body and/or tail of the pancreas.

The terms 'pancreas head, 'pancreas neck,' 'pancreas body' and 'pancreas tail' are well-known and understood by the skilled person. Hence, by 'the head of the pancreas,' 'the neck of the pancreas,' 'the body of the pancreas' and 'the tail of the pancreas' we include the conventional understanding of the terms by the skilled person.

Alternatively or additionally, by 'the head of the pancreas' we mean or include foundational model of anatomy identification number (FMAID) 10468 (for more information on the FMA and FMAIDs, see Rosse & Cornelius, 2003, 'A reference ontology for biomedical informatics: the Foundational Model of Anatomy,' J. Biomed. Informatics, 36(6): 478-500 and the FMA browser, accessible at http://xiphoid.biostr.washington.edu/fma/index.html). Synonyms for 'the head of the pancreas' include 'right extremity of pancreas,' pancreatic head' and *'caput pancreatis'.*

Alternatively or additionally, by 'the neck of the pancreas' we mean or include FMAID 14517. Synonyms for 'the neck of the pancreas' include 'pancreatic neck' and *'collum pancreatis'.*

Alternatively or additionally, by 'the body of the pancreas' we mean or include FMAID 14518. Synonyms for 'the body of the pancreas' include 'pancreatic body' and *'corpus pancreatis'.*

Alternatively or additionally, FMAID numbers comprise or consist of the FMA definitions current on 21 September 2015.

Alternatively or additionally, by 'the head of the pancreas' we include the head and/or neck of the pancreas. Hence, alternatively or additionally, by *'determining the locality of pancreatic cancer,' 'indicative of the pancreatic cancer locality'* and the like we include determining (or providing indication of) whether the pancreatic cancer is located in and/or originated from (a) the head and/or neck of the pancreas; or (b) the body and/or tail of the pancreas.

Alternatively or additionally, by 'the body/tail of the pancreas' we include the neck, body and/or tail of the pancreas. Hence, alternatively or additionally, by *'determining the locality of pancreatic cancer,' 'indicative of the pancreatic cancer locality'* and the like we include determining (or providing indication of) whether the pancreatic cancer is located in and/or originated from (a) the head of the pancreas; or (b) the neck, body and/or tail of the pancreas.

By *'located in the head and*/*or neck of the pancreas*'we include that at least greater than 50% of the tumour is located in the head and/or neck of the pancreas, for example, ≥51%, ≥52%, ≥53%, ≥54%, ≥55%, ≥56%, ≥57%, ≥58%, ≥59%, ≥60%, ≥61%, ≥62%, ≥63%, ≥64%, ≥65%, ≥66%, ≥67%, ≥68%, ≥69%, ≥70%, ≥71%, ≥72%, ≥73%, ≥74%, ≥75%, ≥76%, ≥77%, ≥78%, ≥79%, ≥80%, ≥81%, ≥82%, ≥83%, ≥84%, ≥85%, ≥86%, ≥87%, ≥88%, ≥89%, ≥90%, ≥91%, ≥92%, ≥93%, ≥94%, ≥95%, ≥96%, ≥97%, ≥98%, ≥99% or 100% of the tumour is located in the head and/or neck of the pancreas.

By *'originated from the head and*/*or neck of the pancreas'we* include that the pancreatic cancer comprises or consists of pancreatic cancer that is located outside of the head and/or neck of the pancreas but originated from a primary tumour located in head and/or neck of the pancreas. Thus, metastases of pancreatic cancer from a primary tumour located in the head of the pancreas may be included.

By *'located in the neck, body and*/*or tail of the pancreas'* we include that at least greater than 50% of the tumour is located in the neck, body and/or tail of the pancreas, for example, ≥51%, ≥52%, ≥53%, ≥54%, ≥55%, ≥56%, ≥57%, ≥58%, ≥59%, ≥60%, ≥61%, ≥62%, ≥63%, ≥64%, ≥65%, ≥66%, ≥67%, ≥68%, ≥69%, ≥70%, ≥71%, ≥72%, ≥73%, ≥74%, ≥75%, ≥76%, ≥77%, ≥78%, ≥79%, ≥80%, ≥81%, ≥82%, ≥83%, ≥84%, ≥85%, ≥86%, ≥87%, ≥88%, ≥89%, ≥90%, ≥91%, ≥92%, ≥93%, ≥94%, ≥95%, ≥96%, ≥97%, ≥98%, ≥99% or 100% of the tumour is located in the neck, body and/or tail of the pancreas.

By *'originated from the neck, body and*/*or tail of the pancreas'* we include that the pancreatic cancer comprises or consists of pancreatic cancer that is located outside of the neck, body and/or tail of the pancreas but originated from a primary tumour located in the neck, body and/or tail of the pancreas. Thus, metastases of pancreatic cancer from a primary tumour located in the neck, body and/or tail of the pancreas may be included.

Alternatively or additionally the individual is determined to be afflicted with pancreatic cancer. The individual afflicted with pancreatic cancer may diagnosed as having pancreatic cancer prior to step (a), during step (a) and/or following step (a).

The pancreatic cancer may be diagnosed using one or more biomarkers of the present invention (i.e., concurrent diagnosis and locality determination using the same or different biomarkers of the invention for each).

Alternatively or additionally the pancreatic cancer may be diagnosed using conventional clinical methods known in the art. For example, those methods described in Ducreux et al., 2015, 'Cancer of the pancreas: ESMO Clinical Practice Guidelines for diagnosis, treatment and follow-up' Annals of Oncology, 26 (Supplement 5): v56-v68 and/or Freelove & Walling, 2006, 'Pancreatic Cancer: Diagnosis and Management' American Family Physician, 73(3):485-492.

Accordingly, the pancreatic cancer may be diagnosed using one or more method selected from the group consisting of:
(i) computed tomography (preferably dual-phase helical computed tomography);
(ii) transabdominal ultrasonography;
(iii) endoscopic ultrasonographyguided fine-needle aspiration;
(iv) endoscopic retrograde cholangiopancreatography;
(v) positron emission tomography;
(vi) magnetic resonance imaging;
(vii) physical examination; and
(viii) biopsy.

Alternatively and/or additionally, the pancreatic cancer may be diagnosed using detection of biomarkers for the diagnosis of pancreatic cancer. For example, the pancreatic cancer may be diagnosed with one or more biomarker or diagnostic method described in the group consisting of:
(i) WO 2008/117067 A9;
(ii) WO 2012/120288 A2; and
(iii) WO 2015/067969 A2.

Alternatively or additionally the method further comprises or consists of the steps of:
c) providing a control sample from an individual not afflicted with pancreatic cancer;
d) determining a biomarker signature of the control sample by measuring the expression in the control sample of the two or more biomarkers measured in step (b);
wherein the locality and/or presence of pancreatic cancer is identified in the event that the expression in the test sample of the two or more biomarkers measured in step (b) is different from the expression in the control sample of the two or more biomarkers measured in step (d).

By "is different to the presence and/or amount in a control sample" we mean the presence and/or amount of the two or more biomarkers in the test sample differs from that of the one or more control sample (or to predefined reference values representing the same). Preferably the presence and/or amount is no more than 40% of that of the one or more negative control sample, for example, no more than 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1% or 0%.

In an alternative or additional embodiment the presence and/or amount in the test sample of the two or more biomarker measured in step (b) is significantly different (i.e., statistically significantly different) from the presence and/or amount of the two or more biomarker measured in step (d) or the predetermined reference values. For example, as discussed in the accompanying Examples, significant difference between the presence and/or amount of a particular biomarker in the test and control samples may be classified as those where p<0.05 (for example, where p<0.04, p<0.03, p<0.02 or where p<0.01).

The one or more control sample may be from a healthy individual (i.e., an individual unaffiliated by any disease or condition), an individual afflicted with a non-pancreatic disease or condition or an individual afflicted with a benign pancreatic disease or condition (for example, acute or chronic pancreatitis).

Alternatively or additionally the method further comprises or consists of the steps of:
e) providing a control sample from an individual afflicted with pancreatic cancer;
f) determining a biomarker signature of the control sample by measuring the expression in the control sample of the two or more biomarkers measured in step (b);
wherein the locality and/or presence of pancreatic cancer is identified in the event that the expression in the test sample of the two or more biomarkers measured in step (b) corresponds to the expression in the control sample of the two or more biomarkers measured in step (f).

By "corresponds to the expression in the control sample" we include that the expression of the one or more biomarkers in the sample to be tested is the same as or similar to the expression of the one or more biomarkers of the positive control sample. Preferably the expression of the one or more biomarkers in the sample to be tested is identical to the expression of the one or more biomarkers of the positive control sample.

Differential expression (up-regulation or down regulation) of biomarkers, or lack thereof, can be determined by any suitable means known to a skilled person. Differential expression is determined to a p-value of a least less than 0.05 (p = < 0.05), for example, at least <0.04, <0.03, <0.02, <0.01, <0.009, <0.005, <0.001, <0.0001, <0.00001 or at least <0.000001. Preferably, differential expression is determined using a support vector machine (SVM). Preferably, the SVM is an SVM as described below.

It will be appreciated by persons skilled in the art that differential expression may relate to a single biomarker or to multiple biomarkers considered in combination (i.e., as a biomarker *signature).* Thus, a p-value may be associated with a single biomarker or with a group of biomarkers. Indeed, proteins having a differential expression p-value of greater than 0.05 when considered individually may nevertheless still be useful as biomarkers in accordance with the invention when their expression levels are considered in combination with one or more other biomarkers.

As exemplified in the accompanying examples, the expression of certain proteins in a tissue, blood, serum or plasma test sample may be indicative of pancreatic cancer disease state in an individual (e.g., locality and/or presence). For example, the relative expression of certain serum proteins in a single test sample may be indicative of the locality and/or presence of pancreatic cancer in an individual.

When referring to a "normal" disease state we include individuals not afflicted with chronic pancreatitis (ChP) or acute inflammatory pancreatitis (AIP). Preferably the individuals are not afflicted with any pancreatic disease or disorder. Most preferably, the individuals are healthy individuals, i.e., they are not afflicted with any disease or disorder.

Alternatively or additionally the method further comprises or consists of the steps of:
g) providing a control sample from an individual afflicted with pancreatic cancer located in and/or originating from the head (and/or neck) of the pancreas; and
h) determining a biomarker signature of the control sample by measuring the expression in the control sample of the two or more biomarkers measured in step (b);
wherein the location of pancreatic cancer in the test sample is identified as being located in and/or originating from head (and/or neck) of the pancreas in the event that the expression in the test sample of the two or more biomarkers measured in step (b) corresponds to the expression in the control sample of the two or more biomarkers measured in step (h); and
wherein the location of pancreatic cancer in the test sample is identified as being located in and/or originating from the body and/or tail of the pancreas in the event that the expression in the test sample of the two or more biomarkers measured in step (b) is different from the expression in the control sample of the two or more biomarkers measured in step (h).

Alternatively or additionally the method further comprises or consists of the steps of:
i) providing a control sample from an individual afflicted with pancreatic cancer located in and/or originating from the (neck), body and/or tail of the pancreas; and
j) determining a biomarker signature of the control sample by measuring the expression in the control sample of the two or more biomarkers measured in step (b);
wherein the location of pancreatic cancer in the test sample is identified as being located in and/or originating from the (neck), body and/or tail of the pancreas in the event that the expression in the test sample of the two or more biomarkers measured in step (b) corresponds to the expression in the control sample of the two or more biomarkers measured in step (j); and
wherein the location of pancreatic cancer in the test sample is identified as being located in and/or originating from the head of the pancreas in the event that the expression in the test sample of the two or more biomarkers measured in step (b) is different from the expression in the control sample of the two or more biomarkers measured in step (j).

Alternatively or additionally step (b) comprises or consists of measuring the expression of two or more of the biomarkers listed in Table A, for example, at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123 or 124 of the biomarkers listed in Table A.

Step (b) comprises measuring the expression of PRD14. Step (b) comprises measuring the expression of HsHec1. In an alternative or additional embodiment, step (b) comprises or excludes measuring the expression of hSpindly. In an alternative or additional embodiment, step (b) comprises or excludes measuring the expression of GNAI3. In an alternative or additional embodiment, step (b) comprises or excludes measuring the expression of GRIP-2. In an alternative or additional embodiment, step (b) comprises or excludes measuring the expression of HsMAD2. In an alternative or additional embodiment, step (b) comprises or excludes measuring the expression of TBC1D9. In an alternative or additional embodiment, step (b) comprises or excludes measuring the expression of MAPKK6. In an alternative or additional embodiment, step (b) comprises or excludes measuring the expression of MAPK9. In an alternative or additional embodiment, step (b) comprises or excludes measuring the expression of MAPK8. In an alternative or additional embodiment, step (b) comprises or excludes measuring the expression of ORP-3. In an alternative or additional embodiment, step (b) comprises or excludes measuring the expression of MUC1. In an alternative or additional embodiment, step (b) comprises or excludes measuring the expression of PTK6. In an alternative or additional embodiment, step (b) comprises or excludes measuring the expression of PTPN1. In an alternative or additional embodiment, step (b) comprises or excludes measuring the expression of R-PTP-eta. In an alternative or additional embodiment, step (b) comprises or excludes measuring the expression of R-PTP-O. In an alternative or additional embodiment, step (b) comprises or excludes measuring the expression of PGAM5. In an alternative or additional embodiment, step (b) comprises or excludes measuring the expression of STAT1. In an alternative or additional embodiment, step (b) comprises or excludes measuring the expression of EGFR. In an alternative or additional embodiment, step (b) comprises or excludes measuring the expression of Surface Ag X. In an alternative or additional embodiment, step (b) comprises or excludes measuring the expression of CIMS (1). In an alternative or additional embodiment, step (b) comprises or excludes measuring the expression of CIMS (11). In an alternative or additional embodiment, step (b) comprises or excludes measuring the expression of CIMS (12). In an alternative or additional embodiment, step (b) comprises or excludes measuring the expression of CIMS (13). In an alternative or additional embodiment, step (b) comprises or excludes measuring the expression of CIMS (14). In an alternative or additional embodiment, step (b) comprises or excludes measuring the expression of CIMS (15). In an alternative or additional embodiment, step (b) comprises or excludes measuring the expression of CIMS (16). In an alternative or additional embodiment, step (b) comprises or excludes measuring the expression of CIMS (17). In an alternative or additional embodiment, step (b) comprises or excludes measuring the expression of CIMS (18). In an alternative or additional embodiment, step (b) comprises or excludes measuring the expression of CIMS (2). In an alternative or additional embodiment, step (b) comprises or excludes measuring the expression of CIMS (20). In an alternative or additional embodiment, step (b) comprises or excludes measuring the expression of CIMS (21). In an alternative or additional embodiment, step (b) comprises or excludes measuring the expression of CIMS (22). In an alternative or additional embodiment, step (b) comprises or excludes measuring the expression of CIMS (23). In an alternative or additional embodiment, step (b) comprises or excludes measuring the expression of CIMS (24). In an alternative or additional embodiment, step (b) comprises or excludes measuring the expression of CIMS (25). In an alternative or additional embodiment, step (b) comprises or excludes measuring the expression of CIMS (26). In an alternative or additional embodiment, step (b) comprises or excludes measuring the expression of CIMS (27). In an alternative or additional embodiment, step (b) comprises or excludes measuring the expression of CIMS (28). In an alternative or additional embodiment, step (b) comprises or excludes measuring the expression of CIMS (29). In an alternative or additional embodiment, step (b) comprises or excludes measuring the expression of CIMS (3). In an alternative or additional embodiment, step (b) comprises or excludes measuring the expression of CIMS (30). In an alternative or additional embodiment, step (b) comprises or excludes measuring the expression of CIMS (31). In an alternative or additional embodiment, step (b) comprises or excludes measuring the expression of CIMS (4). In an alternative or additional embodiment, step (b) comprises or excludes measuring the expression of CIMS (5). In an alternative or additional embodiment, step (b) comprises or excludes measuring the expression of CIMS (6). In an alternative or additional embodiment, step (b) comprises or excludes measuring the expression of CIMS (7). In an alternative or additional embodiment, step (b) comprises or excludes measuring the expression of CIMS (9). In an alternative or additional embodiment, step (b) comprises or excludes measuring the expression of Apo-A1. In an alternative or additional embodiment, step (b) comprises or excludes measuring the expression of Apo-A4. In an alternative or additional embodiment, step (b) comprises or excludes measuring the expression of ATP-5B. In an alternative or additional embodiment, step (b) comprises or excludes measuring the expression of B-galactosidase. In an alternative or additional embodiment, step (b) comprises or excludes measuring the expression of BTK. In an alternative or additional embodiment, step (b) comprises or excludes measuring the expression of C1 inh. In an alternative or additional embodiment, step (b) comprises or excludes measuring the expression of C1s. In an alternative or additional embodiment, step (b) comprises or excludes measuring the expression of C3. In an alternative or additional embodiment, step (b) comprises or excludes measuring the expression of C4. In an alternative or additional embodiment, step (b) comprises or excludes measuring the expression of C5. In an alternative or additional embodiment, step (b) comprises or excludes measuring the expression of CD40. In an alternative or additional embodiment, step (b) comprises or excludes measuring the expression of CDK-2. In an alternative or additional embodiment, step (b) comprises or excludes measuring the expression of Cystatin C. In an alternative or additional embodiment, step (b) comprises or excludes measuring the expression of Eotaxin. In an alternative or additional embodiment, step (b) comprises or excludes measuring the expression of Factor B. In an alternative or additional embodiment, step (b) comprises or excludes measuring the expression of FASN. In an alternative or additional embodiment, step (b) comprises or excludes measuring the expression of GAK. In an alternative or additional embodiment, step (b) comprises or excludes measuring the expression of GLP-1 R. In an alternative or additional embodiment, step (b) comprises or excludes measuring the expression of GM-CSF. In an alternative or additional embodiment, step (b) comprises or excludes measuring the expression of Her2/ErbB2. In an alternative or additional embodiment, step (b) comprises or excludes measuring the expression of ICAM-1. In an alternative or additional embodiment, step (b) comprises or excludes measuring the expression of IFN-γ. In an alternative or additional embodiment, step (b) comprises or excludes measuring the expression of IL-10. In an alternative or additional embodiment, step (b) comprises or excludes measuring the expression of IL-13. In an alternative or additional embodiment, step (b) comprises or excludes measuring the expression of IL-1β. In an alternative or additional embodiment, step (b) comprises or excludes measuring the expression of IL-5. In an alternative or additional embodiment, step (b) comprises or excludes measuring the expression of IL-8. In an alternative or additional embodiment, step (b) comprises or excludes measuring the expression of Integrin a-10. In an alternative or additional embodiment, step (b) comprises or excludes measuring the expression of Integrin a-11. In an alternative or additional embodiment, step (b) comprises or excludes measuring the expression of JAK3. In an alternative or additional embodiment, step (b) comprises or excludes measuring the expression of KSYK. In an alternative or additional embodiment, step (b) comprises or excludes measuring the expression of LDL. In an alternative or additional embodiment, step (b) comprises or excludes measuring the expression of Leptin. In an alternative or additional embodiment, step (b) comprises or excludes measuring the expression of MAPK1. In an alternative or additional embodiment, step (b) comprises or excludes measuring the expression of MCP-3. In an alternative or additional embodiment, step (b) comprises or excludes measuring the expression of MCP-4. In an alternative or additional embodiment, step (b) comprises or excludes measuring the expression of MYOM2. In an alternative or additional embodiment, step (b) comprises or excludes measuring the expression of ORP-3. In an alternative or additional embodiment, step (b) comprises or excludes measuring the expression of Osteopontin. In an alternative or additional embodiment, step (b) comprises or excludes measuring the expression of P85A. In an alternative or additional embodiment, step (b) comprises or excludes measuring the expression of Procathepsin W. In an alternative or additional embodiment, step (b) comprises or excludes measuring the expression of Properdin. In an alternative or additional embodiment, step (b) comprises or excludes measuring the expression of PSA. In an alternative or additional embodiment, step (b) comprises or excludes measuring the expression of RPS6KA2. In an alternative or additional embodiment, step (b) comprises or excludes measuring the expression of Sialyl Lewis X. In an alternative or additional embodiment, step (b) comprises or excludes measuring the expression of STAP2. In an alternative or additional embodiment, step (b) comprises or excludes measuring the expression of TM peptide. In an alternative or additional embodiment, step (b) comprises or excludes measuring the expression of TNF-a. In an alternative or additional embodiment, step (b) comprises or excludes measuring the expression of UCHL5. In an alternative or additional embodiment, step (b) comprises or excludes measuring the expression of UPF3B. In an alternative or additional embodiment, step (b) comprises or excludes measuring the expression of Angiomotin. In an alternative or additional embodiment, step (b) comprises or excludes measuring the expression of CD40 ligand. In an alternative or additional embodiment, step (b) comprises or excludes measuring the expression of CHX10. In an alternative or additional embodiment, step (b) comprises or excludes measuring the expression of GLP-1. In an alternative or additional embodiment, step (b) comprises or excludes measuring the expression of HADH2. In an alternative or additional embodiment, step (b) comprises or excludes measuring the expression of HLA-DR/DP. In an alternative or additional embodiment, step (b) comprises or excludes measuring the expression of IgM. In an alternative or additional embodiment, step (b) comprises or excludes measuring the expression of IL-11. In an alternative or additional embodiment, step (b) comprises or excludes measuring the expression of IL-12. In an alternative or additional embodiment, step (b) comprises or excludes measuring the expression of IL-16. In an alternative or additional embodiment, step (b) comprises or excludes measuring the expression of IL-18. In an alternative or additional embodiment, step (b) comprises or excludes measuring the expression of IL-1a. In an alternative or additional embodiment, step (b) comprises or excludes measuring the expression of IL-1ra. In an alternative or additional embodiment, step (b) comprises or excludes measuring the expression of IL-3. In an alternative or additional embodiment, step (b) comprises or excludes measuring the expression of IL-4. In an alternative or additional embodiment, step (b) comprises or excludes measuring the expression of IL-6. In an alternative or additional embodiment, step (b) comprises or excludes measuring the expression of IL-7. In an alternative or additional embodiment, step (b) comprises or excludes measuring the expression of IL-9. In an alternative or additional embodiment, step (b) comprises or excludes measuring the expression of Lewis X. In an alternative or additional embodiment, step (b) comprises or excludes measuring the expression of MCP-1. In an alternative or additional embodiment, step (b) comprises or excludes measuring the expression of RANTES. In an alternative or additional embodiment, step (b) comprises or excludes measuring the expression of sox11a. In an alternative or additional embodiment, step (b) comprises or excludes measuring the expression of TGF-β1. In an alternative or additional embodiment, step (b) comprises or excludes measuring the expression of TNF-b. In an alternative or additional embodiment, step (b) comprises or excludes measuring the expression of TNFRSF3. In an alternative or additional embodiment, step (b) comprises or excludes measuring the expression of VEGF.

By "transmembrane peptide" or "TM peptide" we mean a peptide derived from a 10TM protein, to which the scFv antibody construct of SEQ ID NO: 1 below has specificity (wherein the CDR sequences are indicated by bold, italicised text):

Hence, this scFv may be used or any antibody, or antigen binding fragment thereof, that competes with this scFv for binding to the 10TM protein. For example, the antibody, or antigen binding fragment thereof, may comprise the same CDRs as present in SEQ ID NO:1.

It will be appreciated by persons skilled in the art that such an antibody may be produced with an affinity tag (e.g. at the C-terminus) for purification purposes. For example, an affinity tag of SEQ ID NO: 2 below may be utilised:
DYKDHDGDYKDHDIDYKDDDDKAAAHHHHHH
**[SEQ ID NO: 2]**

Also disclosed herein, step (b) comprises or consists of measuring the expression of one or more of the biomarkers listed in Table A(i), for example, at least 2 of the biomarkers listed in Table A(i).

Step (b) comprises measuring the expression of PRD14 and HsHec1.

Step (b) comprises or consists of measuring the expression of all of the biomarkers listed in Table A(i).

Alternatively or additionally step (b) comprises or consists of measuring the expression of 1 or more of the biomarkers listed in Table (A)(ii), for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 or 46 of the biomarkers listed in Table A(ii).

Alternatively or additionally step (b) comprises or consists of measuring the expression of all of the biomarkers listed in Table A(ii).

Alternatively or additionally step (b) comprises or consists of measuring the expression of 1 or more biomarkers from the biomarkers listed in Table A(iii), for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 of the biomarkers listed in Table A(iii).

Alternatively or additionally step (b) comprises or consists of measuring the expression of all of the biomarkers listed in Table A(iii).

Alternatively or additionally step (b) comprises or consists of measuring the expression of 1 or more biomarkers from the biomarkers listed in Table A(iv), for example at least 2, 3, 4, 5, 6, 7, 8, 9, 10, 11, 12, 13, 14, 15, 16, 17, 18, 19, 20, 21, 22, 23, 24, 25 or 26 of the biomarkers listed in Table A(iv).

Alternatively or additionally step (b) comprises or consists of measuring the expression of all of the biomarkers listed in Table A(iv).

Alternatively or additionally step (b) comprises or consists of measuring the expression in the test sample of all of the biomarkers defined in Table A.

In an alternative or additional embodiment, step (b) comprises or consists of measuring the expression of 1 or more biomarkers from the biomarkers listed in Table 1, for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 or 37 of the biomarkers listed in Table 1.

In an alternative or additional embodiment, step (b) comprises or consists of measuring the expression of 1 or more biomarkers from the biomarkers listed in Table 2, for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 of the biomarkers listed in Table 2.

In an alternative or additional embodiment, step (b) comprises or consists of measuring the expression of 1 or more biomarkers from the biomarkers listed in Table 3.

In an alternative or additional embodiment, step (b) comprises or consists of measuring the expression of 1 or more biomarkers from the biomarkers listed in Table 4.

Alternatively or additionally the pancreatic cancer is selected from the group consisting of adenocarcinoma, adenosquamous carcinoma, signet ring cell carcinoma, hepatoid carcinoma, colloid carcinoma, undifferentiated carcinoma, undifferentiated carcinomas with osteoclast-like giant cells, malignant serous cystadenoma, pancreatic sarcoma, and tubular papillary pancreatic adenocarcinoma.

Alternatively or additionally the pancreatic cancer is an adenocarcinoma, for example, pancreatic ductal adenocarcinoma.

Generally, the diagnosis/determination is made with an ROC AUC of at least 0.51, for example with an ROC AUC of at least, 0.52, 0.53, 0.54, 0.55, 0.60, 0.65, 0.70, 0.75, 0.80, 0.85, 0.90, 0.95, 0.96, 0.97, 0.98, 0.99 or with an ROC AUC of 1.00. Preferably, diagnosis is made with an ROC AUC of at least 0.85, and most preferably with an ROC AUC of 1.

Typically, diagnosis is performed using a support vector machine (SVM), such as those available from http://cran.r-project.org/web/packages/e1071/index.html (e.g. e1071 1.5-24). However, any other suitable means may also be used.

Support vector machines (SVMs) are a set of related supervised learning methods used for classification and regression. Given a set of training examples, each marked as belonging to one of two categories, an SVM training algorithm builds a model that predicts whether a new example falls into one category or the other. Intuitively, an SVM model is a representation of the examples as points in space, mapped so that the examples of the separate categories are divided by a clear gap that is as wide as possible. New examples are then mapped into that same space and predicted to belong to a category based on which side of the gap they fall on.

More formally, a support vector machine constructs a hyperplane or set of hyperplanes in a high or infinite dimensional space, which can be used for classification, regression or other tasks. Intuitively, a good separation is achieved by the hyperplane that has the largest distance to the nearest training datapoints of any class (so-called functional margin), since in general the larger the margin the lower the generalization error of the classifier. For more information on SVMs, see for example, Burges, 1998, Data Mining and Knowledge Discovery, 2:121-167.

In an alternative or additional embodiment of the invention, the SVM is 'trained' prior to performing the methods of the invention using biomarker profiles from individuals with known disease status (for example, individuals known to have pancreatic cancer, individuals known to have acute inflammatory pancreatitis, individuals known to have chronic pancreatitis or individuals known to be healthy). By running such training samples, the SVM is able to learn what biomarker profiles are associated with pancreatic cancer. Once the training process is complete, the SVM is then able whether or not the biomarker sample tested is from an individual with pancreatic cancer.

However, this training procedure can be by-passed by pre-programming the SVM with the necessary training parameters. For example, diagnoses can be performed according to the known SVM parameters using an SVM algorithm based on the measurement of any or all of the biomarkers listed in Table A.

It will be appreciated by skilled persons that suitable SVM parameters can be determined for any combination of the biomarkers listed in Table A by training an SVM machine with the appropriate selection of data (i.e. biomarker measurements from individuals with known pancreatic cancer status). Alternatively, the Table 1-5 data may be used to determine a particular pancreatic cancer-associated disease state according to any other suitable statistical method known in the art.

In an alternative or additional embodiment the presence and/or amount in the test sample of the two or more biomarker measured in step (b) is significantly different (i.e., statistically significantly different) from the presence and/or amount of the two or more biomarker measured in step (d) or the predetermined reference values. For example, as discussed in the accompanying Examples, significant difference between the presence and/or amount of a particular biomarker in the test and control samples may be classified as those where p<0.05 (for example, where p<0.04, p<0.03, p<0.02 or where p<0.01).

Alternatively, the data provided in the present figures and tables may be used to determine a particular pancreatic cancer-associated disease state according to any other suitable statistical method known in the art, such as Principal Component Analysis (PCA) and other multivariate statistical analyses (e.g., backward stepwise logistic regression model). For a review of multivariate statistical analysis see, for example, Schervish, Mark J. (November 1987). "A Review of Multivariate Analysis". Statistical Science 2 (4): 396-413.

Preferably, the method of the invention has an accuracy of at least 60%, for example 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% accuracy.

Preferably, the method of the invention has a sensitivity of at least 60%, for example 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sensitivity.

Preferably, the method of the invention has a specificity of at least 60%, for example 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% specificity.

By "accuracy" we mean the proportion of correct outcomes of a method, by "sensitivity" we mean the proportion of all PaC positive sample that are correctly classified as positives, and by "specificity" we mean the proportion of all PaC negative samples that are correctly classified as negatives.

In an alternative or additional embodiment, the individual not afflicted with pancreatic cancer is not afflicted with pancreatic cancer (PaC), chronic pancreatitis (ChP) or acute inflammatory pancreatitis (AIP). More preferably, the individual not afflicted with pancreatic cancer is a healthy individual not afflicted with any pancreatic disease or condition. Even more preferably, the individual not afflicted with pancreatic cancer is not afflicted with any disease or condition. Most preferably, the individual not afflicted with pancreatic cancer is a healthy individual. Alternatively or additionally, by a "healthy individual" we include individuals considered by a skilled person to be physically vigorous and free from physical disease.

However, in another embodiment the individual not afflicted with pancreatic cancer is afflicted with chronic pancreatitis. In still another embodiment, the individual not afflicted with pancreatic cancer is afflicted with acute inflammatory pancreatitis.

Alternatively or additionally step (b), (d), (f), (h) and/or step (j) is performed using a first binding agent capable of binding to the one or more biomarkers.

It will be appreciated by persons skilled in the art that the first binding agent may comprise or consist of a single species with specificity for one of the protein biomarkers or a plurality of different species, each with specificity for a different protein biomarker.

Suitable binding agents (also referred to as binding molecules) can be selected from a library, based on their ability to bind a given motif, as discussed below.

At least one type of the binding agents, and more typically all of the types, may comprise or consist of an antibody or antigen-binding fragment of the same, or a variant thereof.

Methods for the production and use of antibodies are well known in the art, for example see Antibodies: A Laboratory Manual, 1988, Harlow & Lane, Cold Spring Harbor Press, ISBN-13: 978-0879693145, Using Antibodies: A Laboratory Manual, 1998, Harlow & Lane, Cold Spring Harbor Press, ISBN-13: 978-0879695446 and Making and Using Antibodies: A Practical Handbook, 2006, Howard & Kaser, CRC Press, ISBN-13: 978-0849335280.

Thus, a fragment may contain one or more of the variable heavy (V_{H}) or variable light (V_{L}) domains. For example, the term antibody fragment includes Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544).

The term "antibody variant" includes any synthetic antibodies, recombinant antibodies or antibody hybrids, such as but not limited to, a single-chain antibody molecule produced by phage-display of immunoglobulin light and/or heavy chain variable and/or constant regions, or other immuno-interactive molecule capable of binding to an antigen in an immunoassay format that is known to those skilled in the art.

A general review of the techniques involved in the synthesis of antibody fragments which retain their specific binding sites is to be found in Winter & Milstein (1991) Nature 349, 293-299.

Molecular libraries such as antibody libraries (Clackson et al, 1991, Nature 352, 624-628; Marks et al, 1991, J Mol Biol 222(3): 581-97), peptide libraries (Smith, 1985, Science 228(4705): 1315-7), expressed cDNA libraries (Santi et al (2000) J Mol Biol 296(2): 497-508), libraries on other scaffolds than the antibody framework such as affibodies (Gunneriusson et al, 1999, Appl Environ Microbiol 65(9): 4134-40) or libraries based on aptamers (Kenan et al, 1999, Methods Mol Biol 118, 217-31) may be used as a source from which binding molecules that are specific for a given motif are selected for use in the methods of the invention.

The molecular libraries may be expressed *in vivo* in prokaryotic (Clackson *et al*, 1991, *op. cit.;* Marks *et al,* 1991, *op. cit.*) or eukaryotic cells (Kieke et al, 1999, Proc Natl Acad Sci USA, 96(10):5651-6) or may be expressed *in vitro* without involvement of cells (Hanes & Pluckthun, 1997, Proc Natl Acad Sci USA 94(10):4937-42; He & Taussig, 1997, Nucleic Acids Res 25(24):5132-4; Nemoto et al, 1997, FEBS Lett, 414(2):405-8).

In cases when protein based libraries are used often the genes encoding the libraries of potential binding molecules are packaged in viruses and the potential binding molecule is displayed at the surface of the virus (Clackson *et al*, 1991, *op. cit*.; Marks *et al*, 1991, *op. cit*; Smith, 1985, *op. cit.*).

The most commonly used such system today is filamentous bacteriophage displaying antibody fragments at their surfaces, the antibody fragments being expressed as a fusion to the minor coat protein of the bacteriophage (Clackson *et al,* 1991, *op. cit*.; Marks *et al,* 1991, *op*. *cit).* However, also other systems for display using other viruses (EP 39578), bacteria (Gunneriusson *et al,* 1999, *op. cit*.; Daugherty et al, 1998, Protein Eng 11(9):825-32; Daugherty et al, 1999, Protein Eng 12(7):613-21), and yeast (Shusta et al, 1999, J Mol Biol 292(5):949-56) have been used.

In addition, display systems have been developed utilising linkage of the polypeptide product to its encoding mRNA in so called ribosome display systems (Hanes & Pluckthun, 1997, *op*. *cit*.; He & Taussig, 1997, *op. cit*.; Nemoto *et al*, 1997, *op. cit.),* or alternatively linkage of the polypeptide product to the encoding DNA (see US Patent No. 5,856,090 and WO 98/37186).

When potential binding molecules are selected from libraries one or a few selector peptides having defined motifs are usually employed. Amino acid residues that provide structure, decreasing flexibility in the peptide or charged, polar or hydrophobic side chains allowing interaction with the binding molecule may be used in the design of motifs for selector peptides.

For example:
(i) Proline may stabilise a peptide structure as its side chain is bound both to the alpha carbon as well as the nitrogen;
(ii) Phenylalanine, tyrosine and tryptophan have aromatic side chains and are highly hydrophobic, whereas leucine and isoleucine have aliphatic side chains and are also hydrophobic;
(iii) Lysine, arginine and histidine have basic side chains and will be positively charged at neutral pH, whereas aspartate and glutamate have acidic side chains and will be negatively charged at neutral pH;
(iv) Asparagine and glutamine are neutral at neutral pH but contain a amide group which may participate in hydrogen bonds;
(v) Serine, threonine and tyrosine side chains contain hydroxyl groups, which may participate in hydrogen bonds.

Typically, selection of binding agents may involve the use of array technologies and systems to analyse binding to spots corresponding to types of binding molecules.

In an alternative or additional embodiment, the first binding agent(s) is/are immobilised on a surface (e.g. on a multiwell plate or array).

The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison et al (1984) Proc. Natl. Acad. Sci. USA 81, 6851-6855).

That antigenic specificity is conferred by variable domains and is independent of the constant domains is known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293-299.

By "ScFv molecules" we mean molecules wherein the V_{H} and V_{L} partner domains are linked via a flexible oligopeptide.

The advantages of using antibody fragments, rather than whole antibodies, are several-fold. The smaller size of the fragments may lead to improved pharmacological properties, such as better penetration of solid tissue. Effector functions of whole antibodies, such as complement binding, are removed. Fab, Fv, ScFv and dAb antibody fragments can all be expressed in and secreted from E. *coli,* thus allowing the facile production of large amounts of the said fragments.

Whole antibodies, and F(ab')₂ fragments are "bivalent". By "bivalent" we mean that the said antibodies and F(ab')₂ fragments have two antigen combining sites. In contrast, Fab, Fv, ScFv and dAb fragments are monovalent, having only one antigen combining sites.

The antibodies may be monoclonal or polyclonal. Suitable monoclonal antibodies may be prepared by known techniques, for example those disclosed in "Monoclonal Antibodies: A manual of techniques", H Zola (CRC Press, 1988) and in "Monoclonal Hybridoma Antibodies: Techniques and applications", J G R Hurrell (CRC Press, 1982).

In an alternative or additional embodiment, the first binding agent immobilised on a surface (e.g. on a multiwell plate or array).

The advantages of using antibody fragments, rather than whole antibodies, are several-fold. The smaller size of the fragments may lead to improved pharmacological properties, such as better penetration of solid tissue. Effector functions of whole antibodies, such as complement binding, are removed. Fab, Fv, ScFv and dAb antibody fragments can all be expressed in and secreted from E. *coli,* thus allowing the facile production of large amounts of the said fragments.

Whole antibodies, and F(ab')₂ fragments are "bivalent". By "bivalent" we mean that the said antibodies and F(ab')₂ fragments have two antigen combining sites. In contrast, Fab, Fv, ScFv and dAb fragments are monovalent, having only one antigen combining sites.

The antibodies may be monoclonal or polyclonal. Suitable monoclonal antibodies may be prepared by known techniques, for example those disclosed in "Monoclonal Antibodies: A manual of techniques", H Zola (CRC Press, 1988) and in "Monoclonal Hybridoma Antibodies: Techniques and applications", J G R Hurrell (CRC Press, 1982).

Hence, the first binding agent may comprise or consist of an antibody or an antigen-binding fragment thereof. Preferably, the antibody or antigen-binding fragment thereof is a recombinant antibody or antigen-binding fragment thereof. The antibody or antigen-binding fragment thereof may be selected from the group consisting of: scFv, Fab, and a binding domain of an immunoglobulin molecule.

The first binding agent may be immobilised on a surface.

Alternatively or additionally the first binding agent comprises or consists of an antibody or an antigen-binding fragment thereof, e.g., a recombinant antibody or antigen-binding fragment thereof. The antibody or antigen-binding fragment thereof may be selected from the group consisting of: scFv; Fab; a binding domain of an immunoglobulin molecule.

Alternatively or additionally the two or more biomarkers in the test sample are labelled with a detectable moiety.

By a "detectable moiety" we include the meaning that the moiety is one which may be detected and the relative amount and/or location of the moiety (for example, the location on an array) determined.

Suitable detectable moieties are well known in the art.

Thus, the detectable moiety may be a fluorescent and/or luminescent and/or chemiluminescent moiety which, when exposed to specific conditions, may be detected. For example, a fluorescent moiety may need to be exposed to radiation (*i.e.* light) at a specific wavelength and intensity to cause excitation of the fluorescent moiety, thereby enabling it to emit detectable fluorescence at a specific wavelength that may be detected.

Alternatively, the detectable moiety may be an enzyme which is capable of converting a (preferably undetectable) substrate into a detectable product that can be visualised and/or detected. Examples of suitable enzymes are discussed in more detail below in relation to, for example, ELISA assays.

Alternatively, the detectable moiety may be a radioactive atom which is useful in imaging. Suitable radioactive atoms include ^{99m}Tc and ¹²³I for scintigraphic studies. Other readily detectable moieties include, for example, spin labels for magnetic resonance imaging (MRI) such as ¹²³I again, ¹³¹I, ¹¹¹In, ¹⁹F, ¹³C, ¹⁵N, ¹⁷O, gadolinium, manganese or iron. Clearly, the agent to be detected (such as, for example, the two or more biomarkers in the test sample and/or control sample described herein and/or an antibody molecule for use in detecting a selected protein) must have sufficient of the appropriate atomic isotopes in order for the detectable moiety to be readily detectable.

The radio- or other labels may be incorporated into the agents of the invention (i.e. the proteins present in the samples of the methods of the invention and/or the binding agents of the invention) in known ways. For example, if the binding moiety is a polypeptide it may be biosynthesised or may be synthesised by chemical amino acid synthesis using suitable amino acid precursors involving, for example, fluorine-19 in place of hydrogen. Labels such as ^{99m}Tc, ¹²³I, ¹⁸⁶Rh, ¹⁸⁸Rh and ¹¹¹In can, for example, be attached *via* cysteine residues in the binding moiety. Yttrium-90 can be attached via a lysine residue. The IODOGEN method (Fraker et al (1978) Biochem. Biophys. Res. Comm. 80, 49-57) can be used to incorporate ¹²³I. Reference ("Monoclonal Antibodies in Immunoscintigraphy", J-F Chatal, CRC Press, 1989) describes other methods in detail. Methods for conjugating other detectable moieties (such as enzymatic, fluorescent, luminescent, chemiluminescent or radioactive moieties) to proteins are well known in the art.

Preferably, the two or more biomarkers in the control sample(s) are labelled with a detectable moiety. The detectable moiety may be selected from the group consisting of: a fluorescent moiety; a luminescent moiety; a chemiluminescent moiety; a radioactive moiety; an enzymatic moiety. However, it is preferred that the detectable moiety is biotin.

Alternatively or additionally the two or more biomarkers in the control sample(s) are labelled with a detectable moiety. The detectable moiety may be selected from, for example, the group consisting of: a fluorescent moiety; a luminescent moiety; a chemiluminescent moiety; a radioactive moiety; an enzymatic moiety. Alternatively or additionally the detectable moiety is biotin.

Alternatively or additionally step (b), (d), (f), (h) and/or step (j) is performed using an assay comprising a second binding agent capable of binding to the two or more biomarkers, the second binding agent comprising a detectable moiety.

Alternatively or additionally the second binding agent comprises or consists of an antibody or an antigen-binding fragment thereof, e.g., a recombinant antibody or antigen-binding fragment thereof. The antibody or antigen-binding fragment thereof may be selected from the group consisting of: scFv; Fab; a binding domain of an immunoglobulin molecule.

Alternatively or additionally the detectable moiety is selected from the group consisting of: a fluorescent moiety; a luminescent moiety; a chemiluminescent moiety; a radioactive moiety; an enzymatic moiety, e.g., a fluorescent moiety (for example an Alexa Fluor dye, e.g. Alexa647).

Alternatively or additionally the method comprises or consists of an ELISA (Enzyme Linked Immunosorbent Assay).

Preferred assays for detecting serum or plasma proteins include enzyme linked immunosorbent assays (ELISA), radioimmunoassay (RIA), immunoradiometric assays (IRMA) and immunoenzymatic assays (IEMA), including sandwich assays using monoclonal and/or polyclonal antibodies. Exemplary sandwich assays are described by David et al in US Patent Nos. 4,376,110 and 4,486,530. Antibody staining of cells on slides may be used in methods well known in cytology laboratory diagnostic tests, as well known to those skilled in the art.

Typically, the assay is an ELISA (Enzyme Linked Immunosorbent Assay) which typically involves the use of enzymes giving a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemi-luminescent systems based on enzymes such as luciferase can also be used.

ELISA methods are well known in the art, for example see The ELISA Guidebook (Methods in Molecular Biology), 2000, Crowther, Humana Press, ISBN-13: 978-0896037281.

Conjugation with the vitamin biotin is frequently used since this can readily be detected by its reaction with enzyme-linked avidin or streptavidin to which it binds with great specificity and affinity.

However, step (b), (d), (f), (h) and/or step (j) is alternatively performed using an array. Arrays *per se* are well known in the art. Typically they are formed of a linear or two-dimensional structure having spaced apart (*i.e.* discrete) regions ("spots"), each having a finite area, formed on the surface of a solid support. An array can also be a bead structure where each bead can be identified by a molecular code or colour code or identified in a continuous flow. Analysis can also be performed sequentially where the sample is passed over a series of spots each adsorbing the class of molecules from the solution. The solid support is typically glass or a polymer, the most commonly used polymers being cellulose, polyacrylamide, nylon, polystyrene, polyvinyl chloride or polypropylene. The solid supports may be in the form of tubes, beads, discs, silicon chips, microplates, polyvinylidene difluoride (PVDF) membrane, nitrocellulose membrane, nylon membrane, other porous membrane, non-porous membrane (e.g. plastic, polymer, perspex, silicon, amongst others), a plurality of polymeric pins, or a plurality of microtitre wells, or any other surface suitable for immobilising proteins, polynucleotides and other suitable molecules and/or conducting an immunoassay. The binding processes are well known in the art and generally consist of cross-linking covalently binding or physically adsorbing a protein molecule, polynucleotide or the like to the solid support. By using well-known techniques, such as contact or non-contact printing, masking or photolithography, the location of each spot can be defined. For reviews see Jenkins, R.E., Pennington, S.R. (2001, Proteomics, 2,13-29) and Lal et al (2002, Drug Discov Today 15;7(18 Suppl):S143-9).

Typically the array is a microarray. By "microarray" we include the meaning of an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance. The array may also be a macroarray or a nanoarray.

Once suitable binding molecules (discussed above) have been identified and isolated, the skilled person can manufacture an array using methods well known in the art of molecular biology.

Alternatively or additionally the array is a bead-based array. Alternatively or additionally the array is a surface-based array. Alternatively or additionally the array is selected from the group consisting of: macroarray; microarray; nanoarray.

Alternatively or additionally the method comprises:
(i) labelling biomarkers present in the sample with biotin;
(ii) contacting the biotin-labelled proteins with an array comprising a plurality of scFv immobilised at discrete locations on its surface, the scFv having specificity for one or more of the proteins in Table A;
(iii) contacting the immobilised scFv with a streptavidin conjugate comprising a fluorescent dye; and
(iv) detecting the presence of the dye at discrete locations on the array surface
wherein the expression of the dye on the array surface is indicative of the expression of a biomarker from Table A in the sample.

Alternatively or additionally step (b), (d), (f), (h) and/or (j) comprises measuring the expression of a nucleic acid molecule encoding the two or more biomarkers.

Alternatively or additionally the nucleic acid molecule is a ctDNA molecule, a cDNA molecule or an mRNA molecule. Alternatively or additionally the nucleic acid molecule is not a ctDNA molecule.

Alternatively or additionally the nucleic acid molecule is a cDNA molecule.

Alternatively or additionally measuring the expression of the two or more biomarkers in step (b), (d) and/or (f) is performed using a method selected from the group consisting of Southern hybridisation, Northern hybridisation, polymerase chain reaction (PCR), reverse transcriptase PCR (RT-PCR), quantitative real-time PCR (qRT-PCR), nanoarray, microarray, macroarray, autoradiography and *in situ* hybridisation.

Alternatively or additionally measuring the expression of the two or more biomarkers in step (b) is determined using a DNA microarray.

Alternatively or additionally measuring the expression of the two or more biomarkers in step (b), (d), (f), (h) and/or (j) is performed using one or more binding moieties, each individually capable of binding selectively to a nucleic acid molecule encoding one of the biomarkers identified in Table A.

Alternatively or additionally the one or more binding moieties each comprise or consist of a nucleic acid molecule. Alternatively or additionally the one or more binding moieties each comprise or consist of DNA, RNA, PNA, LNA, GNA, TNA or PMO. Alternatively or additionally the one or more binding moieties each comprise or consist of DNA. Alternatively or additionally the one or more binding moieties are 5 to 100 nucleotides in length.

Alternatively or additionally the one or more nucleic acid molecules are 15 to 35 nucleotides in length. Alternatively or additionally the binding moiety comprises a detectable moiety. The detectable moiety may be selected from the group consisting of: a fluorescent moiety; a luminescent moiety; a chemiluminescent moiety; a radioactive moiety (for example, a radioactive atom); or an enzymatic moiety. The detectable moiety may comprise or consist of a radioactive atom. The radioactive atom may be selected from the group consisting of technetium-99m, iodine-123, iodine-125, iodine-131, indium-111, fluorine-19, carbon-13, nitrogen-15, oxygen-17, phosphorus-32, sulphur-35, deuterium, tritium, rhenium-186, rhenium-188 and yttrium-90. Alternatively or additionally the detectable moiety of the binding moiety may be a fluorescent moiety.

Alternatively or additionally the sample provided in step (b), (d), (f), (h) and/or (j) is selected from the group consisting of unfractionated blood, plasma, serum, tissue fluid, pancreatic tissue, pancreatic juice, bile and urine.

Alternatively or additionally the sample provided in step (b), (d), (f), (h) and/or (j) is selected from the group consisting of unfractionated blood, plasma and serum. Alternatively or additionally the sample provided in step (b), (d), (f), (h) and/or (j) is plasma.

Alternatively or additionally the method comprises the step of:
(k) providing the individual with pancreatic cancer therapy,
wherein, in the event that the pancreatic cancer is determined to be located in and/or originated from the head of the pancreas, the pancreatic cancer therapy is conventional; in the event that pancreatic cancer is determined to be located in and/or originated from the body or tail of the pancreas, the pancreatic cancer therapy is treated more aggressively than dictated by convention; and
wherein, in the event that pancreatic cancer is not found to be present, the individual is not provided pancreatic cancer therapy.

Alternatively or additionally, in the event that the pancreatic cancer is determined to be located in and/or originated from the body/tail of the pancreas, the pancreatic cancer therapy is conventional; in the event that pancreatic cancer is determined to be located in and/or originated from the head of the pancreas, the pancreatic cancer therapy is treated more aggressively than dictated by convention.

In the event that the individual is not diagnosed with pancreatic cancer, they may be subjected to further monitoring for pancreatic cancer (for example, using the methods described in the present specification).

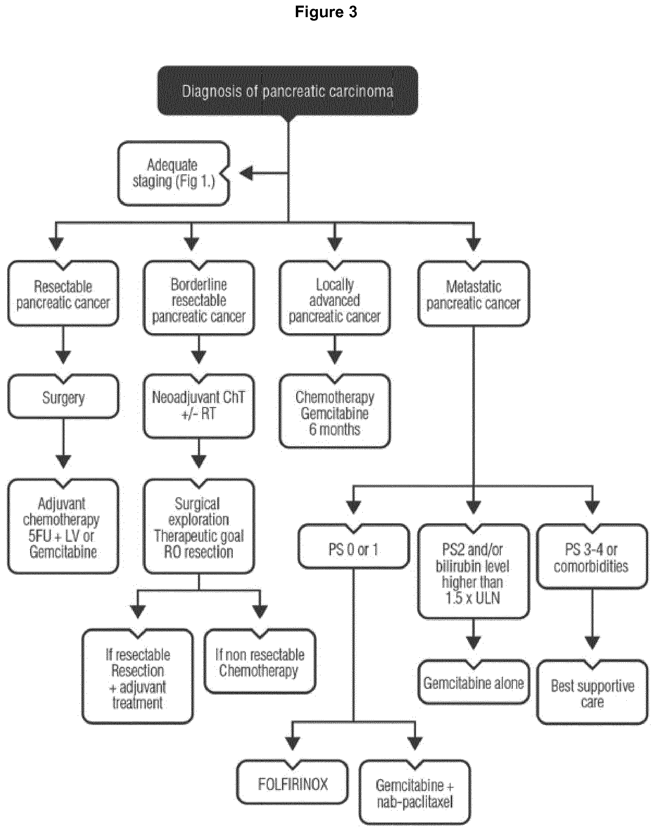

By 'conventional' pancreatic cancer therapy we include those methods known to the skilled person including those described in Ducreux et al., 2015, 'Cancer of the pancreas: ESMO Clinical Practice Guidelines for diagnosis, treatment and follow-up' Annals of Oncology, 26 (Supplement 5): v56-v68 and/or Freelove & Walling, 2006, 'Pancreatic Cancer: Diagnosis and Management' American Family Physician, 73(3):485-492. See also, the treatment strategy shown in Figure 3.

By 'treated more aggressively than dictated by convention' we include that the treatment regime provided to the individual is consistent with the treatment of a high pancreatic cancer grade, for example, one, two or three cancer stages higher. For example, in the treatment strategy shown in Figure 3, a stage 1 cancer may be treated with the regime for a stage 2, 3 or 4 cancer, a stage 2 cancer may be treated with a regime for a stage 3 or 4 cancer, a stage 3 cancer may be treated with a regime for a stage 4 cancer and a stage 4 cancer may be treated with greater dosage, frequency and/ or duration than conventional or usual for stage 4 cancer). Alternatively or additionally, the cancer may be treated with greater dosage, frequency and/ or duration than conventional or usual for that stage of cancer. Alternatively or additionally, the treatment regime provided is consistent with metastatic pancreatic cancer, even where metastases have not yet been detected.

Stage 1 is the earliest stage. The cancer is contained inside the pancreas, although it may be quite large. There is no cancer in the lymph nodes close to the pancreas and no sign that it has spread anywhere else in the body. Stage 1 is also referred to as resectable pancreatic cancer. In Stage 2 the cancer has started to grow outside the pancreas into nearby tissues and/or there is cancer in lymph nodes near the pancreas. Stage 2 is also referred to as borderline resectable pancreatic cancer.

In Stage 3 the cancer has spread into large blood vessels near the pancreas but hasn't spread to distant sites of the body such as the liver or lungs. Stage 3 is also referred to as locally advanced pancreatic cancer.

In Stage 4 the cancer has spread to distant sites such as the liver or lungs. Stage 4 is also referred to as metastatic pancreatic cancer.

Alternatively or additionally the pancreatic cancer therapy is selected from the group consisting of surgery, chemotherapy, immunotherapy, chemoimmunotherapy and thermochemotherapy.

In an alternative or additional embodiment the breast cancer therapy is selected from the group consisting of surgery, chemotherapy, immunotherapy, chemoimmunotherapy and thermochemotherapy (e.g., AC chemotherapy; Capecitabine and docetaxel chemotherapy (Taxotere ®); CMF chemotherapy; Cyclophosphamide; EC chemotherapy; ECF chemotherapy; E-CMF chemotherapy (Epi-CMF); Eribulin (Halaven®); FEC chemotherapy; FEC-T chemotherapy; Fluorouracil (5FU); GemCarbo chemotherapy; Gemcitabine (Gemzar ®); Gemcitabine and cisplatin chemotherapy (GemCis or GemCisplat); GemTaxol chemotherapy; Idarubicin (Zavedos ®); Liposomal doxorubicin (DaunoXome ®); Mitomycin (Mitomycin C Kyowa ®); Mitoxantrone; MM chemotherapy; MMM chemotherapy; Paclitaxel (Taxol ®); TAC chemotherapy; Taxotere and cyclophosphamide (TC) chemotherapy; Vinblastine (Velbe ®); Vincristine (Oncovin ®); Vindesine (Eldisine ®); and Vinorelbine (Navelbine ®)).

Also disclosed herein is an antineoplastic agent for use in treating pancreatic cancer wherein the dosage regime is determined based on the results of the method of the first aspect of the invention.

Also disclosed herein is the use of an antineoplastic agent in treating pancreatic cancer wherein the dosage regime is determined based on the results of the method of the first aspect of the invention.

Also disclosed herein is the use of an antineoplastic agent in the manufacture of a medicament for treating pancreatic cancer wherein the dosage regime is determined based on the results of the method of the first aspect of the invention.

Also disclosed herein is a method of treating pancreatic cancer comprising providing a sufficient amount of an antineoplastic agent wherein the amount of antineoplastic agent sufficient to treat the pancreatic cancer is determined based on the results of the method of the first aspect of the invention.

In one embodiment, the antineoplastic agent comprises or consists of an alkylating agent (ATC code L01a), an antimetabolite (ATC code L01b), a plant alkaloid or other natural product (ATC code L01c), a cytotoxic antibiotic or a related substance (ATC code L01d), or another antineoplastic agents (ATC code L01x).

Hence, in one embodiment the antineoplastic agent comprises or consists of an alkylating agent selected from the group consisting of a nitrogen mustard analogue (for example cyclophosphamide, chlorambucil, melphalan, chlormethine, ifosfamide, trofosfamide, prednimustine or bendamustine) an alkyl sulfonate (for example busulfan, treosulfan, or mannosulfan) an ethylene imine (for example thiotepa, triaziquone or carboquone) a nitrosourea (for example carmustine, lomustine, semustine, streptozocin, fotemustine, nimustine or ranimustine) an epoxides (for example etoglucid) or another alkylating agent (ATC code L01ax, for example mitobronitol, pipobroman, temozolomide or dacarbazine).

In a another embodiment the antineoplastic agent comprises or consists of an antimetabolite selected from the group consisting of a folic acid analogue (for example methotrexate, raltitrexed, pemetrexed or pralatrexate), a purine analogue (for example mercaptopurine, tioguanine, cladribine, fludarabine, clofarabine or nelarabine) or a pyrimidine analogue (for example cytarabine, fluorouracil (5-FU), tegafur, carmofur, gemcitabine, capecitabine, azacitidine or decitabine).

In a still further embodiment the antineoplastic agent comprises or consists of a plant alkaloid or other natural product selected from the group consisting of a vinca alkaloid or a vinca alkaloid analogue (for example vinblastine, vincristine, vindesine, vinorelbine or vinflunine), a podophyllotoxin derivative (for example etoposide or teniposide) a colchicine derivative (for example demecolcine), a taxane (for example paclitaxel, docetaxel or paclitaxel poliglumex) or another plant alkaloids or natural product (ATC code L01cx, for example trabectedin).

In one embodiment the antineoplastic agent comprises or consists of a cytotoxic antibiotic or related substance selected from the group consisting of an actinomycine (for example dactinomycin), an anthracycline or related substance (for example doxorubicin, daunorubicin, epirubicin, aclarubicin, zorubicin, idarubicin, mitoxantrone, pirarubicin, valrubicin, amrubicin or pixantrone) or another (ATC code L01dc, for example bleomycin, plicamycin, mitomycin or ixabepilone).

In a further embodiment the antineoplastic agent comprises or consists of another antineoplastic agent selected from the group consisting of a platinum compound (for example cisplatin, carboplatin, oxaliplatin, satraplatin or polyplatillen) a methylhydrazine (for example procarbazine) a monoclonal antibody (for example edrecolomab, rituximab, trastuzumab, alemtuzumab, gemtuzumab, cetuximab, bevacizumab, panitumumab, catumaxomab or ofatumumab) a sensitizer used in photodynamic/radiation therapy (for example porfimer sodium, methyl aminolevulinate, aminolevulinic acid, temoporfin or efaproxiral) or a protein kinase inhibitor (for example imatinib, gefitinib, erlotinib, sunitinib, sorafenib, dasatinib, lapatinib, nilotinib, temsirolimus, everolimus, pazopanib, vandetanib, afatinib, masitinib or toceranib).

In a still further embodiment the antineoplastic agent comprises or consists of another neoplastic agent selected from the group consisting of amsacrine, asparaginase, altretamine, hydroxycarbamide, lonidamine, pentostatin, miltefosine, masoprocol, estramustine, tretinoin, mitoguazone, topotecan, tiazofurine, irinotecan (camptosar), alitretinoin, mitotane, pegaspargase, bexarotene, arsenic trioxide, denileukin diftitox, bortezomib, celecoxib, anagrelide, oblimersen, sitimagene ceradenovec, vorinostat, romidepsin, omacetaxine mepesuccinate, eribulin or folinic acid.

In one embodiment the antineoplastic agent comprises or consists of a combination of one or more antineoplastic agent, for example, one or more antineoplastic agent defined herein. One example of a combination therapy used in the treatment of pancreatic cancer is FOLFIRINOX which is made up of the following four drugs:
- FOL - folinic acid (leucovorin);
- F - fluorouracil (5-FU);
- IRIN - irinotecan (Camptosar); and
- OX - oxaliplatin (Eloxatin).

A second aspect of the invention provides an array for determining the location of a tumour in the pancreas of an individual with pancreatic cancer, the array comprising antibodies or antigen-binding fragments thereof capable of binding to two or more biomarkers selected from the group defined in Table A (i), (ii) or (iii) wherein the two or more biomarkers selected from the group defined in Table A (i), (ii) or (iii) include PR domain zinc finger protein 14 (PRD14) and kinetochore protein NDC80 homolog (HsHec1).

Alternatively or additionally the two or more antibodies or an antigen-binding fragments thereof are capable of binding to all of the biomarkers/proteins defined in Table A (i.e., at least one binding agent is provided for each of the biomarkers listed in Table A).

In an alternative or additional embodiment, the array does not comprise binding moiety for one or more expressed human gene product absent from those biomarkers defined in step (b); for example, ≥2, ≥3, ≥4, ≥5, ≥6, ≥7, ≥8, ≥9, ≥10, ≥11, ≥12, ≥13, ≥14, ≥15, ≥16, ≥17, ≥18, ≥19, ≥20, ≥21, ≥22, ≥23, ≥24, ≥25, ≥26, ≥27, ≥28, ≥29, ≥30, ≥31, ≥32, ≥33, ≥34, ≥35, ≥36, ≥37, ≥38, ≥39, ≥40, ≥41, ≥42, ≥43, ≥44, ≥45, ≥46, ≥47, ≥48, ≥49, ≥50, ≥51, ≥52, ≥53, ≥54, ≥55, ≥56, ≥57, ≥58, ≥59, ≥60, ≥61, ≥62, ≥63, ≥64, ≥65, ≥66, ≥67, ≥68, ≥69, ≥70, ≥71, ≥72, ≥73, ≥74, ≥75, ≥76, ≥77, ≥78, ≥79, ≥80, ≥81, ≥82, ≥83, ≥84, ≥85, ≥86, ≥87, ≥88, ≥89, ≥90, ≥91, ≥92, ≥93, ≥94, ≥95, ≥96, ≥97, ≥98, ≥99 or ≥100 expressed human gene products absent from those biomarkers defined in step (b).

In an alternative or additional embodiment, the array does not comprise binding moiety for any expressed human gene product except for those biomarkers defined in step (b).

In an alternative or additional embodiment, in addition to the binding moieties for biomarkers defined in step (b), the arrays and methods of the invention include binding moieties for one or more control gene expression product (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 control gene expression products). For example, arrays consisting of binding moieties for only a defined number of Table A biomarkers may (or may not) additional comprise binding moiety for one or more control gene expression product.

By 'gene expression products' we include the same molecule types detected by the binding agents for the biomarkers of the invention.

Also disclosed herein is the use of one or more biomarkers selected from the group defined in Table A as a biomarker for determining the locality and/or presence of pancreatic cancer in an individual.

Alternatively or additionally all of the proteins defined in Table A are used as a marker for determining the locality and/or presence of pancreatic cancer in an individual. Alternatively or additionally the use is *in vitro.*

Also disclosed herein is a kit for determining the locality of pancreatic cancer comprising:
A) one or more first binding agent as defined in the first aspect of the invention or an array according to the first or second aspects of the invention;
B) instructions for performing the method as defined in the first aspect of the invention or the use as disclosed herein.

Alternatively or additionally the kit comprises a second binding agent as defined in the first aspect of the invention.

Preferred, non-limiting examples which embody certain aspects of the invention will now be described, with reference to the following tables and figures:

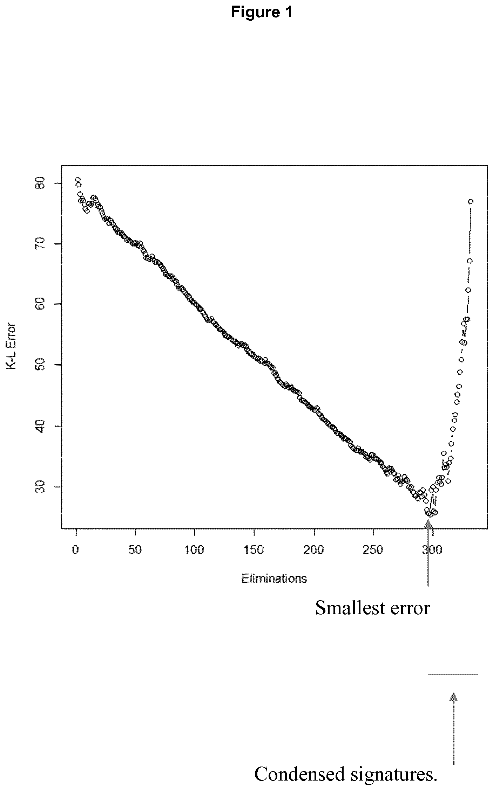

**Figure 1****: Backward elimination, defining a condensed signature differentiating tumours based on location (body/tail vs head)**
   The condensed signature is defined as the remaining antibodies (biomarkers) when the samellest error is obtained. The most important antibodies are retained the longest. The top 3 most important markers are II-12, STAT1, and PGAM5. The elimination order of 37 longest retained biomarkers are shown in Table 1.
**Figure 2****: Differentiating pancreatic cancer patients based on tumour location (body/tail vs head)**
   Principle component analysis is shown. NPC=non-pancreatic cancer
**Figure 3****: Treatment strategy**
   ChT, chemotherapy; RT, radiotherapy; 5-FU, 5-fluorouracil; LV, leucovorin; PS, performance status; ULN, upper limit of normal.

| **TABLE A** | | | |
|---|---|---|---|
| ***(i) Core biomarkers*** | | | |
| **#** | **Short name** | **Full name/CIMS sequence** | **Accession #** |
| | PRD14 | PR domain zinc finger protein 14 | Q9GZV8 |
| | HsHec1 | Kinetochore protein NDC80 homolog | O14777 |

| ***(ii) Preferred biomarkers*** | | | |
|---|---|---|---|
| **#** | **Short name** | **Full name/CIMS sequence** | **Accession #** |
| | hSpindly | Protein Spindly | Q96EA4 |
| | GNAI3 | Guanine nucleotide-binding protein G(k) subunit alpha | P08754 |
| | GRIP-2 | Glutamate receptor-interacting protein 2 | Q9C0E4 |
| | HsMAD2 | Mitotic spindle assembly checkpoint protein MAD2A | Q13257 |
| | TBC1D9 | TBC1 domain family member 9 | Q6ZT07 |
| | MAPKK6 | Dual specificity mitogen-activated protein kinase kinase 6 | P52564 |
| | MAPK9 | Mitogen-activated protein kinase 9 | P45984 |
| | MAPK8 | Mitogen-activated protein kinase 8 | P45983 |
| | ORP-3 | Oxysterol-binding protein-related protein 3 | Q9H4L5 |
| | MUC1 | Mucin-1 | P15941 |
| | PTK6 | Protein-tyrosine kinase 6 | Q13882 |
| | PTPN1 | Tyrosine-protein phosphatase non-receptor type 1 | P18031 |
| | R-PTP-eta | Receptor-type tyrosine-protein phosphatase eta | Q12913 |
| | R-PTP-O | Receptor-type tyrosine-protein phosphatase O | Q16827 |
| | PGAM5 | Serine/threonine-protein phosphatase PGAM5, mitochondrial | Q96HS1 |
| | STAT1 | Signal transducer and activator of transcription 1-alpha/beta | P42224 |
| | EGFR | Epidermal growth factor receptor | P00533 |
| | Surface Ag X | Surface ag x | Ab CDR sequences provided in Table B |
| | CIMS (1) | Selection peptide FLLMQYGGMDEHAR | Ab CDR sequences provided in Table B |
| | CIMS (11) | Selection peptide TEEQLK | Ab CDR sequences provided in Table B |
| | CIMS (12) | Selection peptide TEEQLK | Ab CDR sequences provided in Table B |
| | CIMS (13) | Selection peptide SSAYSR | Ab CDR sequences provided in Table B |
| | CIMS (14) | Selection peptide SSAYSR | Ab CDR sequences provided in Table B |
| | CIMS (15) | Selection peptide EDFR | Ab CDR sequences provided in Table B |
| | CIMS (16) | Selection peptide EDFR | Ab CDR sequences provided in Table B |
| | CIMS (17) | Selection peptide SYVSLK | Ab CDR sequences provided in Table B |
| | CIMS (18) | T Selection peptide LYVGK | Ab CDR sequences provided in Table B |
| | CIMS (2) | Selection peptide AQQHQWDGLLSYQDSLS | Ab CDR sequences provided in Table B |
| | CIMS (20) | Selection peptide EPFR | Ab CDR sequences provided in Table B |
| | CIMS (21) | Selection peptide LNVWGK | Ab CDR sequences provided in Table B |
| | CIMS (22) | Selection peptide QEASFK | Ab CDR sequences provided in Table B |
| | CIMS (23) | Selection peptide QEASFK | Ab CDR sequences provided in Table B |
| | CIMS (24) | Selection peptide LSADHR | Ab CDR sequences provided in Table B |
| | CIMS (25) | Selection peptide LSADHR | Ab CDR sequences provided in Table B |
| | CIMS (26) | Selection peptide SEAHLR | Ab CDR sequences provided in Table B |
| | CIMS (27) | Selection peptide SEAHLR | Ab CDR sequences provided in Table B |
| | CIMS (28) | Selection peptide SEAHLR | Ab CDR sequences provided in Table B |
| | CIMS (29) | Selection peptide SEAHLR | Ab CDR sequences provided in Table B |
| | CIMS (3) | Selection peptide GIVKYLYEDEG | Ab CDR sequences provided in Table B |
| | CIMS (30) | Selection peptide WDSR | Ab CDR sequences provided in Table B |
| | CIMS (31) | Selection peptide WDSR | Ab CDR sequences provided in Table B |
| | CIMS (4) | Selection peptide GIVKYLYEDEG | Ab CDR sequences provided in Table B |
| | CIMS (5) | Selection peptide WTRNSNMNYWLIIRL | Ab CDR sequences provided in Table B |
| | CIMS (6) | Selection peptide WTRNSNMNYWLIIRL | Ab CDR sequences provided in Table B |
| | CIMS (7) | Selection peptide LYEIAR | Ab CDR sequences provided in Table B |
| | CIMS (9) | Selection peptide LTEFAK | Ab CDR sequences provided in Table B |

| ***(iii) Further preferred biomarkers*** | | | |
|---|---|---|---|
| **#** | **Short name** | **Full name/CIMS sequence** | **Accession #** |
| | Apo-A1 | Apolipoprotein A1 | P02647 |
| | Apo-A4 | Apolipoprotein A4 | P06727 |
| | ATP-5B | ATP synthase subunit beta, mitochondrial | P06576 |
| | B-galactosidase | **Beta-galactosidase** | **P16278** |
| | BTK | **Tyrosine-protein kinase BTK** | Q06187 |
| | C1 inh. | Plasma protease C1 inhibitor | P05155 |
| | C1s | **Complement C1s** | P09871 |
| | C3 | Complement C3 | P01024 |
| | C4 | **Complement C4** | P0COL4/5 |
| | C5 | **Complement C5** | P01031 |
| | CD40 | CD40 protein | Q6P2H9 |
| | CDK-2 | **Cyclin-dependent kinase 2** | P24941 |
| | Cystatin C | Cystatin C | P01034 |
| | Eotaxin | **Eotaxin** | P51671 |
| | Factor B | Complement factor B | P00751 |
| | FASN | FASN protein | Q6PJJ3 |
| | GAK | GAK protein | Q5U4P5 |
| | GLP-1 R | Glucagon-like peptide 1 receptor | P43220 |
| | GM-CSF | Granulocyte-macrophage colony-stimulating factor | P04141 |
| | Her2/ErbB2 | Receptor tyrosine-protein kinase erbB-2 | P04626 |
| | ICAM-1 | Intercellular adhesion molecule 1 | P05362 |
| | IFN-γ | Interferon gamma | P01579 |
| | IL-10 | Interleukin-10 | P22301 |
| | IL-13 | Interleukin-13 | P35225 |
| | IL-1β | Interleukin-1 beta | P01584 |
| | IL-5 | Interleukin-5 | P05113 |
| | IL-8 | Interleukin-8 | P10145 |
| | Integrin a-10 | Integrin alpha-10 | O75578 |
| | Integrin a-11 | Integrin alpha-11 | Q9UKX5 |
| | JAK3 | Tyrosine-protein kinase JAK3 | P52333 |
| | KSYK | Tyrosine-protein kinase SYK | P43405 |
| | LDL | **Apolipoprotein B-100** | P04114 |
| | Leptin | **Leptin** | **P41159** |
| | MAPK1 | Mitogen-activated protein kinase 1 | P28482 |
| | MCP-3 | C-C motif chemokine 7 | P80098 |
| | MCP-4 | C-C motif chemokine 13 | Q99616 |
| | MYOM2 | Myomesin-2 | P54296 |
| | ORP-3 | Oxysterol-binding protein-related protein 3 | Q9H4L5 |
| | Osteopontin | **Osteopontin** | P10451 |
| | P85A | Phosphatidylinositol 3-kinase regulatory subunit alpha | P27986 |
| | Procathepsin W | Cathepsin W (N.B. Antibody is raised against the proenzyme of this protein) | P56202 |
| | Properdin | **Properdin** | **P27918** |
| | PSA | Prostate-specific antigen | P07288 |
| | RPS6KA2 | Ribosomal protein S6 kinase alpha-2 | Q15349 |
| | Sialyl Lewis X | Sialyl Lewis X | Carbohydrate: N/A |
| | STAP2 | Signal-transducing adaptor protein 2 | Q9UGK3 |
| | TM peptide | | |
| | TNF-a | Tumor necrosis factor | P01375 |
| | UCHL5 | Ubiquitin carboxyl-terminal hydrolase isozyme L5 | Q9Y5K5 |
| | UPF3B | Regulator of nonsense transcripts 3B | Q9BZI7 |

| ***(iv) Optional biomarkers*** | | | |
|---|---|---|---|
| **#** | **Short name** | **Full name/CIMS sequence** | **Accession #** |
| | Angiomotin | Angiomotin | Q4VCS5 |
| | CD40 ligand | CD40 ligand | P29965 |
| | CHX10 | Visual system homeobox 2 | P58304 |
| | GLP-1 | Glucagon-like peptide-1 | P01275 |
| | HADH2 | HADH2 protein | Q61BS9 |
| | HLA-DR/DP | HLA-DR/DP (N.B. 7 proteins in complex) | P01903/P01911/P79483/P13762/Q30154/P20036/P04440 |
| | IgM | **Immunoglobulin M** | e.g. P01871 (not complete protein); isotype-specific for IgM on Ramos B cells¹⁾ |
| | IL-11 | Interleukin-11 | P20809 |
| | IL-12 | Interleukin-12 | P29459/60 |
| | IL-16 | Interleukin-16 | Q14005 |
| | IL-18 | Interleukin-18 | Q14116 |
| | IL-1a | Interleukin-1 alpha | P01583 |
| | IL-1ra | Interleukin-1 receptor antagonist protein | P18510 |
| | IL-3 | Interleukin-3 | P08700 |
| | IL-4 | Interleukin-4 | P05112 |
| | IL-6 | Interleukin-6 | P05231 |
| | IL-7 | Interleukin-7 | P13232 |
| | IL-9 | Interleukin-9 | P15248 |
| | Lewis X | Lewis X | Carbohydrate: N/A |
| | MCP-1 | C-C motif chemokine 2 | P13500 |
| | RANTES | C-C motif chemokine 5 | P13501 |
| | sox11a | Transcription factor SOX-11 | P35716 |
| | TGF-β1 | Transforming growth factor beta-1 | P01137 |
| | TNF-b | Lymphotoxin-alpha | P01374 |
| | TNFRSF3 | Tumor necrosis factor receptor superfamily member 3 | P36941 |
| | VEGF | Vascular endothelial growth factor | P15692 |

**Table B - CDR regions for CIMS antibodies**

| | CDR regions of the selected antibody | | | | | |
|---|---|---|---|---|---|---|
| **antigen** | **CDR H1** | **CDR H2** | **CDR H3** | **CDR L1** | **CDR L2** | **CDR L3** |
| CIMS (1) | FSNYGMH | SSISSSSSYIF | VKDLYSRSWHAFDV | SGSSSXIGNNAVN | GNSNR | VXRXXXVLXXX |
| CIMS (2) | FRNYGMH | AVISYDGSNKY | ARHGRWGAAAGGFDY | SGTSSNIGTNYVY | GNSNR | QSYDSSLSGVV |
| CIMS (3) | FSSYSMN | AGVSGSGRTTL | ARGGYSSSSPFDY | TGSSSNIGAGYDVH | ANNQR | AAWDDSLNGWV |
| CIMS (4) | FSNYAMH | SSISNRGSRTF | ARDHRWDPGAFDI | TGSSSNIGADYDVH | GNSNR | AAWDDGLSGVV |
| CIMS (5) | FSDHYMD | SGISGSGGSTY | ASRLY | TGSSSNIGAGYVVH | DNDKR | AAWDDSLDAVL |
| CIMS (6) | FSSYAMS | SGISGGGETTN | ARRGVDY | TGSSSNLGAGYDVH | GNSNR | AAWDDSLNGVV |
| CIMS (7) | FSSYAMS | SALSRSGGRTY | ANFRGYSYGALDY | TGSSSNIGAGYDVH | GNSNR | QSYDGSLNSWV |
| CIMS (9) | FSDYYMSWIRQAPG | SSISSRSSYIYYADSVKGR | AKDREYYDILTGYPSMDV | CTGSSSNIGAGYDVH | DNNKRPS | CSAWDESLSGVV |
| CIMS (11) | FTSYSIHWVRQAPG | SAIGTGGGTYYADSVKGR | ARGGYFLDY | CSGSSSNIGSNTVN | GNSNRPS | CQSYDRSLSVNVV |
| CIMS (12) | FSSYGMHWVRQAPG | SAISGSGGSTYYADSVKGR | ARGGVGRYGMDV | CSGSSSNIGNNYVS | SNNQRPS | CATWDDSLSGGV |
| CIMS (13) | FSSYAMSWVRQAPG | SAISGSGGRTYYTDSVRDR | ARDLMPVCQYCYGMDV | CTGSSSNIGAGYDVH | SNNQRPS | CQSYDSSLNKDVV |
| CIMS (14) | FSDYYMSWVRQAPG | ADIKRDGSTRYYGDSVKGR | ARDRLVAGLFDY | CTGSSSNIGAGYDVH | GNSNRPS | CAAWDDSLSVL |
| CIMS (15) | FNTAMSWVRQAPG | SSISAGGTRTFYADSVRGR | ARHRAAGGGYYYGMDV | CSGSSSNIGSNSVN | DNNRRPS | CAAWDDSLNWV |
| CIMS (16) | FDDYGMSWVRQAPG | SAISGSGGSTYYADPVKGR | ARSRYGSGMDV | CSGSSSNIGSNYVY | KSNQRPS | CAAWDDRLNAVV |
| CIMS (17) | FSSYTMD | AKIKQDGSEKY | AGGDGSGWSF | TGNSSNIGAGYDVH | ENNKR | QSFDSSLSGPNWV |
| CIMS (18) | FSSYGMH | SSISSSSNYIY | ARDGGEGYGMDV | SGTNSNIGSNYVY | GNNNR | AAWDDSLNGPR |
| CIMS (20) | FSSYAMT | SAISGSGGSTY | TRWGLYGGARGFDY | SGSSSNIXTNXVX | XXXXX | XXXXXXXXX |
| CIMS (21) | FGDYAMS | AVTSHDESHKA | ARGRGYSYGTPLLDY | SGSSSNIGSISVN | SNNQR | AAWDASLSGWV |
| CIMS (22) | FSSYAMT | SGISGSGVSTY | AKVSSGGIAAAGIDY | TGSSSNLGAGYDVH | SNNQR | AAWDDSLNGPV |
| CIMS (23) | FSSYAMSWVRQAPG | SAISGSGGRTYYADAVKGR | ARHLKHDDGNSGAFDI | CSGSSSNIGTNYVY | SNNQRPS | CAAWDDSLSVWV |
| CIMS (24) | FGDYAMS | SAISGNGGNTY | AREKQWLFPPNIMDV | TGSSSNIGAGYDVH | GNSNR | HSYDSGLSGWA |
| CIMS (25) | FSNYAMSWVRQAPG | AFIRYDGSNKYYADSVKGR | ARDAVGGDSYVLDY | CSGSSSNIGSNAVN | GNSNRPS | CAAWDDSLNGWV |
| CIMS (26) | FSSYAMSWVRQAPG | SSISSSSSYIYYADSVKGR | ARHIQGSGGLDV | CSGGSSNIGSNTVN | RNNQRPS | CAAWDDSLSGW |
| CIMS (27) | FTSYSMSWVRQAPG | SAIGTGGGTYYADSVKGR | ARVNWNDAFDY | CSGSSSNIGNNAVN | RNDQRPS | CSTWDDSLSGVF |
| CIMS (28) | FSSYAMSWVRQAPG | AAIWSDGSNKYYADSVKGR | AKVGATDDAFDI | CSGSSSNIGSNTVN | GNSNRPS | CAAWDDSLNGPV |
| CIMS (29) | FNNYWMT | SAISGSGGSTY | ARHYGDYSLDAFDI | TGSSSNIGTAYGVH | GNSNR | AAWDDSLNGWV |
| CIMS (30) | FSSYWMH | SGINWNGGSTG | ARSRDGAFDI | TGTGSNIGAGYDVH | SNNQR | AAWDDSLNGPV |
| CIMS (31) | FSSYAMS | SGINWNGGSTG | AKLGGSYRAFDY | SGSSSNIGTNAVN | RNNQR | ASWDDSLSGPV |

### EXAMPLE A

### Summary

We have defined plasma biomarker capable of differentiating pancreatic cancer tumours based on localization in the pancreas (body/tail vs head).

### Material and Methods

*Plasma Samples:* This study was approved by the Ethics Committee of Tianjin Medical University Cancer Institute and Hospital (TMUCIH). After informed consent, blood was collected at TMUCIH, plasma was isolated and stored at -80°C. A total of 213 plasma samples were used for this study (Table I). The enrolled PDAC patients (n=118) were all Chinese Han ethnicity and treated at TMUCIH. None of the patients had received chemotherapy or radiotherapy at the time the samples were taken. All PDAC samples were confirmed by cytology. Patients were diagnosed with pancreatic ductal adenocarcinoma (PDAC) with the following exceptions: Malignant serous cystadenoma (n=1), pancreatic sarcoma (n=2), tubular papillary pancreatic adenocarcinoma (n=1). Five patients were diagnosed with PDAC with liver metastasis. Data on tumour stage and size at diagnosis (Table I), and tumour location within the pancreas were based on clinical pathology. Normal control (NC) samples (n=95) were collected from healthy inhabitants of Tianjin at their routine physical examination at TMUCIH, and were genetically unrelated to the PDAC patients.

The entire set of samples was labelled at one single occasion, using a previously optimized protocol (14). Briefly, 5 µL of crude samples were diluted 1:45 in PBS-EDTA (4 mM), resulting in an approximate protein concentration of 2 mg/mL, and labelled with a 15:1 molar excess of biotin to protein, using 0.6 mM EZ-Link Sulfo-NHS-LC-Biotin (Thermo Fisher Scientific, Rockford, IL, USA). Unbound biotin was removed by dialysis against PBS-EDTA for 72 hours, using Slide-A-Lyzer MINI dialysis device with 10K MWCO (Thermo Fisher Scientific). Labelled samples were aliquoted and stored at -20°C until used for microarray experiments.

*Generation of antibody microarrays:* The antibody microarrays contained 350 human recombinant scFv antibodies, selected and generated from in-house designed phage display antibody libraries (Table II). Most of the antibodies have previously been used in array applications (18-20), and a majority has been validated, using e.g. ELISA, mass spectrometry, spiking and/or blocking experiments (Table II). Eighty-six antibodies raised against cancer related biomarker proteins as part of the EU funded AFFINOMICS project (21) were novel to this study, but the high on-chip functionality of the scFv framework used has been demonstrated in an independent study (Säll et al, manuscript in preparation). The antibodies were produced in *E. coli and* purified from the periplasm, using a MagneHis Protein Purification system (Promega, Madison, WI, USA). The elution buffer was exchanged for PBS, using Zeba 96-well desalt spin plates (Pierce). The protein concentration was measured, using a NanoDrop spectrophotometer and the purity was checked using 10% SDS-PAGE. The entire set of 350 antibodies were produced in less than three weeks, and used for microarray printing within two weeks upon completion of production. The optimal printing concentration, defined as the highest concentration not resulting in a saturated signal was determined for each antibody by titrations in an arbitrarily selected biotinylated plasma and serum samples.

Antibody microarrays were produced on black MaxiSorp slides (NUNC, Roskilde, Denmark), using a non-contact printer (SciFlexarrayer S11, Scienion, Berlin, Germany). Fourteen identical subarrays (16,600 data points) were printed on each slide, each array consisting of 35x34 spots with a spot diameter of 130 µm and a spot-to-spot center distance of 200 µm. Each subarray consisted of three segments, separated by rows of Alexa Fluor647-labelled BSA. Antibodies were diluted to their optimal printing concentration (50-300 µg/mL) in a black polypropylene 384-well plate (NUNC). Alexa Fluor555-Cadeverine (0.1 µg/mL, ThermoFisher Scientific, Waltham, MA, USA) was added to each well to assist the spot localization and signal quantification. Each antibody was printed in three replicates, one in each array segment. The entire set of slides used for this study was printed at a single occasion. Slides were stored in plastic boxes, contained in laminated foil pouches (Corning, Corning, NY, USA), with silica gel. The pouches were heat sealed to protect from light and humidity. The slides were shipped to TMUCIH, Tianjin, China, and used for analysis within four weeks after printing.

*Antibody microarray analysis:* Ten slides (140 individual subarrays) were run per day. The slides were mounted in hybridization gaskets (Schott, Jena, Germany) and blocked with 150 µL PBSMT (1 % (w/v) milk, 1 % (v/v) Tween-20 in PBS) per array for 1.5h. All incubation steps were performed at RT in Biomixer II hybridization stations (CaptialBio, Beijing, China) on slow rotation (6 rpm). Meantime, aliquots of labelled serum samples were thawed on ice, diluted 1:10 in PBSMT in 96-well dilution plates. The arrays were washed four times with PBST (0.05 % (v/v) Tween-20 in PBS), before transferring 120 µL of each sample from the dilution plates, and incubated for 2h. Next, slides were washed four times with PBST, before applying 1 µg/mL Alexa Fluor647-Streptavidin (ThermoFisher Scientific, Waltham, MA, USA), in PBSMT and incubated for 1h. Again, slides were washed four times with PBST before being dismounted from the hybridization chambers, quickly immersed in dH₂O, and dried under a stream of N₂. The slides were immediately scanned in a LuxScan 10K Microarray scanner (CapitalBio) at 10 µm resolution using the 635 nm excitation laser for visualizing bound proteins, and the 532 nm excitation laser for visualizing printed antibodies.

*Data acquisition, quality control and pre-processing:* Signal intensities were quantified using the ScanArray Express software version 4.0 (PerkinElmer Life and Analytical Sciences) with the fixed circle option. For each microarray, a grid was positioned using the Alexa Fluor555 signals from microarray printing. The same grid was then used to quantify the Alexa Fluor647 signal corresponding to the relative level of bound protein. Eleven samples (10 PDAC and 1 NC) were not quantified due to poor quality images resulting from of high background and/or low overall signals. For quantified arrays, the spot saturation, mean intensity and signal-to-noise ratio of each spot were evaluated. Fourteen antibodies were excluded because (i) the median signal intensity was below the cut-off limit, defined as the background (average PBS signal) +2 standard deviations (n=8), (ii) saturated signal in the lowest scanner intensity setting in more than 50% of samples (n=1), and (iii) inadequate antibody printing (n=5). Based on the remaining 202 samples and 336 antibodies, a dataset was assembled using the mean spot intensity after local background subtraction. Each data point represented an average of the three replicate spots, unless any replicate CV exceeded 15 % from the mean value, in which case it was dismissed and the average of the two remaining replicates was used instead. The average CV of replicates was 7.9 % (±4.1 %). Applying a cut-off CV of 15 %, 79 % of data values were calculated from all three replicates and the remaining 21 % from two replicates.

The logged data was normalized, using the empirical Bayes algorithm ComBat (22) for adjusting technical variation, followed by a linear scaling of data from each array to adjust for variations in sample background level. The scaling factor was based on the 20 % of antibodies with the lowest standard deviation across all samples and was calculated by dividing the intensity sum of these antibodies on each array with the average sum across all arrays (13, 23).

*Data analysis:* The sample and variable distribution was analyzed and visualized, using a principal component analysis based program (Qlucore, Lund, Sweden). ANOVA was applied for an initial filtering of data. The performance of individual markers was evaluated, using Student's t-test, Benjamini Hochberg procedure for false discovery rate control (q-values), and fold changes. Separation of different subgroups within the data was also assessed, using the support vector machine (SVM) function in R, applying a linear kernel with the cost of constraints set to 1. Models for discriminating two groups were created, using a leave-one-out cross validation procedure. When defining a condensed biomarker signature (body/tail vs head), the antibodies were filtered, using a SVM-based Backward Elimination algorithm which excludes one antibody at the time and iteratively eliminates the antibody that was excluded when the smallest Kullback-Leibler divergence was obtained in the classification analysis (body/tail vs head), as previously described (24). Using the R-package, the performance of the SVM models were assessed, using receiver operating characteristics (ROC) curves and reported as area under the curve (AUC) values.

### Results

### Markers associated with tumour location:

The samples were grouped by the primary tumour location in the pancreas. Backward elimination was used to define the best condensed signature capable of differentiating tumours based on localization, body/tail vs. head. The condensed signature, composed of 37 antibodies, including a core of three antibodies directed against IL-12, STAT1 and PGAM5, is shown in Table 1. The ROC AUC values describing the differentiation is shown for the core signature, and then for adding the biomarkers one by one, is also shown in Table 2. The AUC for the core signature was found to be 0.73, and was 1.0 for the full condensed signature.

Next, additionally important analytes for differentiating tumours located in body/tail vs head was identified by defining differentially expressed biomarkers. To this end, the samples were grouped by the primary tumour location in the pancreas. The AUC for Head (n=63) vs. Body/Tail (n=39) localized tumours was 0.64 (p=5.4e-3). Applying a cut-off of p<0.05, 37 antibodies showed significantly different intensity levels in Head vs. Body/Tail (Table 2).

### Discussion

The biological diversity of tumours due to its localization in pancreatic cancer has been previously demonstrated (42). Tumours in the body/tail of pancreas are rarer than tumour in the head of pancreas (77% of PDAC) (43). Because of differences in e.g. blood supply and lymphatic and venous backflow, there are also differences in the disease presentation with body/tail tumours causing less jaundice, more pain, higher albumin and CEA levels and lower CA19-9 levels (44, 45). Body/tail tumours are more often detected at a later stage than head tumours and have a higher rate of metastasis. As the biological differences can result in different treatment efficiency (46), biomarkers that can discriminate between tumour localization would be of clinical relevance and could pave the way for personalized treatment strategies. However, few differences have been found on a genetic level, with no significant variation in the overall number of mutations, deletions and amplifications, or in K-ras point mutations (42). In the current study, several antibodies identified markers that showed differential protein expression levels between head and body/tail tumours. A condensed signature, based on 37 antibodies, differentiating the groups was defined.

### References

1. Yachida S, Jones S, Bozic I, Antal T, Leary R, Fu B, et al. Distant metastasis occurs late during the genetic evolution of pancreatic cancer. Nature. 2010;467(7319):1114-7.
2. Conlon KC, Klimstra DS, Brennan MF. Long-term survival after curative resection for pancreatic ductal adenocarcinoma. Clinicopathologic analysis of 5-year survivors. Annals of surgery. 1996;223(3):273-9.
3. Sohn TA, Yeo CJ, Cameron JL, Koniaris L, Kaushal S, Abrams RA, et al. Resected adenocarcinoma of the pancreas-616 patients: results, outcomes, and prognostic indicators. Journal of gastrointestinal surgery : official journal of the Society for Surgery of the Alimentary Tract. 2000;4(6):567-79.
4. Furukawa H, Okada S, Saisho H, Ariyama J, Karasawa E, Nakaizumi A, et al. Clinicopathologic features of small pancreatic adenocarcinoma. A collective study. Cancer. 1996;78(5):986-90.
5. Shimizu Y, Yasui K, Matsueda K, Yanagisawa A, Yamao K. Small carcinoma of the pancreas is curable: new computed tomography finding, pathological study and postoperative results from a single institute. Journal of gastroenterology and hepatology. 2005;20(10):1591-4.
6. Gangi S, Fletcher JG, Nathan MA, Christensen JA, Harmsen WS, Crownhart BS, et al. Time interval between abnormalities seen on CT and the clinical diagnosis of pancreatic cancer: retrospective review of CT scans obtained before diagnosis. AJR American journal of roentgenology. 2004;182(4):897-903.
7. Pelaez-Luna M, Takahashi N, Fletcher JG, Chari ST. Resectability of presymptomatic pancreatic cancer and its relationship to onset of diabetes: a retrospective review of CT scans and fasting glucose values prior to diagnosis. The American journal of gastroenterology. 2007;102(10):2157-63.
8. Locker GY, Hamilton S, Harris J, Jessup JM, Kemeny N, Macdonald JS, et al. ASCO 2006 update of recommendations for the use of tumour markers in gastrointestinal cancer. Journal of clinical oncology : official journal of the American Society of Clinical Oncology. 2006;24(33):5313-27.
9. Brand RE, Nolen BM, Zeh HJ, Allen PJ, Eloubeidi MA, Goldberg M, et al. Serum biomarker panels for the detection of pancreatic cancer. Clinical cancer research : an official journal of the American Association for Cancer Research. 2011;17(4):805-16.
10. Bunger S, Laubert T, Roblick UJ, Habermann JK. Serum biomarkers for improved diagnostic of pancreatic cancer: a current overview. Journal of cancer research and clinical oncology. 2011;137(3):375-89.
11. Coussens LM, Werb Z. Inflammation and cancer. Nature. 2002;420(6917):860-7.
12. Carlsson A, Wuttge DM, Ingvarsson J, Bengtsson AA, Sturfelt G, Borrebaeck CA, et al. Serum protein profiling of systemic lupus erythematosus and systemic sclerosis using recombinant antibody microarrays. Molecular & cellular proteomics : MCP. 2011;10(5):M110 005033.
13. Ingvarsson J, Wingren C, Carlsson A, Ellmark P, Wahren B, Engstrom G, et al. Detection of pancreatic cancer using antibody microarray-based serum protein profiling. Proteomics. 2008;8(11):2211-9.
14. Wingren C, Sandstrom A, Segersvard R, Carlsson A, Andersson R, Lohr M, et al. Identification of serum biomarker signatures associated with pancreatic cancer. Cancer research. 2012;72(10):2481-90.
15. Surinova S, Schiess R, Huttenhain R, Cerciello F, Wollscheid B, Aebersold R. On the development of plasma protein biomarkers. Journal of proteome research. 2011;10(1):5-16.
16. Haab BB, Geierstanger BH, Michailidis G, Vitzthum F, Forrester S, Okon R, et al. Immunoassay and antibody microarray analysis of the HUPO Plasma Proteome Project reference specimens: systematic variation between sample types and calibration of mass spectrometry data. Proteomics. 2005;5(13):3278-91.
17. Alonzo TA, Pepe MS, Moskowitz CS. Sample size calculations for comparative studies of medical tests for detecting presence of disease. Statistics in medicine. 2002;21(6):835-52.
18. Wingren C, Borrebaeck CA. Antibody microarray analysis of directly labelled complex proteomes. Current opinion in biotechnology. 2008;19(1):55-61.
19. Steinhauer C, Wingren C, Hager AC, Borrebaeck CA. Single framework recombinant antibody fragments designed for protein chip applications. BioTechniques. 2002;Suppl:38-45.
20. Wingren C, Steinhauer C, Ingvarsson J, Persson E, Larsson K, Borrebaeck CA. Microarrays based on affinity-tagged single-chain Fv antibodies: sensitive detection of analyte in complex proteomes. Proteomics. 2005;5(5):1281-91.
21. Stoevesandt O, Taussig MJ. European and international collaboration in affinity proteomics. New biotechnology. 2012;29(5):511-4.
22. Johnson WE, Li C, Rabinovic A. Adjusting batch effects in microarray expression data using empirical Bayes methods. Biostatistics. 2007;8(1):118-27.
23. Carlsson A, Wingren C, Ingvarsson J, Ellmark P, Baldertorp B, Ferno M, et al. Serum proteome profiling of metastatic breast cancer using recombinant antibody microarrays. Eur J Cancer. 2008;44(3):472-80.
24. Carlsson A, Wingren C, Kristensson M, Rose C, Ferno M, Olsson H, et al. Molecular serum portraits in patients with primary breast cancer predict the development of distant metastases. Proceedings of the National Academy of Sciences of the United States of America. 2011;108(34):14252-7.
25. Itakura J, Ishiwata T, Friess H, Fujii H, Matsumoto Y, Buchler MW, et al. Enhanced expression of vascular endothelial growth factor in human pancreatic cancer correlates with local disease progression. Clinical cancer research : an official journal of the American Association for Cancer Research. 1997;3(8):1309-16.
26. Shaw VE, Lane B, Jenkinson C, Cox T, Greenhalf W, Halloran CM, et al. Serum cytokine biomarker panels for discriminating pancreatic cancer from benign pancreatic disease. Molecular cancer. 2014;13:114.
27. Chen J, Wu W, Zhen C, Zhou H, Yang R, Chen L, et al. Expression and clinical significance of complement C3, complement C4b1 and apolipoprotein E in pancreatic cancer. Oncology letters. 2013;6(1):43-8.
28. Jimenez-Vidal M, Srivastava J, Putney LK, Barber DL. Nuclear-localized calcineurin homologous protein CHP1 interacts with upstream binding factor and inhibits ribosomal RNA synthesis. The Journal of biological chemistry. 2010;285(47):36260-6.
29. Jin Q, Kong B, Yang X, Cui B, Wei Y, Yang Q. Overexpression of CHP2 enhances tumour cell growth, invasion and metastasis in ovarian cancer. In vivo. 2007;21(4):593-8.
30. Honda K, Okusaka T, Felix K, Nakamori S, Sata N, Nagai H, et al. Altered plasma apolipoprotein modifications in patients with pancreatic cancer: protein characterization and multi-institutional validation. PLoS One. 2012;7(10):e46908.
31. Pannala R, Basu A, Petersen GM, Chari ST. New-onset diabetes: a potential clue to the early diagnosis of pancreatic cancer. The Lancet Oncology. 2009;10(1):88-95.
32. Feldmann G, Mishra A, Bisht S, Karikari C, Garrido-Laguna I, Rasheed Z, et al. Cyclin-dependent kinase inhibitor Dinaciclib (SCH727965) inhibits pancreatic cancer growth and progression in murine xenograft models. Cancer biology & therapy. 2011;12(7):598-609.
33. Xia C, Ma W, Stafford LJ, Liu C, Gong L, Martin JF, et al. GGAPs, a new family of bifunctional GTP-binding and GTPase-activating proteins. Molecular and cellular biology. 2003;23(7):2476-88.
34. Hou J, Xu J, Jiang R, Wang Y, Chen C, Deng L, et al. Estrogen-sensitive PTPRO expression represses hepatocellular carcinoma progression by control of STAT3. Hepatology. 2013;57(2):678-88.
35. Motiwala T, Kutay H, Ghoshal K, Bai S, Seimiya H, Tsuruo T, et al. Protein tyrosine phosphatase receptor-type O (PTPRO) exhibits characteristics of a candidate tumour suppressor in human lung cancer. Proceedings of the National Academy of Sciences of the United States of America. 2004;101(38):13844-9.
36. Huang YT, Li FF, Ke C, Li Z, Li ZT, Zou XF, et al. PTPRO promoter methylation is predictive of poorer outcome for HER2-positive breast cancer: indication for personalized therapy. Journal of translational medicine. 2013;11:245.
37. Song MS, Salmena L, Carracedo A, Egia A, Lo-Coco F, Teruya-Feldstein J, et al. The deubiquitinylation and localization of PTEN are regulated by a HAUSP-PML network. Nature. 2008;455(7214):813-7.
38. Zhao GY, Lin ZW, Lu CL, Gu J, Yuan YF, Xu FK, et al. USP7 overexpression predicts a poor prognosis in lung squamous cell carcinoma and large cell carcinoma. Tumour biology : the journal of the International Society for Oncodevelopmental Biology and Medicine. 2014.
39. Winter JM, Tang LH, Klimstra DS, Brennan MF, Brody JR, Rocha FG, et al. A novel survival-based tissue microarray of pancreatic cancer validates MUC1 and mesothelin as biomarkers. PLoS One. 2012;7(7):e40157.
40. Bellone G, Smirne C, Mauri FA, Tonel E, Carbone A, Buffolino A, et al. Cytokine expression profile in human pancreatic carcinoma cells and in surgical specimens: implications for survival. Cancer immunology, immunotherapy : CII. 2006;55(6):684-98.
41. Gabitass RF, Annels NE, Stocken DD, Pandha HA, Middleton GW. Elevated myeloid-derived suppressor cells in pancreatic, esophageal and gastric cancer are an independent prognostic factor and are associated with significant elevation of the Th2 cytokine interleukin-13. Cancer immunology, immunotherapy : CII. 2011;60(10):1419-30.
42. Ling Q, Xu X, Zheng SS, Kalthoff H. The diversity between pancreatic head and body/tail cancers: clinical parameters and in vitro models. Hepatobiliary & pancreatic diseases international : HBPD INT. 2013;12(5):480-7.
43. Lau MK, Davila JA, Shaib YH. Incidence and survival of pancreatic head and body and tail cancers: a population-based study in the United States. Pancreas. 2010;39(4):458-62.
44. Watanabe I, Sasaki S, Konishi M, Nakagohri T, Inoue K, Oda T, et al. Onset symptoms and tumour locations as prognostic factors of pancreatic cancer. Pancreas. 2004;28(2):160-5.
45. Eyigor C, Karaca B, Kuzeyli-Yildirim Y, Uslu R, Uyar M, Coker A. Does the tumour localization in advanced pancreatic cancer have an influence on the management of symptoms and pain? Journal of BUON : official journal of the Balkan Union of Oncology. 2010;15(3):543-6.
46. Wu TC, Shao YF, Shan Y, Wu JX, Zhao P. [Surgical effect of malignant tumour of body and tail of the pancreas: compare with pancreatic head cancer]. Zhonghua wai ke za zhi [Chinese journal of surgery]. 2007;45(1):30-3.
47. Gupta S, Venkatesh A, Ray S, Srivastava S. Challenges and prospects for biomarker research: a current perspective from the developing world. Biochimica et biophysica acta. 2014;1844(5):899-908.
48. Rastogi T, Hildesheim A, Sinha R. Opportunities for cancer epidemiology in developing countries. Nature reviews Cancer. 2004;4(11):909-17.
49. Parker LA, Porta M, Lumbreras B, Lopez T, Guarner L, Hernandez-Aguado I, et al. Clinical validity of detecting K-ras mutations for the diagnosis of exocrine pancreatic cancer: a prospective study in a clinically-relevant spectrum of patients. European journal of epidemiology. 2011;26(3):229-36.
50. Porta M, Malats N, Jariod M, Grimalt JO, Rifa J, Carrato A, et al. Serum concentrations of organochlorine compounds and K-ras mutations in exocrine pancreatic cancer. PANKRAS II Study Group. Lancet. 1999;354(9196):2125-9.

**Table 1: Backward elimination, defining a condensed signature differentiating tumours based on location (body/tail vs head)**

| ***Order of elimir*** | ***smallestErrorP*** | ***Antibody*** | ***Uniprot entry ID*** | ***Full antigen name*** | ***Publication name*** |
|---|---|---|---|---|---|
| 333 | NA | IL-12-54 | P29459/60 | Interleukin-12 | IL-12 (2) |
| 332 | 76,9508035 | C-STAT1-2 | P42224 | Signal transducer and activator of transcription 1-alpha/beta | STAT1 (1) |
| 331 | 67,2133979 | I-PGAM5-3 | Q96HS1 | Serine/threonine-protein phosphatase PGAM5, mitochondrial | PGAM5 (3) |
| 330 | 62,39696715 | C-UCHL5-1 | Q9Y5K5 | Ubiquitin carboxyl-terminal hydrolase isozyme L5 | UCHL5 |
| 329 | 57,45193012 | oxy_2 | Q9H4L5 | Oxysterol-binding protein-related protein 3 | ORP-3 (2) |
| 328 | 57,56668957 | IL-7-31 | P13232 | Interleukin-7 | IL-7 (2) |
| 327 | 53,61853414 | ATP5B_3 | P06576 | ATP synthase subunit beta, mitochondrial | ATP-5B (3) |
| 326 | 56,80894214 | CHX10_3 | P58304 | Visual system homeobox 2 | CHX10 (3) |
| 325 | 53,72049779 | KIA_G4 | Q6ZT07 | TBC1 domain family member 9 | TBC1D9 (1) |
| 324 | 50,80419368 | I-MD2L1-2 | Q13257 | Mitotic spindle assembly checkpoint protein MAD2A | HsMAD2 (1) |
| 323 | 48,76074331 | HLA-DR/DP | P01903/P01911/P79483/P13762/Q30154/P20036/P04440 | | HLA-DR/DP |
| 322 | 46,52775576 | I-PRD14-2 | Q9GZV8 | PR domain zinc finger protein 14 | PRD14 (2) |
| 321 | 45,2275185 | I-SPDLY-1 | Q96EA4 | Protein Spindly | hSpindly (1) |
| 320 | 43,92896544 | I-MD2L1-3 | Q13257 | Mitotic spindle assembly checkpoint protein MAD2A | HsMAD2 (2) |
| 319 | 41,87125871 | IL-3-58 | P08700 | Interleukin-3 | IL-3 (1) |
| 318 | 40,89489309 | IL-1a-145 | P01583 | Interleukin-1 alpha | IL-1α (1) |
| 317 | 39,54974253 | I-NDC80-2 | 014777 | Kinetochore protein NDC80 homolog | HsHec1 (1) |
| 316 | 37,10317265 | C-OSTP-3 | P10451 | Osteopontin | Osteopontin (3) |
| 315 | 34,70505536 | MCP-1-1 | P13500 | C-C motif chemokine 2 | MCP-1 (3) |
| 314 | 33,89539191 | C-CDK2-1 | P24941 | Cyclin-dependent kinase 2 | CDK-2 (2) |
| 313 | 30,86338891 | C1s-8 | P09871 | Complement C1s | C1s |
| 312 | 33,26761341 | D-Her2-22 | P04626 | Receptor tyrosine-protein kinase erbB-2 | Her2/ErbB2 (3) |
| 311 | 33,74360213 | IL-6-21 | P05231 | Interleukin-6 | IL-6 (3) |
| 310 | 33,1840744 | IgM-4 | N/A | N/A | IgM (4) |
| 309 | 35,56098202 | Integrin a-10 | O75578 | Integrin alpha-10 | Integrin a10 |
| 308 | 31,52235506 | I-PTPRJ-7 | Q12913 | Receptor-type tyrosine-protein phosphatase eta | R-PTP-eta (7) |
| 307 | 30,3979861 | Lewis X-2 | N/A | N/A | Lewis^{X} (2) |
| 306 | 30,82001962 | I-GRIP2-4 | Q9C0E4 | Glutamate receptor-interacting protein 2 | GRIP-2 (4) |
| 305 | 31,51176605 | C-PTK6-1 | Q13882 | Protein-tyrosine kinase 6 | PTK6 |
| 304 | 30,62409564 | P3-15 | P15941 | Mucin-1 | MUC1 (4) |
| 303 | 29,48152534 | I-PRD14-1 | Q9G7V8 | PR domain zinc finger protein 14 | PRD14 (1) |
| 302 | 25,74003763 | C-PTPN1-3 | P18031 | Tyrosine-protein phosphatase non-receptor type 1 | PTPN1 (2) |
| 301 | 25,98991287 | C4_022_B02 | P0COL4/5 | Complement C4 | C4 (3) |
| 300 | 29,94162743 | Prop-3 | P27918 | Properdin | Properdin |
| 299 | 29,46495477 | C-GAK-1 | Q5U4P5 | GAK protein | GAK (1) |
| 298 | 25,32187898 | TNF-b-1 | P01374 | Lymphotoxin-alpha | TNP-β (1) |
| 297 | 25,57403136 | IL-5-21 | P05113 | Interleukin-5 | IL-5 (3) |

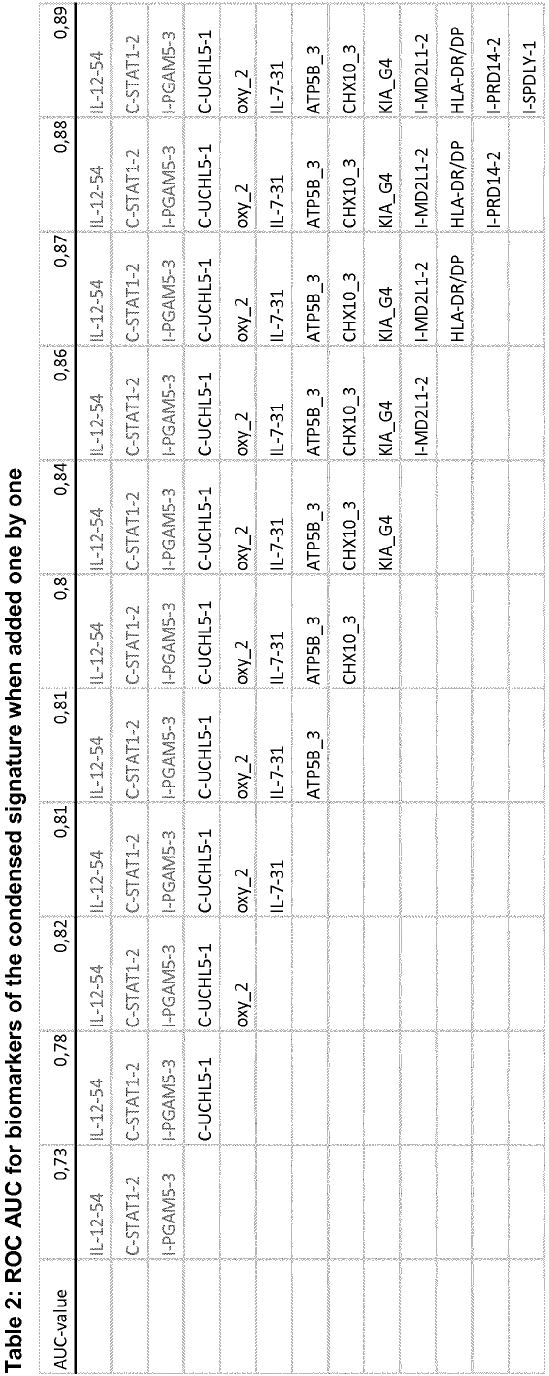

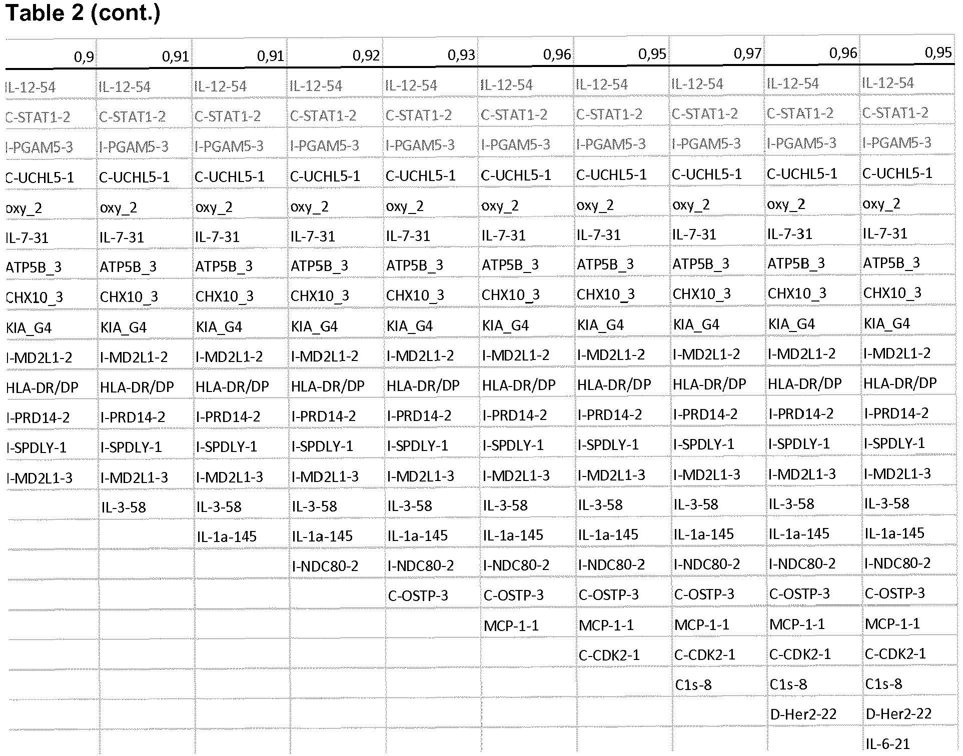

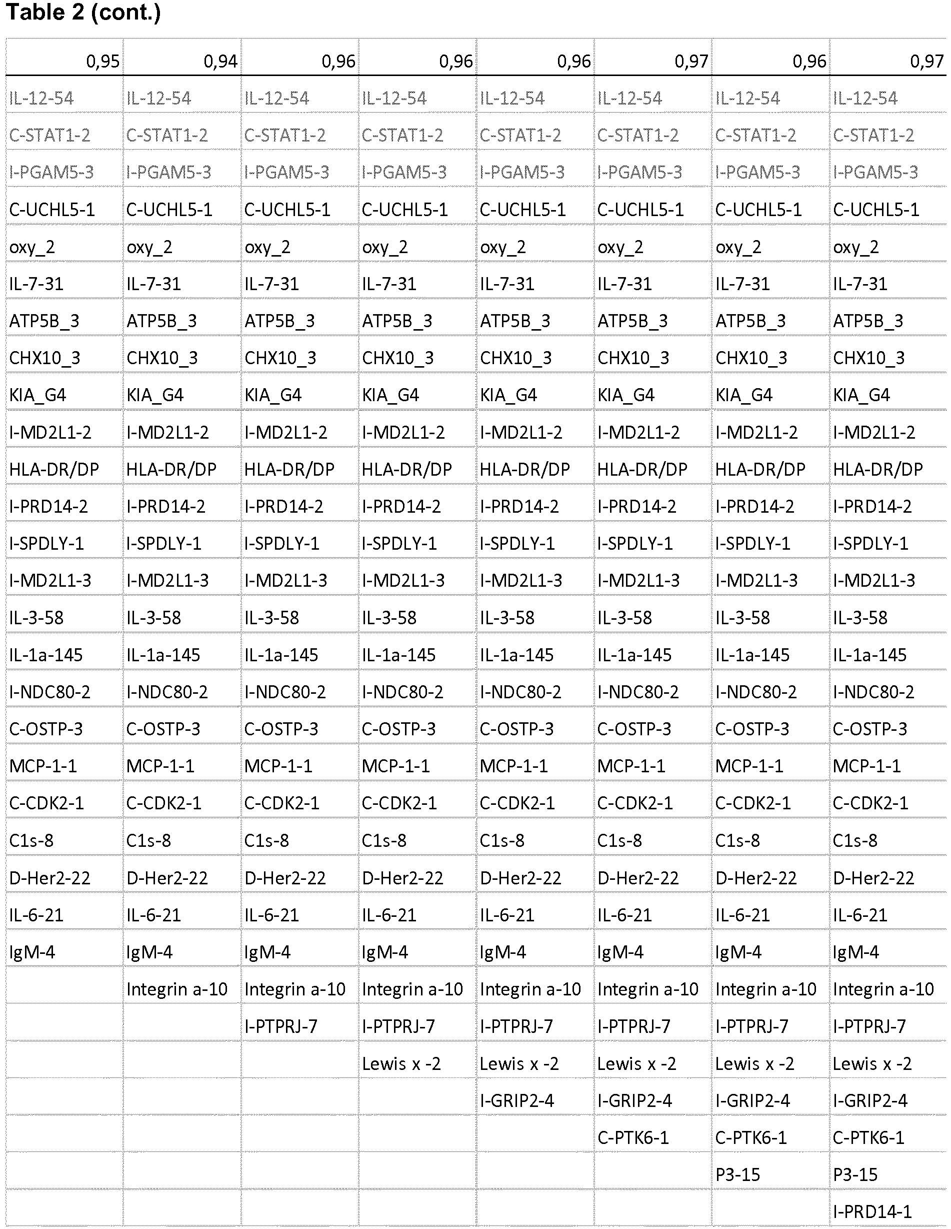

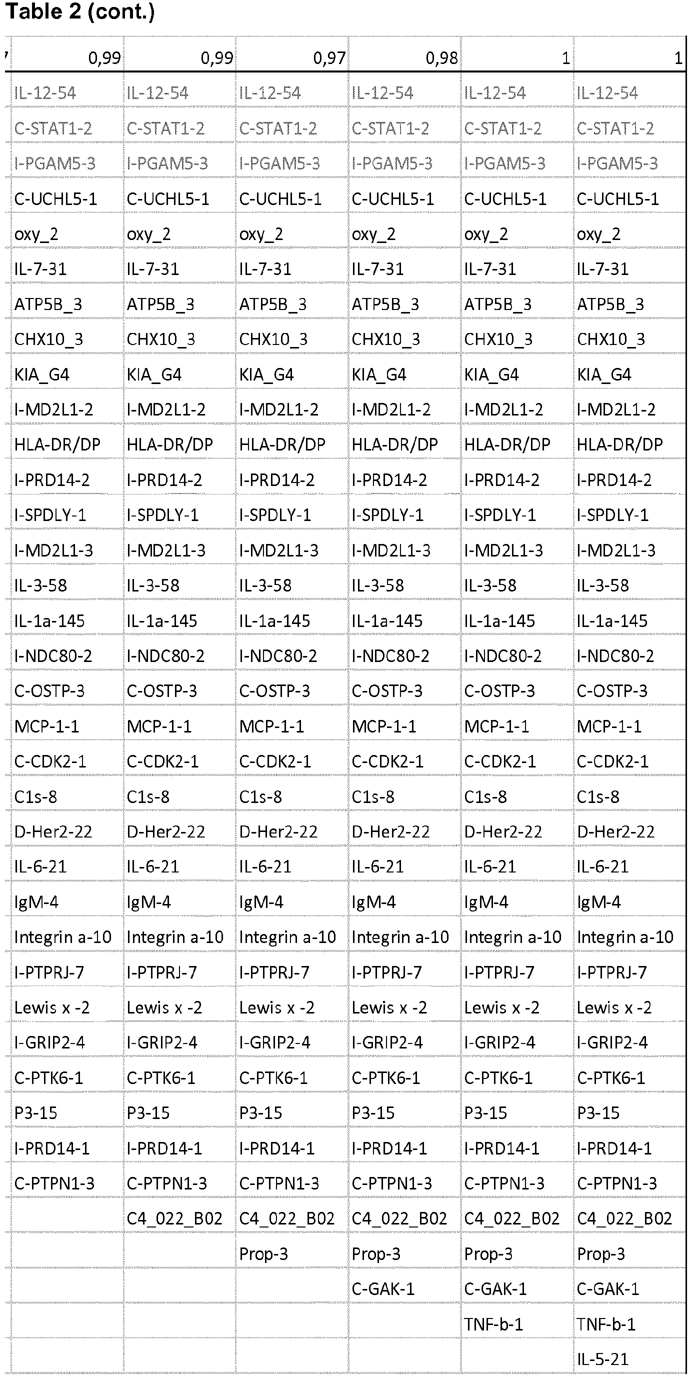

**Table 3: Differentially expressed markers when grouping the tumours based on location (body/tail vs head)**

| SIGNIFICANT (p<0.05) ANTIBODIES FOR BODY-TAIL VS HEAD | | | | | | |
|---|---|---|---|---|---|---|
| ***Antibody name*** | ***Uniprot entry ID*** | ***Full antigen name*** | ***Publication name*** | ***Fold change*** | ***Wilcox p-value*** | ***BH Q-value*** |
| ApoA1_001_C08 | P02647 | Apolipoprotein A1 | Apo-A1 (1) | 1,127956708 | 0,019939682 | 0,327105216 |
| APOA4_3 | P06727 | Apolipoprotein A4 | Apo-A4 (1) | 0,881452663 | 0,030601529 | 0,327105216 |
| APOA4_5 | P06727 | Apolipoprotein A4 | Apo-A4 (3) | 0,897489907 | 0,027076146 | 0,327105216 |
| BTK | Q06187 | Tyrosine-protein kinase BTK | BTK (1) | 0,911614891 | 0,011274875 | 0,266195034 |
| C3_016_A06 | P01024 | Complement C3 | C3 (3) | 1,133372415 | 0,044354705 | 0,327105216 |
| C3_019_G12 | P01024 | Complement C3 | C3 (6) | 1,120903515 | 0,028541635 | 0,327105216 |
| C5-9 | P01031 | Complement C5 | C5 (2) | 1,159380602 | 0,010214199 | 0,266195034 |
| C-BTK-2 | Q06187 | Tyrosine-protein kinase BTK | BTK (3) | 0,922634192 | 0,003135718 | 0,266195034 |
| C-BTK-3 | Q06187 | Tyrosine-protein kinase BTK | BTK (4) | 0,902986966 | 0,006947987 | 0,266195034 |
| CD40L | P29965 | CD40 ligand | CD40 ligand | 0,892371302 | 0,01001305 | 0,266195034 |
| C-KSYK-2 | P43405 | Tyrosine-protein kinase SYK | KSYK (2) | 0,914311324 | 0,039501381 | 0,327105216 |
| C-STAP2-1 | Q9UGK3 | Signal-transducing adaptor protein 2 | STAP2 (1) | 1,082961427 | 0,008526774 | 0,266195034 |
| C-TNFRSF3-2 | P36941 | Tumor necrosis factor receptor superfamily member 3 | TNFRSF3 (2) | 0,924564729 | 0,030601529 | 0,327105216 |
| C-UCHL5-1 | Q9Y5K5 | Ubiquitin carboxyl-terminal hydrolase isozyme L5 | UCHL5 | 0,913114753 | 0,001270036 | 0,266195034 |
| CystC_001_A11 | P01034 | Cystatin-C | Cystatin C (3) | 0,915729347 | 0,032229074 | 0,327105216 |
| CystC_002_B02 | P01034 | Cystatin-C | Cystatin C (2) | 0,907933657 | 0,03631742 | 0,327105216 |
| FB-7 | P00751 | Complement factor B | Factor B (1) | 1,159472427 | 0,02348161 | 0,327105216 |
| GLP-1R | P43220 | Glucagon-like peptide 1 receptor | GLP-1 R | 0,92851089 | 0,033930236 | 0,327105216 |
| IgM-1 | N/A | N/A | IgM (1) | 0,913178107 | 0,038200439 | 0,327105216 |
| IgM-4 | N/A | N/A | IgM (4) | 0,897498152 | 0,019219138 | 0,327105216 |
| I-GNAI3-5 | P08754 | Guanine nucleotide-binding protein G(k) subunit alpha | GNAI3 (2) | 0,927396373 | 0,038200439 | 0,327105216 |
| IL-10-32 | P22301 | Interleukin-10 | IL-10 (1) | 0,90413138 | 0,012675954 | 0,266195034 |
| IL-16-5 | Q14005 | Interleukin-16 | IL-16 (1) | 0,918919649 | 0,005289629 | 0,266195034 |
| IL-1b-2 | P01584 | Interleukin-1 beta | IL-Iβ (2) | 0,93527344 | 0,046583769 | 0,327105216 |
| IL-1-ra-65 | P18510 | Interleukin-1 receptor antagonist protein | IL-1ra (3) | 0,902229182 | 0,008355592 | 0,266195034 |
| IL-6-21 | P05231 | Interleukin-6 | IL-6 (3) | 0,907882964 | 0,036936106 | 0,327105216 |
| IL-7-31 | P13232 | Interleukin-7 | IL-7 (2) | 0,906130718 | 0,011957233 | 0,266195034 |
| I-MAP2K6-3 | P52564 | Dual specificity mitogen-activated protein kinase kinase 6 | MAPKK 6 (2) | 0,911077427 | 0,048121604 | 0,327105216 |
| I-MAP2K6-7 | P52564 | Dual specificity mitogen-activated protein kinase kinase 6 | MAPKK 6 (4) | 0,938310967 | 0,047347436 | 0,327105216 |
| I-MAPK9-3 | P45984 | Mitogen-activated protein kinase 9 | MAPK9 (2) | 0,929911203 | 0,033930236 | 0,327105216 |
| I-NDC80-2 | O14777 | Kinetochore protein NDC80 homolog | HsHec1 (1) | 0,924355128 | 0,024339054 | 0,327105216 |
| I-PTPRO-4 | Q16827 | Receptor-type tyrosine-protein phosphatase O | R-PTP-O (2) | 0,921525326 | 0,008701086 | 0,266195034 |
| JAK3 | P52333 | Tyrosine-protein kinase JAK3 | JAK3 | 0,919028602 | 0,009243285 | 0,266195034 |
| KIA_H3 | Q6ZT07 | TBC1 domain family member 9 | TBC1D9 (2) | 0,917198457 | 0,015355447 | 0,303495897 |
| Lewis x -2 | N/A | N/A | Lewis^{X} (2) | 0,924133254 | 0,040165833 | 0,327105216 |
| MCP1_005_A11 | P13500 | C-C motif chemokine 2 | MCP-1 (8) | 0,879808595 | 0,032229074 | 0,327105216 |
| MCP-3-2 | P80098 | C-C motif chemokine 7 | MCP-3 (2) | 0,909805618 | 0,012675954 | 0,266195034 |
| MYOM2_1 | P54296 | Myomesin-2 | MYOM2 (2) | 1,194887415 | 0,007240947 | 0,266195034 |
| oxy_1 | Q9H4L5 | Oxysterol-binding protein-related protein 3 | ORP-3 (1) | 0,937065178 | 0,029556151 | 0,327105216 |
| P3-06 | P15941 | Mucin-1 | MUC1 (2) | 0,936348013 | 0,04291922 | 0,327105216 |
| RANTES-1 | P13501 | C-C motif chemokine 5 | RANTES (1) | 0,924874699 | 0,049701869 | 0,327105216 |
| Sialyl Lewis x | N/A | N/A | Sialle x | 0,940195431 | 0,009621306 | 0,266195034 |
| Smuc-159 | P15941 | Mucin-1 | MUC1 (1) | 0,920160352 | 0,042216331 | 0,327105216 |
| TGF-bl-34 | P01137 | Transforming growth factor beta-1 | TGF-β1 (3) | 0,908217903 | 0,023906998 | 0,327105216 |
| TGF-b1-64 | P01137 | Transforming growth factor beta-1 | TGF-β1 (1) | 0,923512121 | 0,031678505 | 0,327105216 |
| TNF-b-3 | P01374 | Lymphotoxin-alpha | TNP-β (2) | 0,911743101 | 0,042216331 | 0,327105216 |

**Table I. Clinical Samples**

| *Group* | *No of samples* | *M*/*F* | *Median age (range)* |
|---|---|---|---|
| **PDAC** | **118** | **76/42** | **59 (21-83)** |
| **NC** | **95** | **20/75** | **63 (52-74)** |
| *Total* | *213* | *96*/*117* | *62 (21-83)* |

**Table II. Antibody specificities**

| *Antigen* | *Full name* | *No of scFvs* |
|---|---|---|
| AGAP-2 | Arf-GAP with GTPase, ANK repeat and PH-dom.-containing protein 2 | 4 |
| Apo-A1 | Apolipoprotein A1 | 3 |
| Apo-A4 | Apolipoprotein A4 | 3 |
| ATP-5B | ATP synthase subunit beta | 3 |
| BTK | Tyrosine-protein kinase BTK | 4 |
| C1 inh. | C1 esterase inhibitor | 4 |
| C1q* | Complement C1q | 1 |
| C1s | Complement C1s | 1 |
| C3* | Complement C3 | 6 |
| C4* | Complement C4 | 4 |
| C5* | Complement C5 | 3 |
| CD40 | CD40 protein | 4 |
| CD40L | CD40 ligand | 1 |
| CDK-2 | Cyclin-dependent kinase 2 | 2 |
| CHP-1 | Calcineurin B homologous protein 1 | 2 |
| CHX-10 | Visual system homeobox 2 | 3 |
| CIMS-10 | Selection motif TEEQLK | 1 |
| CIMS-13 | Selection motif SSAYSR | 1 |
| CIMS-5 | Selection motif WTRNSNMNYWLIIRL | 1 |
| CK19 | Cytokeratin 19 | 3 |
| CT17 | Cholera toxin subunit B | 1 |
| CystC | Cystatin C | 4 |
| Digoxin | Digoxin | 1 |
| DUSP-9 | Dual specificity protein phosphatase 9 | 1 |
| EGFR | Epidermal growth factor receptor | 1 |
| Eotaxin | Eotaxin | 3 |
| ErbB-2 | Receptor tyrosine-protein kinase erbB-2 | 4 |
| Factor B* | Complement factor B | 4 |
| FASN | Fatty acid synthase | 4 |
| GAK | Cyclin G-associated kinase | 3 |
| GEM | GTP-binding protein GEM | 2 |
| GLP-1 | Glucagon-like peptide-1 | 1 |
| GM-CSF | Granulocyte-macrophage colony-stimulating factor | 6 |
| GNAI-3 | Guanine nucleotide-binding protein G(k) subunit alpha | 4 |
| GRIP-2 | Glutamate receptor-interacting protein 2 | 8 |
| HADH-2 | 3-hydroxyacyl-CoA dehydrogenase type-2 | 4 |
| HLA-DR/DP | HLA-DR/DP | 1 |
| ICAM-1 | Intercellular adhesion molecule 1 | 1 |
| | | |
| IFN-g | Interferon gamma | 3 |
| IgM | Immunoglobulin M | 5 |
| IL-10* | Interleukin 10 | 3 |
| IL-11 | Interleukin 11 | 3 |
| IL-12* | Interleukin 12 | 4 |
| IL-13* | Interleukin 13 | 3 |
| IL-16 | Interleukin 16 | 3 |
| IL-18 | Interleukin 18 | 3 |
| IL-1a* | Interleukin 1 alpha | 3 |
| IL-1b | Interleukin 1 beta | 3 |
| IL-1ra | Interleukin-1 receptor antagonist protein | 3 |
| IL-2 | Interleukin 2 | 3 |
| IL-3 | Interleukin 3 | 3 |
| IL-4* | Interleukin 4 | 4 |
| IL-5* | Interleukin 5 | 3 |
| IL-6* | Interleukin 6 | 4 |
| IL-7 | Interleukin 7 | 2 |
| IL-8* | Interleukin 8 | 3 |
| IL-9 | Interleukin 9 | 3 |
| Integrin a-10 | Integrin alpha-10 | 1 |
| Integrin a-11 | Integrin alpha-11 | 1 |
| JAK3 | Tyrosine-protein kinase JAK3 | 1 |
| | | |

| *Antigen* | *Full name* | *No of scFvs* |
|---|---|---|
| KRAS | GTPase KRas | 1 |
| KSYK | Tyrosine-protein kinase SYK | 2 |
| LDL | Low-density Lipoprotein | 2 |
| Leptin | Leptin | 1 |
| Lewis x | Lewis X | 2 |
| Lewis y | Lewis y | 1 |
| LUM | Lumican | 1 |
| MAD2L-1 | Mitotic arrest deficient 2-like protein 1 | 3 |
| MAP2K-2 | Mitogen-activated protein kinase kinase 2 | 3 |
| MAP2K-6 | Mitogen-activated protein kinase kinase 6 | 4 |
| MAPK-1 | Mitogen-activated protein kinase 1 | 4 |
| MAPK-8 | Mitogen-activated protein kinase 8 | 3 |
| MAPK-9 | Mitogen-activated protein kinase 9 | 6 |
| MATK | Megakaryocyte-associated tyrosine-protein kinase | 3 |
| MCP-1 * | Monocyte chemotactic protein 1 | 9 |
| MCP-3 | Monocyte chemotactic protein 3 | 7 |
| MCP-4 | Monocyte chemotactic protein 4 | 3 |
| MUC-1 | Mucin 1 | 6 |
| Myom-2 | Myomesin-2 | 2 |
| NDC80 | Kinetochore protein NDC80 homolog | 3 |
| ORP-3 | Oxysterol-binding protein-related protein 3 | 2 |
| OSTP | Osteopontin | 3 |
| P85A | PI3-kinase subunit p85-alpha | 3 |
| PAK-7 | Serine/threonine-protein kinase PAK 7 | 3 |
| PAR-6B | Partitioning defective 6 homolog beta | 2 |
| PARP-1 | Poly [ADP-ribose] polymerase 1 | 1 |
| PGAM-5 | Phosphoglycerate mutase family member 5 | 4 |
| PKB gamma | RAC-gamma serine/threonine-protein kinase | 2 |
| PRD-14 | PR domain zinc finger protein 14 | 5 |
| Procath W | Procathepsin W | 1 |
| Properdin* | Properdin | 1 |
| PSA | Prostate-specific antigen | 1 |
| PTK-6 | Protein-tyrosine kinase 6 | 1 |
| PTPN-1 | Tyrosine-protein phosphatase non-receptor type 1 | 3 |
| PTPRJ | Protein-tyrosine phosphatase receptor type J | 8 |
| PTPRK | Protein-tyrosine phosphatase kappa | 8 |
| PTPRO | Protein tyrosine phosphatase U2 | 4 |
| PTPRT | Protein tyrosine phosphatase rho | 3 |
| | | |
| RANTES | RANTES | 3 |
| RPS6KA2 | Ribosomal protein S6 kinase alpha-2 | 3 |
| Sialle x | Sialyl Lewis x | 1 |
| Sox11A | Transcription factor SOX-11 | 1 |
| SPDLY-1 | Spindly | 2 |
| STAP-2 | Signal-transducing adaptor protein 2 | 4 |
| STAT-1 | Signal transducer and activator of transcription 1-alpha/beta | 2 |
| TBC1D-9 | TBC1 domain family member 9 | 3 |
| TENS-4 | Tensin 4 | 1 |
| TGF-b1 | Transforming growth factor beta-1 | 3 |
| TM peptide | Transmembrane peptide | 1 |
| TNF-a | Tumour necrosis factor | 3 |
| TNF-b* | Lymphotoxin-alpha | 4 |
| TNFRSF-14 | Tumour necrosis factor receptor superfamily member 14 | 2 |
| TNFRSF-3 | Tumour necrosis factor receptor superfamily member 3 | 3 |
| TOPBP-1 | DNA topoisomerase 2-binding protein 1 | 2 |
| UBC-9 | Ubiquitin carrier protein 9 | 3 |
| UBE2C | Ubiquitin-conjugating enzyme E2 C | 2 |
| UCHL5 | Ubiquitin carboxyl-terminal hydrolase isozyme L5 | 1 |
| UPF3B | Regulator of nonsense transcripts 3B | 2 |
| USP-7 | Ubiquitin-specific-processing protease 7 | 4 |
| VEGF* | Vascular endothelial growth factor | 4 |

| | | |
|---|---|---|
| *Specificity determined by protein arrays, cytokine arrays, ELISA, blocking/spiking experiments and/or mass spectrometry. | | |

### EXAMPLE B

### ABSTRACT

**Background:** Pancreatic ductal adenocarcinoma (PDAC) is an aggressive disease with rapid tumour progression and poor prognosis.

**Methods:** To mimic a real life test situation, a multicenter trial comprising a serum sample cohort, including 338 patients with either PDAC, other pancreatic diseases (OPD) or controls with non-pancreatic conditions (NPC), were analyzed on 293-plex recombinant antibody microarrays targeting immunoregulatory and cancer-associated antigens.

**Results:** We have identified protein profiles associated with the location of the primary tumour in the pancreas.

### INTRODUCTION

Pancreatic ductal adenocarcinoma (PDAC) is the 4th most common cancer-related cause of death (Siegel *et al,* 2012). Multiple factors account for its poor prognosis and early diagnosis provides today the only possibility for cure. PDAC is often detected at late stages with 80% of patients not eligible for surgery due to either locally advanced or metastatic disease (Hidalgo, 2010; Porta *et al,* 2005; Siegel *et al,* 2012).

### MATERIAL AND METHODS

### Samples

This retrospective study analyzed 338 serum samples from patients with PDAC (n=156), other pancreatic disease (OPD) (n=152), and controls (NPC) (n=30) that were collected after local ethical approval and informed consent at five different hospitals in Spain (Hospital del Mar, Barcelona; Hospital Vall Hebron, Barcelona; Hospital Mútua de Terrassa, Terrassa; Hospital Son Dureta, Palma de Mallorca; Hospital General Universitario de Elche, Elche), as part of the PANKRAS II study (Parker *et al,* 2011; Porta *et al,* 1999) from 1992-1995 (Table 1). The study included patients with a suspicion of PDAC managed in the participating hospitals, and one sample drawn from each patient, using standardized protocols. A panel of experts validated by consensus the final diagnosis of all patients through a careful revision of clinical and pathological records and follow-up information (Porta *et al,* 2000). NPC control patients were mainly attended in the services of general surgery & digestive and traumatology of the participant hospitals, mostly including orthopedic fractures and hernias (Table 1, footnote). Samples were collected before any treatment was given, separated within 3h and stored as 1 mL aliquots at -80°C. The entire set of samples was labelled at a single occasion, using a previously optimized protocol (Carlsson *et al,* 2010; Wingren *et al,* 2007). Briefly, crude samples were diluted 1:45 in PBS, resulting in an approximate protein concentration of 2 mg/mL, and labelled with a 15:1 molar excess of biotin to protein, using 0.6 mM EZ-Link Sulfo-NHS-LC-Biotin (Pierce, Rockford, IL, USA). Unbound biotin was removed by dialysis against PBS for 72 hours. Labelled samples were aliquoted and stored at -20°C.

### Antibodies

The antibody microarrays contained 293 human recombinant scFv antibodies directed against 98 known antigens and 31 peptides motifs (Olsson *et al,* 2012). Most antibodies were selected against immunoregulatory proteins and have previously demonstrated robust on-chip functionality (Steinhauer *et al,* 2002; Wingren & Borrebaeck, 2008; Wingren *et al,* 2005). Several binders have also been validated, using ELISA, mass spectrometry, spiking and/or blocking experiments (Supplementary Table I). In addition, 76 scFvs targeting 28 additionally antigens were selected from the Hell-11 phage display library (Säll et al, manuscript in preparation) against predominantly cancer-associated targets, including kinases and other enzymes, transcriptional regulators, cytokines, and receptors. Although these binders have not previously been used in microarray applications, their on-chip functionallity has been demonstrated in an independent study (Säll et al, manuscript in preparation). The antibodies were produced in *E. coli and* purified from the periplasm, using a MagneHis Protein Purification system (Promega, Madison, WI, USA). The elution buffer was exchanged for PBS, using Zeba 96-well desalt spin plates (Pierce). The protein yield was measured using NanoDrop (Thermo Scientific, Wilmington, DE, USA) and the purity was checked using 10% SDS-PAGE (Invitrogen, Carlsbad, CA, USA).

### Antibody microarrays

Antibody microarrays were produced on black MaxiSorp slides (NUNC, Roskilde, Denmark), using a non-contact printer (SciFlexarrayer S11, Scienion, Berlin, Germany). Thirteen identical subarrays were printed on each slide, each array consisting of 33x31 spots (130 µm spot diameter) with 200 µm spot-to-spot center distance. Each subarray consisted of 3 segments, separated by rows of labelled BSA (Supplementary Fig. 1) and each antibody was printed in 3 replicates, one in each segment and in different segment positions for each replicate. For each round of analysis, 8 slides (104 arrays), were printed overnight and the slides were used for array analysis the following day. All samples were blindly analyzed over the course of 5 consecutive days.

Each slide was mounted in a hybridization gasket (Schott, Jena, Germany) and blocked with PBSMT (1% (w/v) milk, 1% (v/v) Tween-20 in PBS) for 1h. Meantime, aliquots of labelled serum samples were thawed on ice and diluted 1:10 in PBSMT. The slides were washed 4 times with PBST (0.05% (v/v) Tween-20 in PBS) before 120 µL of the samples were added. Samples were incubated for 2h on a rocking table, slides washed 4 times with PBST, incubated with 1 µg/mL Streptavidin-Alexa in PBSMT for 1h on a rocking table, and again washed 4 times with PBST. Finally, the slides were dismounted from the hybridization chambers, quickly immersed in dH₂O, and dried under a stream of N₂. The slides were immediately analyzed, using a confocal microarray scanner (PerkinElmer Life and Analytical Sciences, Wellesley, MA, USA) at 10 µm resolution, using 60% PMT gain and 90% laser power. Signal intensities were quantified, using the ScanArray Express software version 4.0 (PerkinElmer Life and Analytical Sciences) with the fixed circle option. After local background subtraction, intensity values were used for data analysis. Data acquisition was performed by a trained member of the research team and blinded to the sample classification and clinical data.

### Data pre-processing

An average of the 3 replicate spots was used, unless any replicate CV exceeded 15% from the mean value, in which case it was dismissed and the average of the 2 remaining replicates was used instead. The average CV of replicates was 8.3% (±5.5%). Applying a cut-off CV of 15%, 70% of data values were calculated from all 3 replicates and the remaining 30% from 2 replicates.

For evaluation of normalization strategies and data distribution, the data was visualized using 3D principal component analysis (PCA) with ANOVA filtering (Qlucore AB, Lund, Sweden). Two samples (OPD) were excluded as barely any signals were obtained from them for reasons that were not further explored. Of note, PCA on log10 raw data showed no significant (p<0.01) differences between: i) sample subarray positioning on slide, ii) patient gender, iii) patient age, and iv) participating clinical centre. Minor systematic differences were observed between days of analysis (rounds 1-5, likely due to small differences in humidity during array printing, in particular for day 1), which could be neutralized by normalization. The data was normalized in two steps. First, differences between rounds (days) of analysis was eliminated, using a subtract group mean strategy (Wu & Wooldridge, 2005). The average intensity from each antibody was calculated within each round of analysis and subtracted from the single values, thus zero-centering the data. The global mean signal from each antibody was added to each respective data point to avoid negative values. Second, array-to-array differences (e.g. inherent sample background fluorescence differences) were handled by calculating a scaling factor for each subarray, based on the 20% of antibodies with the lowest CV, as has been previously described (Carlsson *et al,* 2008; Ingvarsson *et al,* 2008). Normalization of data was visualized in PCA plots).

### Data analysis

Two-group comparisons were performed using PCA, Student's t-test, Benjamini Hochberg procedure for false discovery rate control (q-values), and fold changes. A group ANOVA was also performed (Qlucore). SVM analysis was performed in R, using a linear kernel with the cost of constraints set to 1

### RESULTS

### Tumour site location

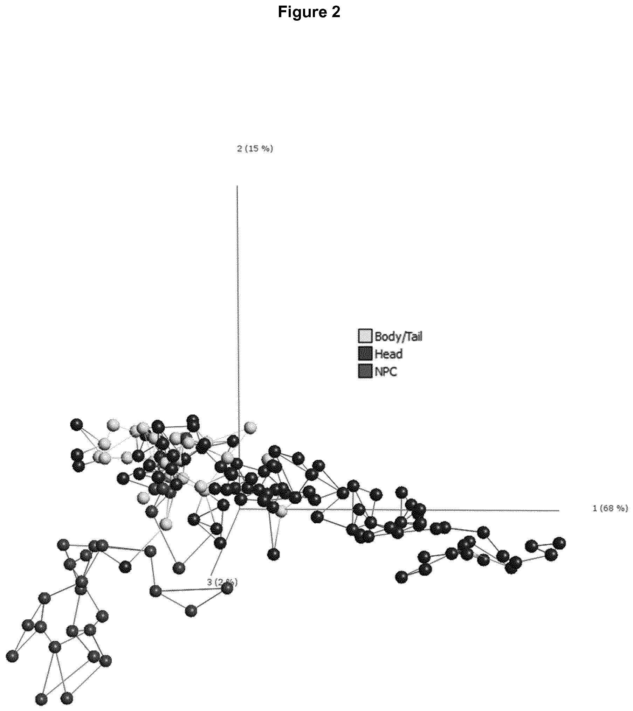

The serum samples could be discriminated depending on the location of the primary tumour in the pancreas. PCA indicated that patients with tumours located in the body or the tail of the pancreas clustered closer to NPC subjects compared to patients with tumours in the head of the pancreas (Fig. 2). Of note, protein markers in samples derived from patients with a tumour location in the head of pancreas could still discriminate body/tail tumour samples vs. NPC, indicating that the general PDAC signature is not affected by tumour site. The differential protein expression analysis revealed an extensive list of (different) markers in the intra-pancreatic comparison of head vs. body/tail tumours, with 39% of the markers displaying p-values < 0.001, almost exclusively upregulated levels in serum from head tumours compared to body/tail tumour samples (Table 4). In Table 4, the full list of all differentially expressed antibodies (biomarkers are listed).

### DISCUSSION

A new finding was the observation that serum protein markers associated with tumour localization were identified. A major problem with tumours of the body/tail in comparison with pancreatic head cancer is distant metastasis, especially in the liver, and resection of the tumour does not increase postoperative survival in metastatic disease (Wu *et al,* 2007). On the other hand, patients with local-stage body/tail tumours had higher survival rates compared with local-stage pancreatic head cancer (Lau *et al,* 2010). Our data indicated that markers in samples from patients with body/tail tumours clustering closer to the NPC controls, as compared to samples from patients with pancreatic head tumours. This may be explained by a more profound systemic impact of the head tumours, as these are prone to invade the surrounding mesenteric blood vessels connecting the pancreas to the duodenum (Hidalgo, 2010), or by changes secondary to biliary obstruction. As the biological differences can result in different treatment efficiency (Wu TC et al 2007), biomarkers that can discriminate between tumour localization are of clinical relevance.

### References

Alonzo TA, Pepe MS, Moskowitz CS (2002) Sample size calculations for comparative studies of medical tests for detecting presence of disease. Statistics in medicine 21(6): 835-52
Anderson NL, Anderson NG (2002) The human plasma proteome: history, character, and diagnostic prospects. Molecular & cellular proteomics : MCP 1(11): 845-67
Arlt A, Muerkoster SS, Schafer H (2013) Targeting apoptosis pathways in pancreatic cancer. Cancer letters 332(2): 346-58
Borrebaeck CA, Wingren C (2009) Design of high-density antibody microarrays for disease proteomics: key technological issues. Journal of proteomics 72(6): 928-35
Brand RE, Nolen BM, Zeh HJ, Allen PJ, Eloubeidi MA, Goldberg M, Elton E, Arnoletti JP, Christein JD, Vickers SM, Langmead CJ, Landsittel DP, Whitcomb DC, Grizzle WE, Lokshin AE (2011) Serum biomarker panels for the detection of pancreatic cancer. Clinical cancer research : an official journal of the American Association for Cancer Research 17(4): 805-16
Bunger S, Laubert T, Roblick UJ, Habermann JK (2011) Serum biomarkers for improved diagnostic of pancreatic cancer: a current overview. Journal of cancer research and clinical oncology 137(3): 375-89
Carlsson A, Persson O, Ingvarsson J, Widegren B, Salford L, Borrebaeck CA, Wingren C (2010) Plasma proteome profiling reveals biomarker patterns associated with prognosis and therapy selection in glioblastoma multiforme patients. Proteomics Clinical applications 4(6-7): 591-602
Carlsson A, Wingren C, Ingvarsson J, Ellmark P, Baldertorp B, Ferno M, Olsson H, Borrebaeck CA (2008) Serum proteome profiling of metastatic breast cancer using recombinant antibody microarrays. Eur J Cancer 44(3): 472-80
Carlsson A, Wingren C, Kristensson M, Rose C, Ferno M, Olsson H, Jernstrom H, Ek S, Gustavsson E, Ingvar C, Ohlsson M, Peterson C, Borrebaeck CA (2011a) Molecular serum portraits in patients with primary breast cancer predict the development of distant metastases. Proceedings of the National Academy of Sciences of the United States of America 108(34): 14252-7
Carlsson A, Wuttge DM, Ingvarsson J, Bengtsson AA, Sturfelt G, Borrebaeck CA, Wingren C (2011b) Serum protein profiling of systemic lupus erythematosus and systemic sclerosis using recombinant antibody microarrays. Molecular & cellular proteomics: MCP 10(5): M110 005033 Coussens LM, Werb Z (2002) Inflammation and cancer. Nature 420(6917): 860-7
Duraker N, Hot S, Polat Y, Hobek A, Gender N, Urhan N (2007) CEA, CA 19-9, and CA 125 in the differential diagnosis of benign and malignant pancreatic diseases with or without jaundice. Journal of surgical oncology 95(2): 142-7
Faca VM, Song KS, Wang H, Zhang Q, Krasnoselsky AL, Newcomb LF, Plentz RR, Gurumurthy S, Redston MS, Pitteri SJ, Pereira-Faca SR, Ireton RC, Katayama H, Glukhova V, Phanstiel D, Brenner DE, Anderson MA, Misek D, Scholler N, Urban ND, Barnett MJ, Edelstein C, Goodman GE, Thornquist MD, McIntosh MW, DePinho RA, Bardeesy N, Hanash SM (2008) A mouse to human search for plasma proteome changes associated with pancreatic tumour development. PLoS medicine 5(6): e123
Firpo MA, Gay DZ, Granger SR, Scaife CL, DiSario JA, Boucher KM, Mulvihill SJ (2009) Improved diagnosis of pancreatic adenocarcinoma using haptoglobin and serum amyloid A in a panel screen. World journal of surgery 33(4): 716-22
Ghatnekar O, Andersson R, Svensson M, Persson U, Ringdahl U, Zeilon P, Borrebaeck CA (2013) Modelling the benefits of early diagnosis of pancreatic cancer using a biomarker signature. International journal of cancer Journal international du cancer
Haab BB, Geierstanger BH, Michailidis G, Vitzthum F, Forrester S, Okon R, Saviranta P, Brinker A, Sorette M, Perlee L, Suresh S, Drwal G, Adkins JN, Omenn GS (2005) Immunoassay and antibody microarray analysis of the HUPO Plasma Proteome Project reference specimens: systematic variation between sample types and calibration of mass spectrometry data. Proteomics 5(13): 3278-91
Hidalgo M (2010) Pancreatic cancer. The New England journal of medicine 362(17): 1605-17
Ingvarsson J, Wingren C, Carlsson A, Ellmark P, Wahren B, Engstrom G, Harmenberg U, Krogh M, Peterson C, Borrebaeck CA (2008) Detection of pancreatic cancer using antibody microarray-based serum protein profiling. Proteomics 8(11): 2211-9
Jiang JT, Wu CP, Deng HF, Lu MY, Wu J, Zhang HY, Sun WH, Ji M (2004) Serum level of TSGF, CA242 and CA19-9 in pancreatic cancer. World journal of gastroenterology : WJG 10(11): 1675-7
Konstantinou F, Syrigos KN, Saif MW (2013) Pancreatic cancer: what about screening and detection? JOP : Journal of the pancreas 14(4): 312-5
Koopmann J, Rosenzweig CN, Zhang Z, Canto MI, Brown DA, Hunter M, Yeo C, Chan DW, Breit SN, Goggins M (2006) Serum markers in patients with resectable pancreatic adenocarcinoma: macrophage inhibitory cytokine 1 versus CA19-9. Clinical cancer research : an official journal of the American Association for Cancer Research 12(2): 442-6
Kudo-Saito C, Shirako H, Ohike M, Tsukamoto N, Kawakami Y (2013) CCL2 is critical for immunosuppression to promote cancer metastasis. Clinical & experimental metastasis 30(4): 393-405
Lau MK, Davila JA, Shaib YH (2010) Incidence and survival of pancreatic head and body and tail cancers: a population-based study in the United States. Pancreas 39(4): 458-62
Locker GY, Hamilton S, Harris J, Jessup JM, Kemeny N, Macdonald JS, Somerfield MR, Hayes DF, Bast RC, Jr. (2006) ASCO 2006 update of recommendations for the use of tumour markers in gastrointestinal cancer. Journal of clinical oncology : official journal of the American Society of Clinical Oncology 24(33): 5313-27
Ma Y, Hwang RF, Logsdon CD, Ullrich SE (2013) Dynamic mast cell-stromal cell interactions promote growth of pancreatic cancer. Cancer research 73(13): 3927-37
Malvezzi M, Bertuccio P, Levi F, La Vecchia C, Negri E (2014) European cancer mortality predictions for the year 2014. Annals of oncology: official journal of the European Society for Medical Oncology / ESMO
McDade TP, Perugini RA, Vittimberga FJ, Jr., Carrigan RC, Callery MP (1999) Salicylates inhibit NF-kappaB activation and enhance TNF-alpha-induced apoptosis in human pancreatic cancer cells. The Journal of surgical research 83(1): 56-61
Ni XG, Bai XF, Mao YL, Shao YF, Wu JX, Shan Y, Wang CF, Wang J, Tian YT, Liu Q, Xu DK, Zhao P (2005) The clinical value of serum CEA, CA19-9, and CA242 in the diagnosis and prognosis of pancreatic cancer. European journal of surgical oncology : the journal of the European Society of Surgical Oncology and the British Association of Surgical Oncology 31(2): 164-9
Olsson N, Wallin S, James P, Borrebaeck CA, Wingren C (2012) Epitope-specificity of recombinant antibodies reveals promiscuous peptide-binding properties. Protein science : a publication of the Protein Society 21(12): 1897-910
Orchekowski R, Hamelinck D, Li L, Gliwa E, vanBrocklin M, Marrero JA, Vande Woude GF, Feng Z, Brand R, Haab BB (2005) Antibody microarray profiling reveals individual and combined serum proteins associated with pancreatic cancer. Cancer research 65(23): 11193-202
Parker LA, Porta M, Lumbreras B, Lopez T, Guarner L, Hernandez-Aguado I, Carrato A, Corominas JM, Rifa J, Fernandez E, Alguacil J, Malats N, Real FX (2011) Clinical validity of detecting K-ras mutations for the diagnosis of exocrine pancreatic cancer: a prospective study in a clinically-relevant spectrum of patients. European journal of epidemiology 26(3): 229-36
Porta M, Costafreda S, Malats N, Guarner L, Soler M, Gubern JM, Garcia-Olivares E, Andreu M, Salas A, Corominas JM, Alguacil J, Carrato A, Rifa J, Real FX (2000) Validity of the hospital discharge diagnosis in epidemiologic studies of biliopancreatic pathology. PANKRAS II Study Group. European journal of epidemiology 16(6): 533-41
Porta M, Fabregat X, Malats N, Guarner L, Carrato A, de Miguel A, Ruiz L, Jariod M, Costafreda S, Coll S, Alguacil J, Corominas JM, Sola R, Salas A, Real FX (2005) Exocrine pancreatic cancer: symptoms at presentation and their relation to tumour site and stage. Clinical & translational oncology : official publication of the Federation of Spanish Oncology Societies and of the National Cancer Institute of Mexico 7(5): 189-97
Porta M, Malats N, Jariod M, Grimalt JO, Rifa J, Carrato A, Guarner L, Salas A, Santiago-Silva M, Corominas JM, Andreu M, Real FX (1999) Serum concentrations of organochlorine compounds and K-ras mutations in exocrine pancreatic cancer. PANKRAS II Study Group. Lancet 354(9196): 2125-9
Quackenbush J (2001) Computational analysis of microarray data. Nature reviews Genetics 2(6): 418-27
Rahib L, Smith BD, Aizenberg R, Rosenzweig AB, Fleshman JM, Matrisian LM (2014) Projecting cancer incidence and deaths to 2030: the unexpected burden of thyroid, liver, and pancreas cancers in the United States. Cancer research 74(11): 2913-21
Sandstrom A, Andersson R, Segersvard R, Lohr M, Borrebaeck CA, Wingren C (2012) Serum proteome profiling of pancreatitis using recombinant antibody microarrays reveals disease-associated biomarker signatures. Proteomics Clinical applications 6(9-10): 486-96
Shaib YH, Davila JA, EI-Serag HB (2006) The epidemiology of pancreatic cancer in the United States: changes below the surface. Alimentary pharmacology & therapeutics 24(1): 87-94 Siegel R, Naishadham D, Jemal A (2012) Cancer statistics, 2012. CA: a cancer journal for clinicians 62(1): 10-29
Steinhauer C, Wingren C, Hager AC, Borrebaeck CA (2002) Single framework recombinant antibody fragments designed for protein chip applications. BioTechniques Suppl: 38-45
Surinova S, Schiess R, Huttenhain R, Cerciello F, Wollscheid B, Aebersold R (2011) On the development of plasma protein biomarkers. Journal of proteome research 10(1): 5-16
Wingren C, Borrebaeck CA (2008) Antibody microarray analysis of directly labelled complex proteomes. Current opinion in biotechnology 19(1): 55-61
Wingren C, Ingvarsson J, Dexlin L, Szul D, Borrebaeck CA (2007) Design of recombinant antibody microarrays for complex proteome analysis: choice of sample labeling-tag and solid support. Proteomics 7(17): 3055-65
Wingren C, Sandstrom A, Segersvard R, Carlsson A, Andersson R, Lohr M, Borrebaeck CA (2012) Identification of serum biomarker signatures associated with pancreatic cancer. Cancer research 72(10): 2481-90
Wingren C, Steinhauer C, Ingvarsson J, Persson E, Larsson K, Borrebaeck CA (2005) Microarrays based on affinity-tagged single-chain Fv antibodies: sensitive detection of analyte in complex proteomes. Proteomics 5(5): 1281-91
Wu TC, Shao YF, Shan Y, Wu JX, Zhao P (2007) [Surgical effect of malignant tumour of body and tail of the pancreas: compare with pancreatic head cancer]. Zhonghua wai ke za zhi [Chinese journal of surgery] 45(1): 30-3
Wu YW, Wooldridge PJ (2005) The impact of centering first-level predictors on individual and contextual effects in multilevel data analysis. Nursing research 54(3): 212-6
Yachida S, Jones S, Bozic I, Antal T, Leary R, Fu B, Kamiyama M, Hruban RH, Eshleman JR, Nowak MA, Velculescu VE, Kinzler KW, Vogelstein B, lacobuzio-Donahue CA (2010) Distant metastasis occurs late during the genetic evolution of pancreatic cancer. Nature 467(7319): 1114-7
Zhang H, Liu AY, Loriaux P, Wollscheid B, Zhou Y, Watts JD, Aebersold R (2007) Mass spectrometric detection of tissue proteins in plasma. Molecular & cellular proteomics : MCP 6(1): 64-71

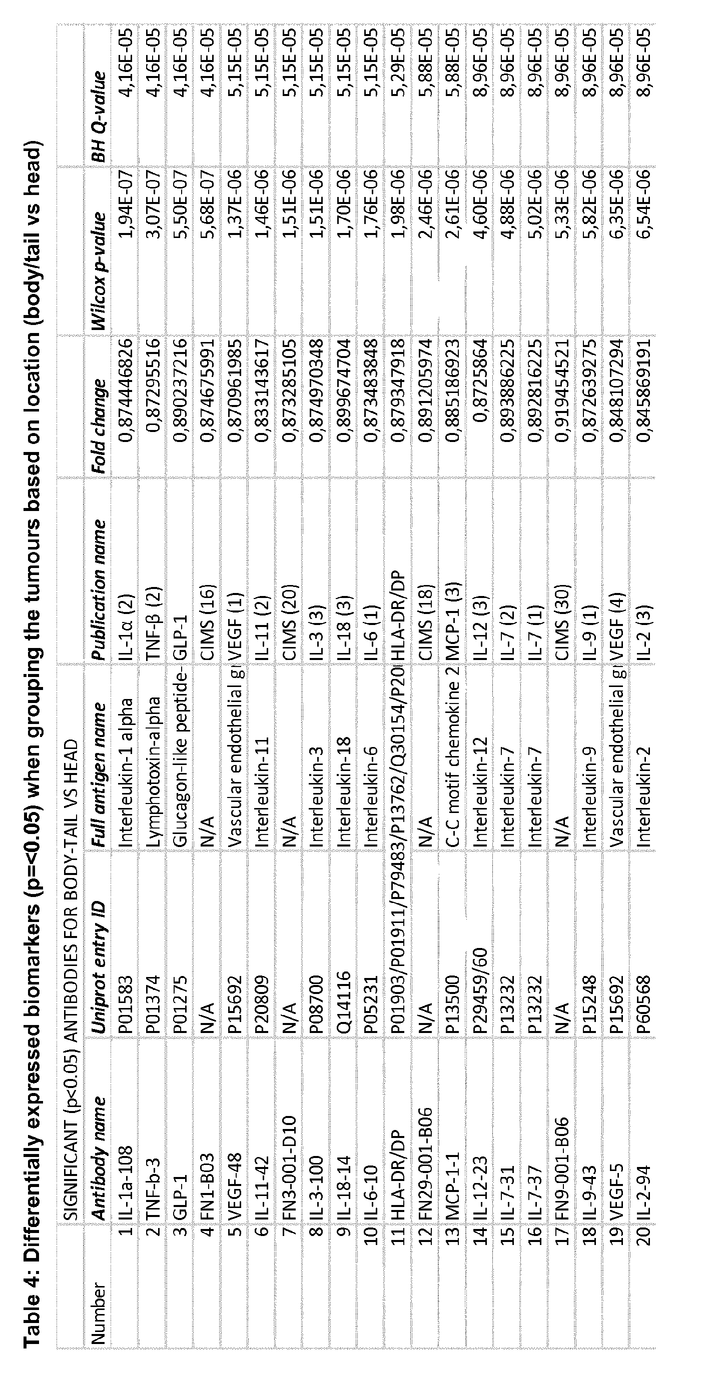

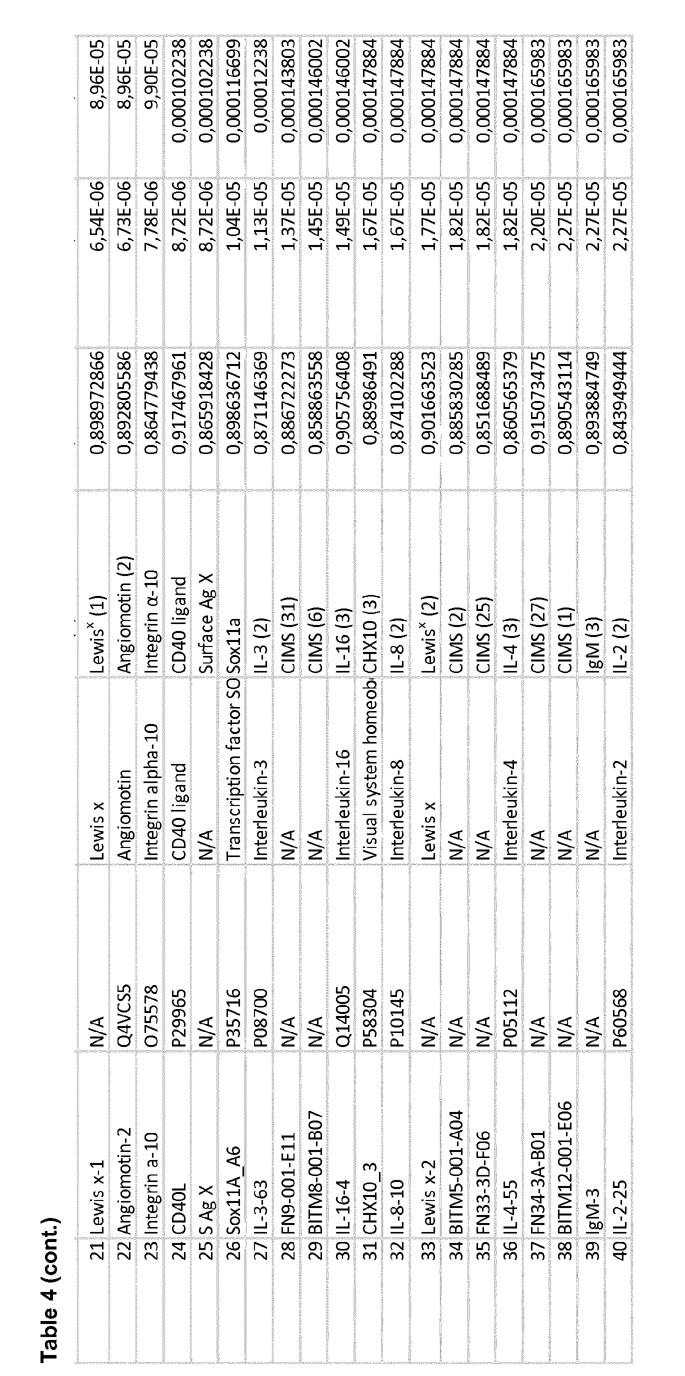

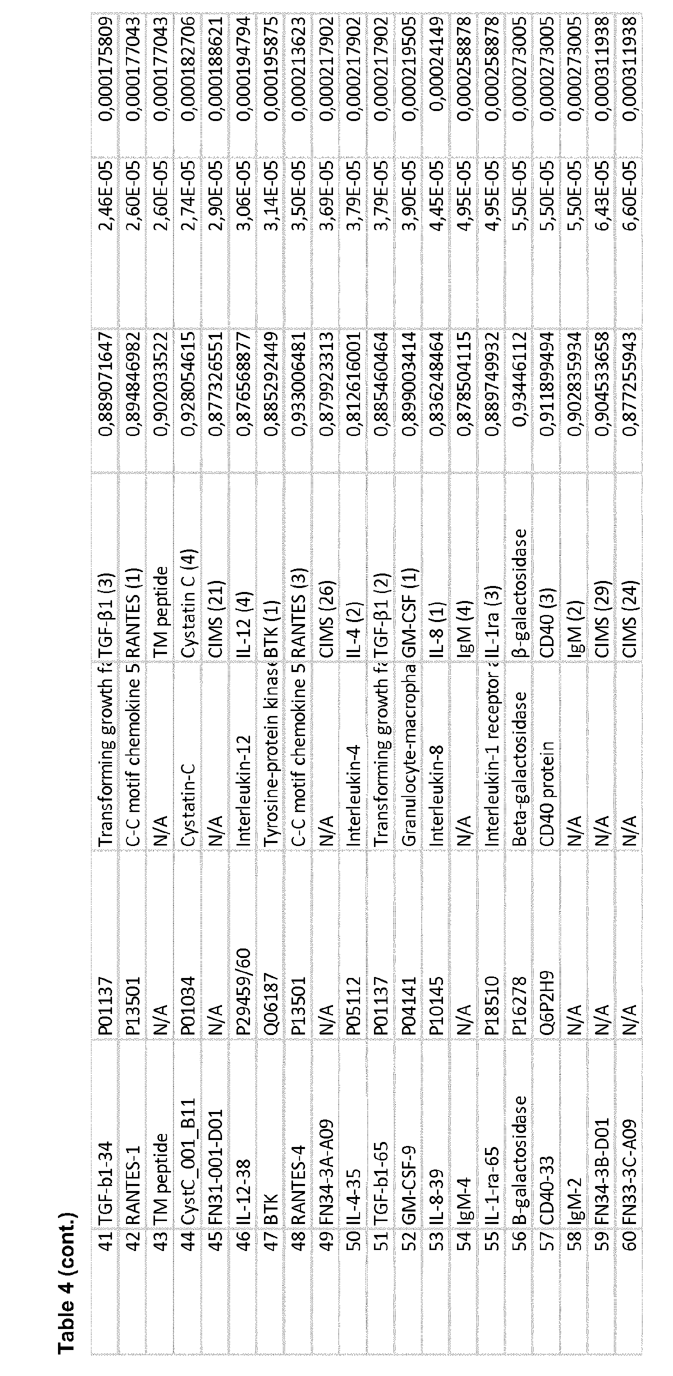

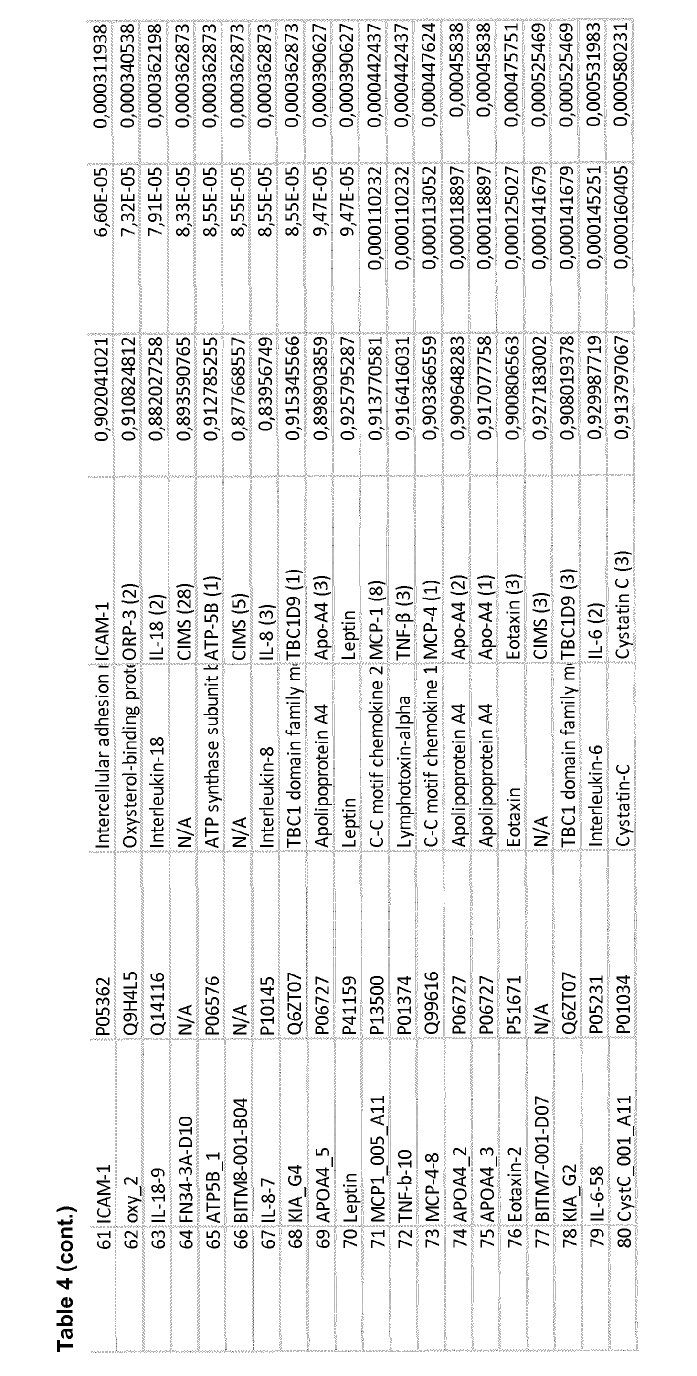

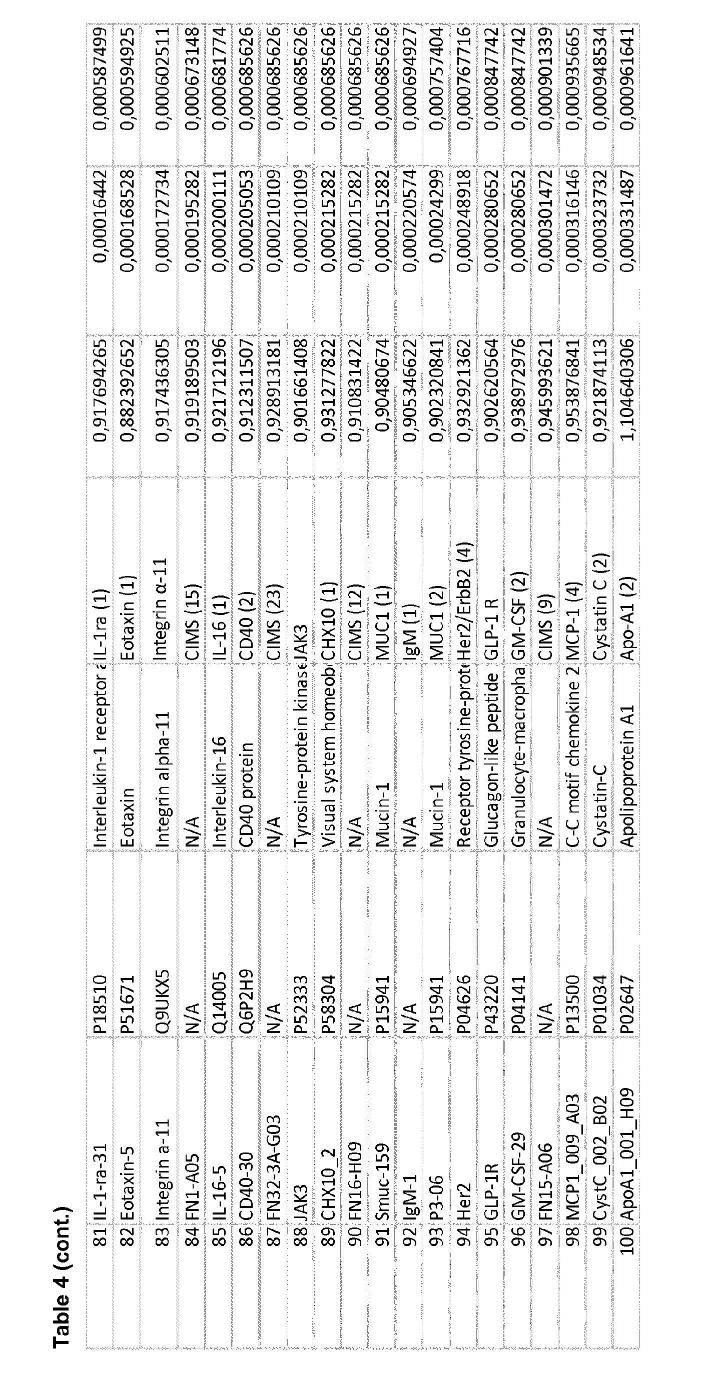

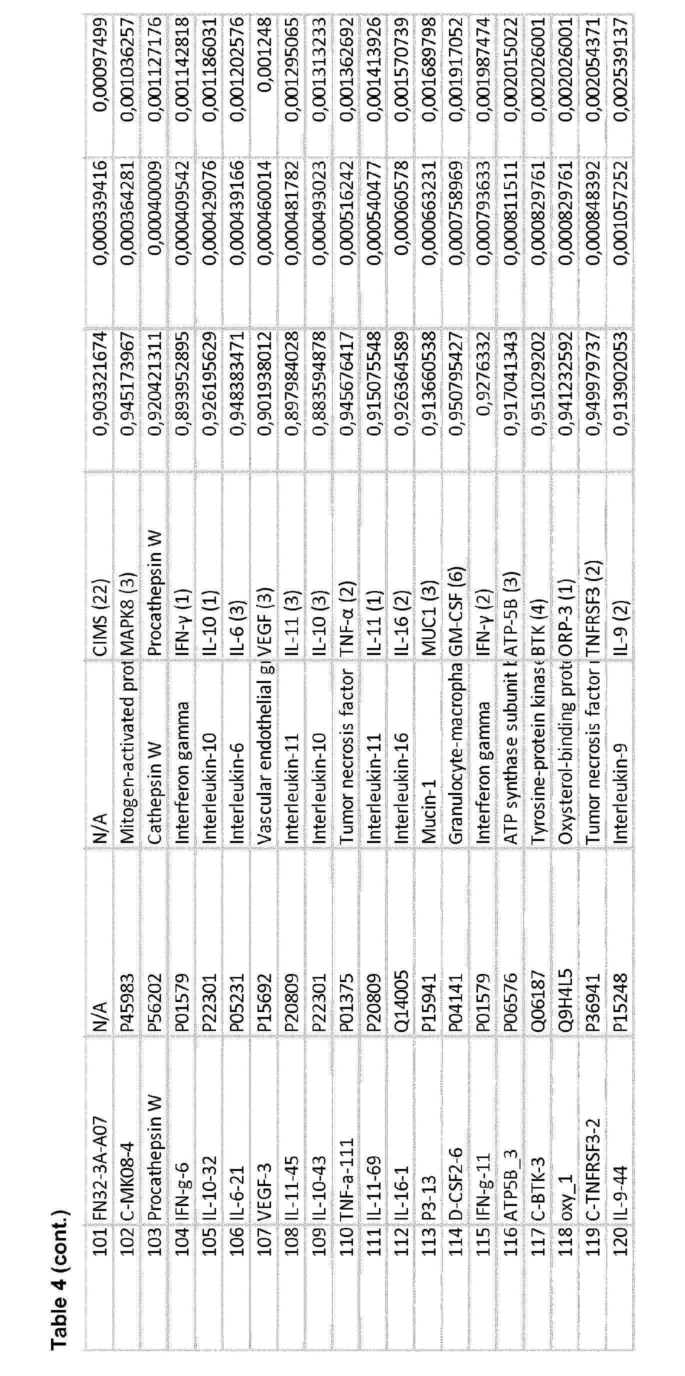

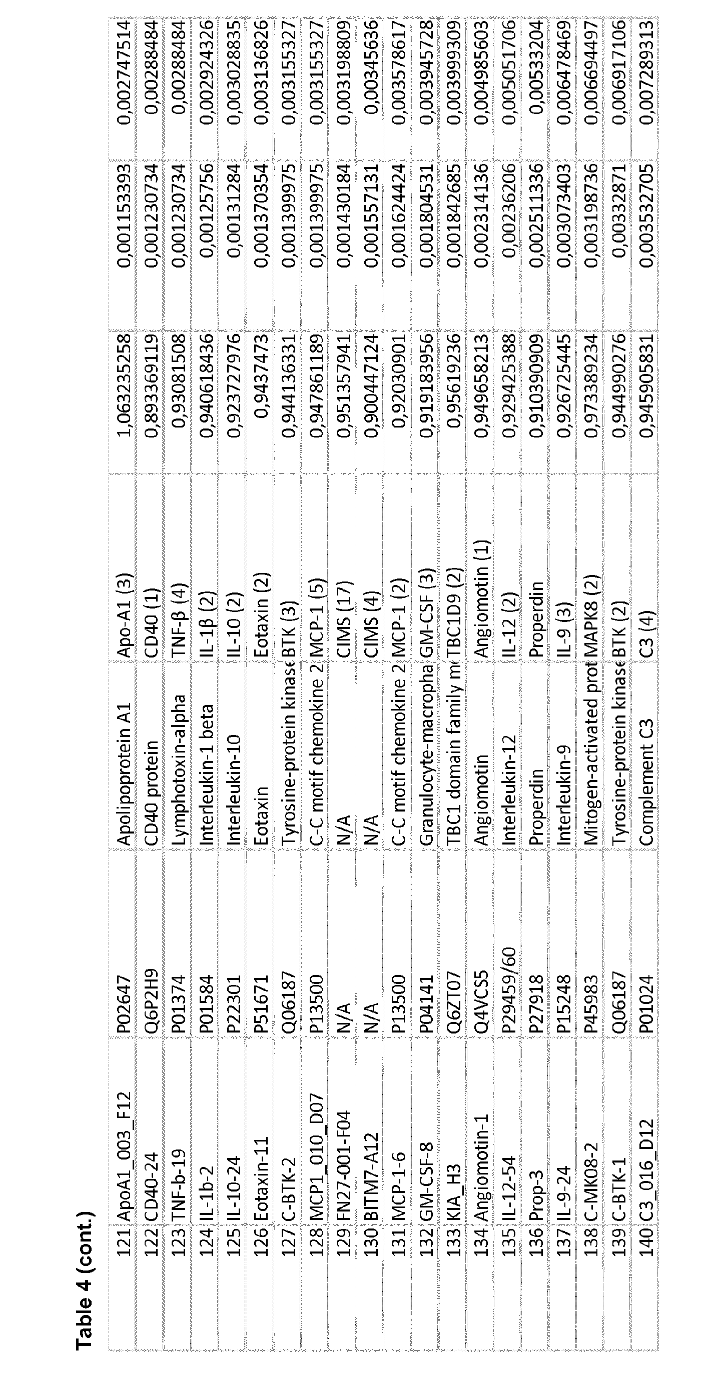

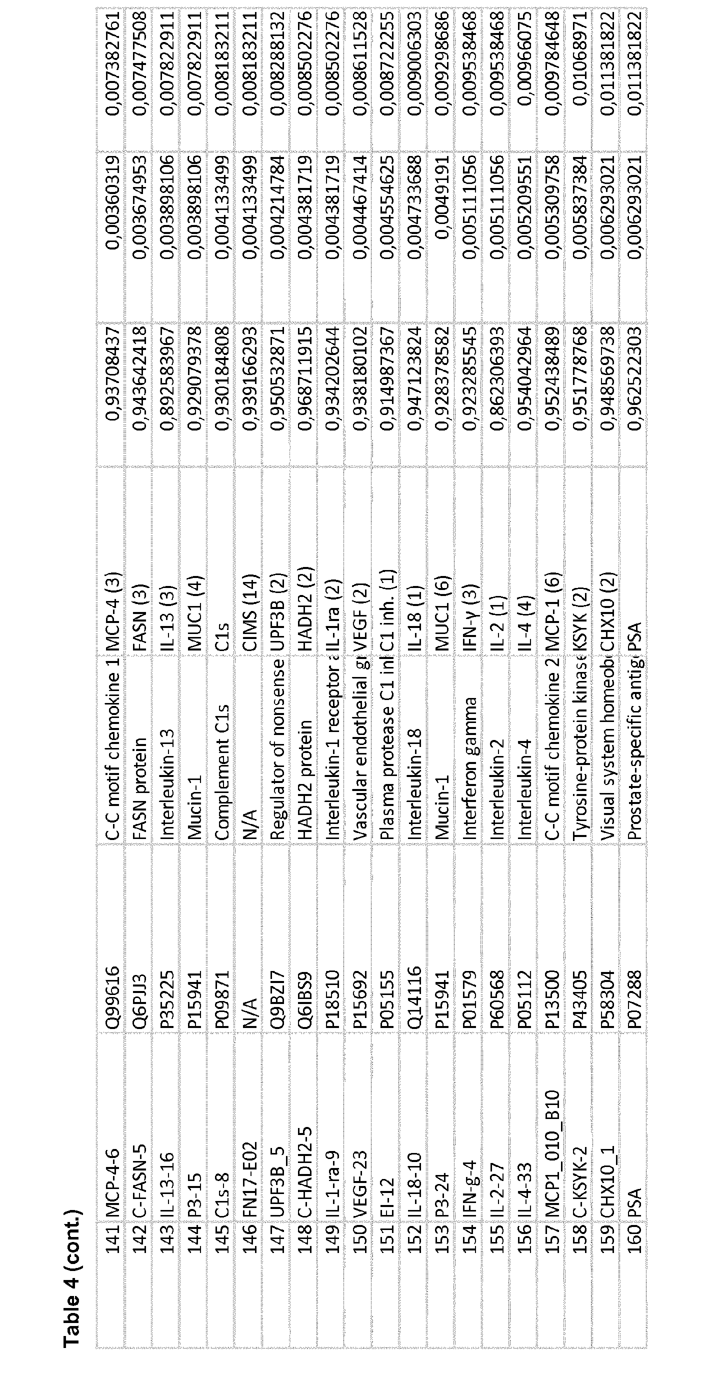

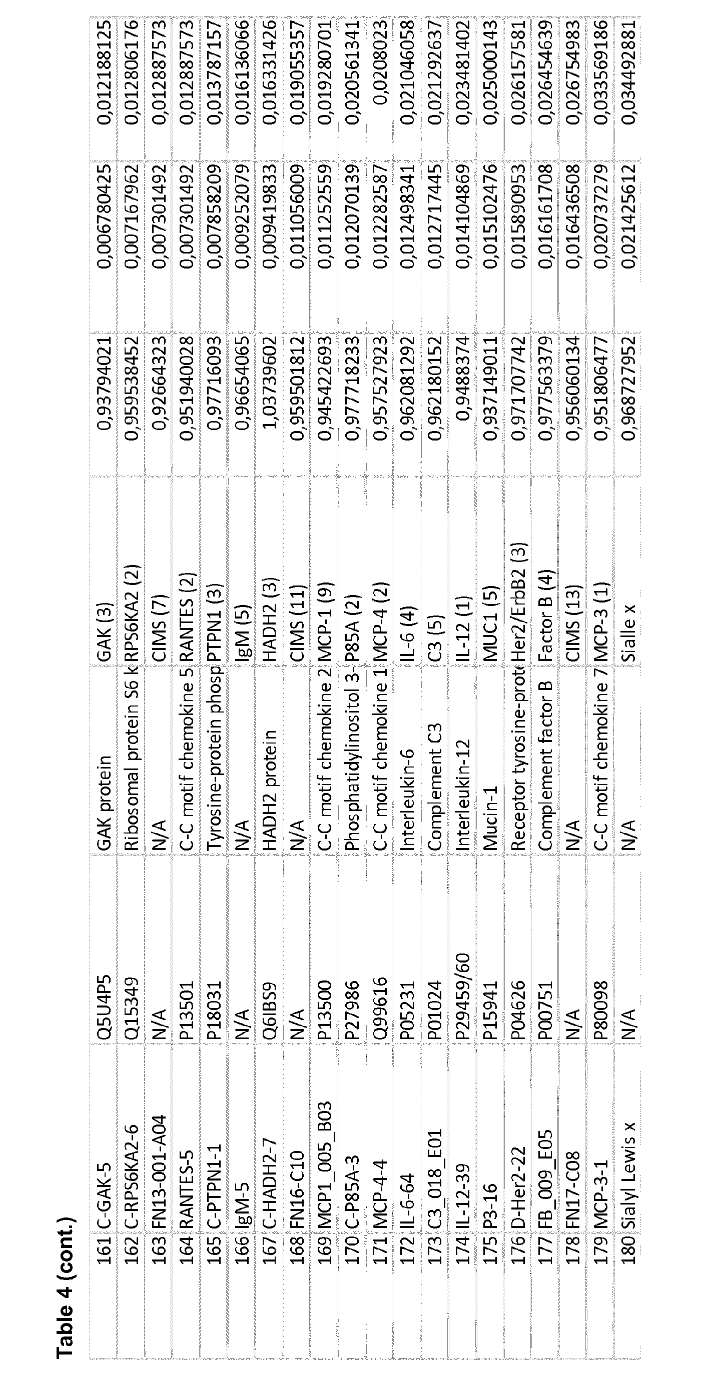

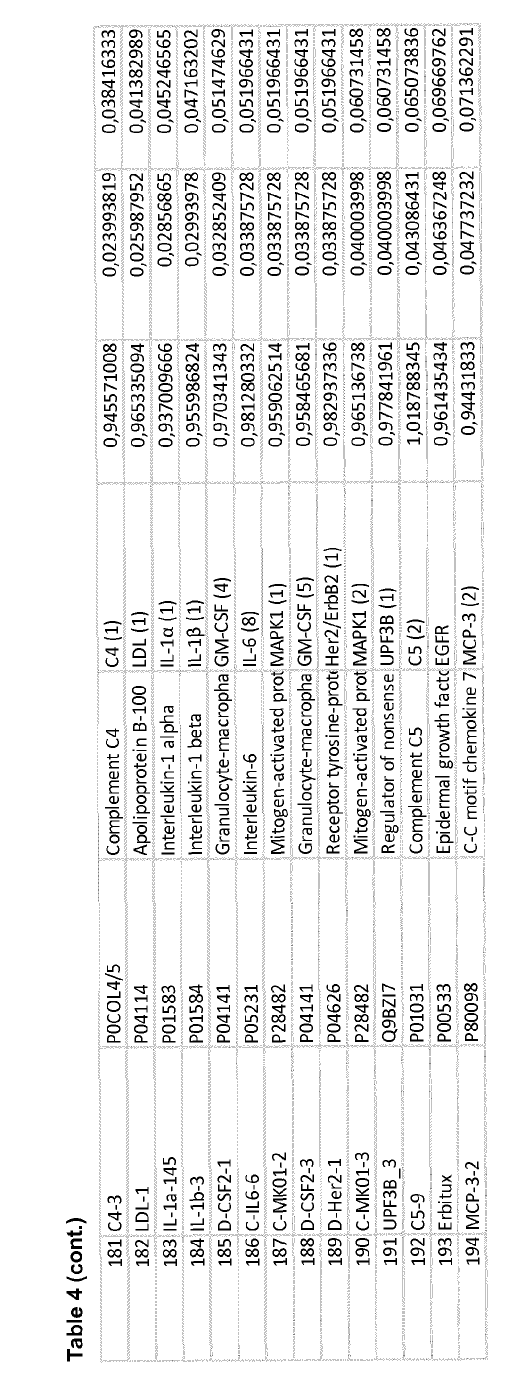

**Table 5: list of analytes**

| ***Ab clone name*** | ***Antigen name*** | ***Gene name*** | ***Uniprot entry ID*** | ***Recommended protein name*** | ***Short name*** |
|---|---|---|---|---|---|
| IL-1a-145 | IL-1α | IL1A | P01583 | Interleukin-1 alpha | IL-1a |
| IL-1a-108 | IL-1α | IL1A | P01583 | Interleukin-1 alpha | IL-1a |
| IL-2-27 | IL-2 | IL2 | P60568 | Interleukin-2 | IL-2 |
| IL-2-25 | IL-2 | IL2 | P60568 | Interleukin-2 | IL-2 |
| IL-2-94 | IL-2 | IL2 | P60568 | Interleukin-2 | IL-2 |
| IL-3-58 | IL-3 | IL3 | P08700 | Interleukin-3 | IL-3 |
| IL-3-63 | IL-3 | IL3 | P08700 | Interleukin-3 | IL-3 |
| IL-3-100 | IL-3 | IL3 | P08700 | Interleukin-3 | IL-3 |
| IL-4-37 | IL-4 | IL4 | P05112 | Interleukin-4 | IL-4 |
| IL-4-35 | IL-4 | IL4 | P05112 | Interleukin-4 | IL-4 |
| IL-5-18 | IL-5 | IL5 | P05113 | Interleukin-5 | IL-5 |
| IL-5-20 | IL-5 | IL5 | P05113 | Interleukin-5 | IL-5 |
| IL-5-21 | IL-5 | IL5 | P05113 | Interleukin-5 | IL-5 |
| IL-6-10 | IL-6 | IL6 | P05231 | Interleukin-6 | IL-6 |
| IL-6-58 | IL-6 | IL6 | P05231 | Interleukin-6 | IL-6 |
| IL-7-37 | IL-7 | IL7 | P13232 | Interleukin-7 | IL-7 |
| IL-7-31 | IL-7 | IL7 | P13232 | Interleukin-7 | IL-7 |
| IL-8-39 | IL-8 | IL8 | P10145 | Interleukin-8 | IL-8 |
| IL-8-10 | IL-8 | IL8 | P10145 | Interleukin-8 | IL-8 |
| IL-9-43 | IL-9 | IL9 | P15248 | Interleukin-9 | IL-9 |
| IL-9-44 | IL-9 | IL9 | P15248 | Interleukin-9 | IL-9 |
| IL-9-24 | IL-9 | IL9 | P15248 | Interleukin-9 | IL-9 |
| IL-10-32 | IL-10 | IL10 | P22301 | Interleukin-10 | IL-10 |
| IL-10-24 | IL-10 | IL10 | P22301 | Interleukin-10 | IL-10 |
| IL-10-43 | IL-10 | IL10 | P22301 | Interleukin-10 | IL-10 |
| IL-11-69 | IL-11 | IL11 | P20809 | Interleukin-11 | IL-11 |
| IL-11-42 | IL-11 | IL11 | P20809 | Interleukin-11 | IL-11 |
| IL-11-45 | IL-11 | IL11 | P20809 | Interleukin-11 | IL-11 |
| IL-12-39 | IL-12 | IL12A/B | P29459/60 | Interleukin-12 | IL-12 |
| IL-12-54 | IL-12 | IL12A/B | P29459/60 | Interleukin-12 | IL-12 |
| IL-13-1 | IL-13 | IL13 | P35225 | Interleukin-13 | IL-13 |
| IL-13-5 | IL-13 | IL13 | P35225 | Interleukin-13 | IL-13 |
| IL-13-16 | IL-13 | IL13 | P35225 | Interleukin-13 | IL-13 |
| VEGF-48 | VEGF | VEGFA | P15692 | Vascular endothelial growth factor | VEGF |
| VEGF-23 | VEGF | VEGFA | P15692 | Vascular endothelial growth factor | VEGF |
| TGF-b1-64 | TGF-β1 | TGFB | P01137 | Transforming growth factor beta-1 | TGF-b1 |
| TGF-b1-65 | TGF-β1 | TGFB | P01137 | Transforming growth factor beta-1 | TGF-b1 |
| TGF-b1-34 | TGF-β1 | TGFB | P01137 | Transforming growth factor beta-1 | TGF-b1 |
| TNF-a-89 | TNF-α | TNF | P01375 | Tumor necrosis factor | TNF-a |
| TNF-a-111 | TNF-α | TNF | P01375 | Tumor necrosis factor | TNF-a |
| TNF-a-126 | TNF-α | TNF | P01375 | Tumor necrosis factor | TNF-a |
| GM-CSF-9 | GM-CSF | CSF2 | P04141 | Granulocyte-macrophage colony-stimulating factor | GM-CSF |
| GM-CSF-29 | GM-CSF | CSF2 | P04141 | Granulocyte-macrophage colony-stimulating factor | GM-CSF |
| GM-CSF-8 | GM-CSF | CSF2 | P04141 | Granulocyte-macrophage colony-stimulating factor | GM-CSF |
| TNF-b-1 | TNF-β | LTA | P01374 | Lymphotoxin-alpha | TNF-b |
| TNF-b-3 | TNF-β | LTA | P01374 | Lymphotoxin-alpha | TNF-b |
| IL-1-ra-31 | IL-1-ra | ILIRA | P18510 | Interleukin-1 receptor antagonist protein | IL-1ra |
| IL-1-ra-9 | IL-1-ra | ILIRA | P18510 | Interleukin-1 receptor antagonist protein | IL-1ra |
| IL-1-ra-65 | IL-1-ra | ILIRA | P18510 | Interleukin-1 receptor antagonist protein | IL-1ra |
| IL-16-5 | IL-16 | IL16 | Q14005 | Interleukin-16 | IL-16 |
| IL-16-1 | IL-16 | IL16 | Q14005 | Interleukin-16 | IL-16 |
| IL-18-10 | IL-18 | IL18 | Q14116 | Interleukin-18 | IL-18 |
| IL-18-9 | IL-18 | IL18 | Q14116 | Interleukin-18 | IL-18 |
| MCP-4-8 | MCP-4 | CCL13 | Q99616 | C-C motif chemokine 13 | MCP-4 |
| MCP-4-4 | MCP-4 | CCL13 | Q99616 | C-C motif chemokine 13 | MCP-4 |
| IFN-g-6 | IFN-γ | IFNG | P01579 | Interferon gamma | IFN-g |
| IFN-g-11 | IFN-γ | IFNG | P01579 | Interferon gamma | IFN-g |
| IFN-g-4 | IFN-γ | IFNG | P01579 | Interferon gamma | IFN-g |
| IL-1b-3 | IL-1β | IL1B | P01584 | Interleukin-1 beta | IL-1b |
| IL-1b-2 | IL-1β | IL1B | P01584 | Interleukin-1 beta | IL-1b |
| IL-1b-1 | IL-1β | IL1B | P01584 | Interleukin-1 beta | IL-1b |
| Eotaxin-5 | Eotaxin | CCL11 | P51671 | Eotaxin | Eotaxin |
| Eotaxin-11 | Eotaxin | CCL11 | P51671 | Eotaxin | Eotaxin |
| Eotaxin-2 | Eotaxin | CCL11 | P51671 | Eotaxin | Eotaxin |
| RANTES-1 | RANTES | CCL5 | P13501 | C-C motif chemokine 5 | RANTES |
| RANTES-5 | RANTES | CCL5 | P13501 | C-C motif chemokine 5 | RANTES |
| RANTES-4 | RANTES | CCL5 | P13501 | C-C motif chemokine 5 | RANTES |
| MCP-1-9 | MCP-1 | CCL2 | P13500 | C-C motif chemokine 2 | MCP-1 |
| MCP-1-6 | MCP-1 | CCL2 | P13500 | C-C motif chemokine 2 | MCP-1 |
| MCP-1-1 | MCP-1 | CCL2 | P13500 | C-C motif chemokine 2 | MCP-1 |
| MCP-3-1 | MCP-3 | CCL7 | P80098 | C-C motif chemokine 7 | MCP-3 |
| MCP-3-2 | MCP-3 | CCL7 | P80098 | C-C motif chemokine 7 | MCP-3 |
| MCP-3-3 | MCP-3 | CCL7 | P80098 | C-C motif chemokine 7 | MCP-3 |
| *CB1* | *Streptavidin* | | | | |
| CB2 | β-galactosidase | GLB1 | P16278 | Beta-galactosidase | B-galactosidase |
| CB3 | CD40L | CD40LG | P29965 | CD40 ligand | CD40L |
| CB4 | Angiomotin | AMOT | Q4VCS5 | Angiomotin | Angiomotin |
| CB5 | Angiomotin | AMOT | Q4VCS5 | Angiomotin | Angiomotin |
| CB6 | Leptin | LEP | P41159 | Leptin | Leptin |
| CB7 | Integrin α-10 | ITGA10 | O75578 | Integrin alpha-10 | Integrin a-10 |
| CB8 | Integrin α-11 | ITGA11 | Q9UKX5 | Integrin alpha-11 | Integrin a-11 |
| CB9 | IgM (initialt angiven som B) | | | | |
| *CB10* | *TAT* | | | | |
| *CB11* | *TAT* | | | | |
| CB12 | LDL | APOB OBS, APOB endast en del av LDL | P04114 | Apolipoprotein B-100 | LDL |
| | | | | | |
| CB13 | LDL | APOB OBS, APOB endast en del av LDL | P04114 | Apolipoprotein B-100 | LDL |
| CB14 | PSA | KLK3 | P07288 | Prostate-specific antigen | PSA |
| CB15 | Lewis x | | | | |
| CB16 | Lewis x | | | | |
| CB17 | Lewis y | | | | |
| CB18 | Sialyl Lewis x | | | | |
| CB19 | TM peptide | | | | |
| | | | | | |
| CB20 | Procathepsin W | CTSW, OBS finns inget id för pro-enzymet | P56202 | Cathepsin W | Procathepsin W |
| CB21 | BTK (Bruton's Tyrosine Kinase) | BTK | Q06187 | Tyrosine-protein kinase BTK | BTK |
| CB22 | JAK3 (Tyrosine protein kinase) | JAK3 | P52333 | Tyrosine-protein kinase JAK3 | JAK3 |
| CB23 | Digoxin | | | | |
| CB24 | GLP-1R | GLP1R | P43220 | Glucagon-like peptide 1 receptor | GLP-1R |
| CB25 | GLP-1 | GCG | P01275 | Glucagon-like peptide-1 | GLP-1 |
| C1q-4 | C1q | C1QA/B/C | P02745/6/7 | Complement C1q | C1q |
| C1s-8 | C1s | C1S | P09871 | Complement C1s | C1s |
| C3-28 | C3 | C3 | P01024 | Complement C3 | C3 |
| C3-7 | C3 | C3 | P01024 | Complement C3 | C3 |
| C4-3 | C4 | C4A/B | P0COL4/5 | Complement C4 | C4 |
| C5-12 | C5 | C5 | P01031 | Complement C5 | C5 |
| C5-9 | C5 | C5 | P01031 | Complement C5 | C5 |
| EI-12 | C1 esterase inhibitor | SERPING1 | P05155 | Plasma protease C1 inhibitor | C1 inh |
| FB-7 | Factor B | CFB | P00751 | Complement factor B | Factor B |
| IL-12-23 | IL-12 | IL12A/B | P29459/60 | Interleukin-12 | IL-12 |
| IL-12-38 | IL-12 | IL12A/B | P29459/60 | Interleukin-12 | IL-12 |
| IL-16-4 | IL-16 | IL16 | Q14005 | Interleukin-16 | IL-16 |
| IL-18-14 | IL-18 | IL18 | Q14116 | Interleukin-18 | IL-18 |
| IL-1a-122 | IL-1α | IL1A | P01583 | Interleukin-1 alpha | IL-1a |
| IL-6-21 | IL-6 | IL6 | P05231 | Interleukin-6 | IL-6 |
| IL-6-64 | IL-6 | IL6 | P05231 | Interleukin-6 | IL-6 |
| IL-8-7 | IL-8 | IL8 | P10145 | Interleukin-8 | IL-8 |
| MCP-4-6 | MCP-4 | CCL13 | Q99616 | C-C motif chemokine 13 | MCP-4 |
| Prop-3 | Properdin (Factor P) | CFP | P27918 | Properdin | Properdin |
| TNF-b-10 | TNF-β | LTA | P01374 | Lymphotoxin-alpha | TNF-b |
| TNF-b-19 | TNF-β | LTA | P01374 | Lymphotoxin-alpha | TNF-b |
| VEGF-3 | VEGF | VEGFA | P15692 | Vascular endothelial growth factor | VEGF |
| VEGF-5 | VEGF | VEGFA | P15692 | Vascular endothelial growth factor | VEGF |
| IL-4-55 | IL-4 | IL4 | P05112 | Interleukin-4 | IL-4 |
| IL-4-33 | IL-4 | IL4 | P05112 | Interleukin-4 | IL-4 |
| *Fitc8* | *FITC* | | | | |
| Smuc-159 | Mucin-1 | MUC1 | P15941 | Mucin-1 | MUC-1 |
| CD40-24 | CD40 | CD40 | Q6P2H9 | CD40 protein | CD40 |
| CD40-30 | CD40 | CD40 | Q6P2H9 | CD40 protein | CD40 |
| CD40-33 | CD40 | CD40 | Q6P2H9 | CD40 protein | CD40 |
| CD40-44 | CD40 | CD40 | Q6P2H9 | CD40 protein | CD40 |
| CT17 (new) | Choleratoxin subunit B | | | | |
| B10 | IgM | | | | |
| C10 | IgM | | | | |
| C11 | IgM | | | | |
| F1 | Surface antigen X (unknown) | | | | |
| CB26 | HLA-DR/DP | HLA-DRA/DRB1/DRB3/DRB4/DRB5/DPA1/DPB1 | P01903/P01911/P79483/P13762/Q30154/P20036/P04440 | | HLA-DR/DP |
| CB27 | ICAM-1 | ICAM1 | P05362 | Intercellular adhesion molecule 1 | ICAM-1 |
| CB28 | IgM | | | | |
| P3-06 | Mucin-1 | MUC1 | P15941 | Mucin-1 | MUC-1 |
| P3-13 | Mucin-1 | MUC1 | P15941 | Mucin-1 | MUC-1 |
| P3-15 | Mucin-1 | MUC1 | P15941 | Mucin-1 | MUC-1 |
| P3-16 | Mucin-1 | MUC1 | P15941 | Mucin-1 | MUC-1 |
| P3-24 | Mucin-1 | MUC1 | P15941 | Mucin-1 | MUC-1 |
| M1 | MCP-1 | CCL2 | P13500 | C-C motif chemokine 2 | MCP-1 |
| M2 | MCP-1 | CCL2 | P13500 | C-C motif chemokine 2 | MCP-1 |
| M3 | MCP-1 | CCL2 | P13500 | C-C motif chemokine 2 | MCP-1 |
| M4 | MCP-1 | CCL2 | P13500 | C-C motif chemokine 2 | MCP-1 |
| M5 | MCP-1 | CCL2 | P13500 | C-C motif chemokine 2 | MCP-1 |
| M6 | MCP-1 | CCL2 | P13500 | C-C motif chemokine 2 | MCP-1 |
| Cy1 | Cystatin C | CST3 | P01034 | Cystatin-C | Cystatin C |
| Cy2 | Cystatin C | CST3 | P01034 | Cystatin-C | Cystatin C |
| Cy3 | Cystatin C | CST3 | P01034 | Cystatin-C | Cystatin C |
| Cy4 | Cystatin C | CST3 | P01034 | Cystatin-C | Cystatin C |
| A1 | Apolipoprotein A1 | APOA1 | P02647 | Apolipoprotein A1 | Apo-A1 |
| A2 | Apolipoprotein A1 | APOA1 | P02647 | Apolipoprotein A1 | Apo-A1 |
| A3 | Apolipoprotein A1 | APOA1 | P02647 | Apolipoprotein A1 | Apo-A1 |
| B1 | Factor B | CFB | P00751 | Complement factor B | Factor B |
| B2 | Factor B | CFB | P00751 | Complement factor B | Factor B |
| B3 | Factor B | CFB | P00751 | Complement factor B | Factor B |
| Cest1 | C1 esterase inhibitor | SERPING1 | P05155 | Plasma protease C1 inhibitor | C1 inh |
| Cest2 | C1 esterase inhibitor | SERPING1 | P05155 | Plasma protease C1 inhibitor | C1 inh |
| Cest3 | C1 esterase inhibitor | SERPING1 | P05155 | Plasma protease C1 inhibitor | C1 inh |
| Ca | C5 | C5 | P01031 | Complement C5 | C5 |
| Cb | C4 | C4A/B | P0COL4/5 | Complement C4 | C4 |
| Cc | C4 | C4A/B | P0COL4/5 | Complement C4 | C4 |
| Cd | C4 | C4A/B | P0COL4/5 | Complement C4 | C4 |
| Ce | C3 | C3 | P01024 | Complement C3 | C3 |
| Cf | C3 | C3 | P01024 | Complement C3 | C3 |
| Cg | C3 | C3 | P01024 | Complement C3 | C3 |
| Ch | C3 | C3 | P01024 | Complement C3 | C3 |
| EG1 | MYOM2 | MYOM2 | P54296 | Myomesin-2 | Myomesin-2 |
| EG2 | MYOM2 | MYOM2 | P54296 | Myomesin-2 | Myomesin-2 |
| EG3 | LUM | LUM | P51884 | Lumican | Lumican |
| EG4 | DUSP9 | DUSP9 | Q99956 | Dual specificity protein phosphatase 9 | DUSP9 |
| EG5 | CHX10 | CHX10/VSX2 | P58304 | Visual system homeobox 2 | CHX10 |
| EG6 | CHX10 | CHX10/VSX2 | P58304 | Visual system homeobox 2 | CHX10 |
| EG7 | CHX10 | CHX10/VSX2 | P58304 | Visual system homeobox 2 | CHX10 |
| EG8 | ATP5B | ATP5B | P06576 | ATP synthase subunit beta, mitochondrial | ATP-5B |
| EG9 | ATP5B | ATP5B | P06576 | ATP synthase subunit beta, mitochondrial | ATP-5B |
| EG10 | ATP5B | ATP5B | P06576 | ATP synthase subunit beta, mitochondrial | ATP-5B |
| EG11 | Sox11a | SOX11 | P35716 | Transcription factor SOX-11 | Sox11A |
| EG12 | KIAA0882 | TBC1D9 | Q6ZT07 | TBC1 domain family member 9 | TBC1D9 |
| EG15 | UPF3B | UPF3B | Q9BZI7 | Regulator of nonsense transcripts 3B | UPF3B |
| EG16 | UPF3B | UPF3B | Q9BZI7 | Regulator of nonsense transcripts 3B | UPF3B |
| EG20 | APOA4 | APOA4 | P06727 | Apolipoprotein A4 | Apo-A4 |
| EG21 | APOA4 | APOA4 | P06727 | Apolipoprotein A4 | Apo-A4 |
| EG22 | APOA4 | APOA4 | P06727 | Apolipoprotein A4 | Apo-A4 |
| EG23 | KIAA0882 | TBC1D9 | Q6ZT07 | TBC1 domain family member 9 | TBC1D9 |
| EG24 | KIAA0882 | TBC1D9 | Q6ZT07 | TBC1 domain family member 9 | TBC1D9 |
| EG25 | OSBPL3 | OSBPL3 | Q9H4L5 | Oxysterol-binding protein-related protein 3 | ORP-3 |
| EG26 | OSBPL3 | OSBPL3 | Q9H4L5 | Oxysterol-binding protein-related protein 3 | ORP-3 |
| BITM12-001-E06 | | | | | |
| BITM5-001-A04 | | | | | |
| BITM7-001-D07 | | | | | |
| BITM7-A12 | | | | | |
| BITM8-001-B04 | | | | | |
| BITM8-001-B07 | | | | | |
| FN13-001-A04 | | | | | |
| FN14-H07 | | | | | |
| FN15-A06 | | | | | |
| FN16-B01 | | | | | |
| FN16-C10 | | | | | |
| FN16-H09 | | | | | |
| FN17-C08 | | | | | |
| FN17-E02 | | | | | |
| FN1-A05 | | | | | |
| FN1-B03 | | | | | |
| FN27-001-F04 | | | | | |
| FN29-001-B06 | | | | | |
| FN3-001-B04 | | | | | |
| FN3-001-D10 | | | | | |
| FN31-001-D01 | | | | | |
| FN32-3A-A07 | | | | | |
| FN32-3AG03 | | | | | |
| FN33-3C-A09 | | | | | |
| FN33-3D-F06 | | | | | |
| FN34-3A-A09 | | | | | |
| FN34-3A-B01 | | | | | |
| FN34-3AD10 | | | | | |
| FN34-3B-D01 | | | | | |
| FN9-001-B06 | | | | | |
| FN9-001-E11 | | | | | |
| C-AKT3-1 | AKT3 | AKT3 | Q9Y243 | RAC-gamma serine/threonine-protein kinase | PKB gamma |
| C-AKT3-2 | AKT3 | AKT3 | Q9Y243 | RAC-gamma serine/threonine-protein kinase | PKB gamma |
| C-BTK-1 | BTK | BTK | Q06187 | Tyrosine-protein kinase BTK | BTK |
| C-BTK-2 | BKT | BTK | Q06187 | Tyrosine-protein kinase BTK | BTK |
| C-BTK-3 | BTK | BTK | Q06187 | Tyrosine-protein kinase BTK | BTK |
| C-CDK2-2 | CDK2 | CDK2 | P24941 | Cyclin-dependent kinase 2 | CDK-2 |
| C-CDK2-1 | CDK2 | CDK2 | P24941 | Cyclin-dependent kinase 2 | CDK-2 |
| D-CSF2-1 | GM-CSF | CSF2 | P04141 | Granulocyte-macrophage colony-stimulating factor | GM-CSF |
| D-CSF2-3 | GM-CSF | CSF2 | P04141 | Granulocyte-macrophage colony-stimulating factor | GM-CSF |
| D-CSF2-6 | GM-CSF | CSF2 | P04141 | Granulocyte-macrophage colony-stimulating factor | GM-CSF |
| C-FASN-1 | FASN | FASN | Q6PJJ3 | FASN protein | FASN |
| C-FASN-2 | FASN | FASN | Q6PJJ3 | FASN protein | FASN |
| C-FASN-5 | FASN | FASN | Q6PJJ3 | FASN protein | FASN |
| C-FASN-6 | FASN | FASN | Q6PJJ3 | FASN protein | FASN |
| C-GAK-1 | GAK | GAK | Q5U4P5 | GAK protein | GAK |
| C-GAK-3 | GAK | GAK | Q5U4P5 | GAK protein | GAK |
| C-GAK-5 | GAK | GAK | Q5U4P5 | GAK protein | GAK |
| C-HADH2-2 | HADH2 | HADH2 | Q6IBS9 | HADH2 protein | HADH2 |
| C-HADH2-5 | HADH2 | HADH2 | Q6IBS9 | HADH2 protein | HADH2 |
| C-HADH2-7 | HADH2 | HADH2 | Q6IBS9 | HADH2 protein | HADH2 |
| C-HADH2-8 | HADH2 | HADH2 | Q6IBS9 | HADH2 protein | HADH2 |
| D-Her2-1 | Her2/ErbB2 | ERBB2 | P04626 | Receptor tyrosine-protein kinase erbB-2 | Her2/ErbB-2 |
| D-Her2-17 | Her2/ErbB2 | ERBB2 | P04626 | Receptor tyrosine-protein kinase erbB-2 | Her2/ErbB-2 |
| D-Her2-22 | Her2/ErbB2 | ERBB2 | P04626 | Receptor tyrosine-protein kinase erbB-2 | Her2/ErbB-2 |
| C-IL6-3 | IL-6 | IL6 | P05231 | Interleukin-6 | IL-6 |
| C-IL6-4 | IL-6 | IL6 | P05231 | Interleukin-6 | IL-6 |
| C-IL6-5 | IL-6 | IL6 | P05231 | Interleukin-6 | IL-6 |
| C-IL6-6 | IL-6 | IL6 | P05231 | Interleukin-6 | IL-6 |
| C-Keratin19-2 | Keratin 19 | KRT19 | P08727 | Keratin, type I cytoskeletal 19 | Keratin19 |
| C-Keratin19-3 | Keratin 19 | KRT19 | P08727 | Keratin, type I cytoskeletal 19 | Keratin19 |
| C-Keratin19-1 | Keratin 19 | KRT19 | P08727 | Keratin, type I cytoskeletal 19 | Keratin19 |
| C-KSYK-1 | KSYK | SYK | P43405 | Tyrosine-protein kinase SYK | KSYK |
| C-KSYK-2 | KSYK | SYK | P43405 | Tyrosine-protein kinase SYK | KSYK |
| C-MATK-1 | MATK | MATK | P42679 | Megakaryocyte-associated tyrosine-protein kinase | MATK |
| C-MATK-3 | MATK | MATK | P42679 | Megakaryocyte-associated tyrosine-protein kinase | MATK |
| C-MATK-5 | MATK | MATK | P42679 | Megakaryocyte-associated tyrosine-protein kinase | MATK |
| C-MK01-2 | MK01 | MAPK1 | P28482 | Mitogen-activated protein kinase 1 | MAPK1 |
| C-MK01-3 | MK01 | MAPK1 | P28482 | Mitogen-activated protein kinase 1 | MAPK1 |
| C-MK01-5 | MK01 | MAPK1 | P28482 | Mitogen-activated protein kinase 1 | MAPK1 |
| C-MK01-6 | MK01 | MAPK1 | P28482 | Mitogen-activated protein kinase 1 | MAPK1 |
| C-MK08-1 | MK08 | MAPK8 | P45983 | Mitogen-activated protein kinase 8 | MAPK8 |
| C-MK08-2 | MK08 | MAPK8 | P45983 | Mitogen-activated protein kinase 8 | MAPK8 |
| C-MK08-4 | MK08 | MAPK8 | P45983 | Mitogen-activated protein kinase 8 | MAPK8 |
| C-OSTP-1 | OSTP | SPP1 | P10451 | Osteopontin | Osteopontin |
| C-OSTP-2 | OSTP | SPP1 | P10451 | Osteopontin | Osteopontin |
| C-OSTP-3 | OSTP | SPP1 | P10451 | Osteopontin | Osteopontin |
| C-P85A-2 | P85A | PIK3R1 | P27986 | Phosphatidylinositol 3-kinase regulatory subunit alpha | P85A |
| C-P85A-3 | P85A | PIK3R1 | P27986 | Phosphatidylinositol 3-kinase regulatory subunit alpha | P85A |
| C-P85A-4 | P85A | PIK3R1 | P27986 | Phosphatidylinositol 3-kinase regulatory subunit alpha | P85A |
| C-PTK6-1 | PTK6 | PTK6 | Q13882 | Protein-tyrosine kinase 6 | PTK-6 |
| C-PTPN1-2 | PTPN1 | PTPN1 | P18031 | Tyrosine-protein phosphatase non-receptor type 1 | PTP-1B |
| C-PTPN1-3 | PTPN1 | PTPN1 | P18031 | Tyrosine-protein phosphatase non-receptor type 1 | PTP-1B |
| C-PTPN1-1 | PTPN1 | PTPN1 | P18031 | Tyrosine-protein phosphatase non-receptor type 1 | PTP-1B |
| C-RPS6KA2-3 | RPS6KA2 | RPS6KA2 | Q15349 | Ribosomal protein S6 kinase alpha-2 | RPS6KA2 |
| C-RPS6KA2-6 | RPS6KA2 | RPS6KA2 | Q15349 | Ribosomal protein S6 kinase alpha-2 | RPS6KA2 |
| C-RPS6KA2-1 | RPS6KA2 | RPS6KA2 | Q15349 | Ribosomal protein S6 kinase alpha-2 | RPS6KA2 |
| C-STAP2-1 | STAP2 | STAP2 | Q9UGK3 | Signal-transducing adaptor protein 2 | STAP2 |
| C-STAP2-2 | STAP2 | STAP2 | Q9UGK3 | Signal-transducing adaptor protein 2 | STAP2 |
| C-STAP2-3 | STAP2 | STAP2 | Q9UGK3 | Signal-transducing adaptor protein 2 | STAP2 |
| C-STAP2-4 | STAP2 | STAP2 | Q9UGK3 | Signal-transducing adaptor protein 2 | STAP2 |
| C-STAT1-2 | STAT1 | STAT1 | P42224 | Signal transducer and activator of transcription 1-alpha/beta | STAT1 |
| C-STAT1-3 | STAT1 | STAT1 | P42224 | Signal transducer and activator of transcription 1-alpha/beta | STAT1 |
| C-TENS4-1 | TE TENS4 | TNS4 | Q81ZW8 | Tensin-4 | TENS4 |
| C-TNFRSF14-1 | TNFRSF14 | TNFRSF14 | Q92956 | Tumor necrosis factor receptor superfamily member 14 | TNFRSF14 |
| C-TNFRSF14-2 | TNFRSF14 | TNFRSF14 | Q92956 | Tumor necrosis factor receptor superfamily member 14 | TNFRSF14 |
| C-TNFRSF3-1 | TNFRSF3 | LTBR | P36941 | Tumor necrosis factor receptor superfamily member 3 | TNFRSF3 |
| C-TNFRSF3-2 | TNFRSF3 | LTBR | P36941 | Tumor necrosis factor receptor superfamily member 3 | TNFRSF3 |
| C-TNFRSF3-3 | TNFRSF3 | LTBR | P36941 | Tumor necrosis factor receptor superfamily member 3 | TNFRSF3 |
| C-UBC9-1 | UBC9 | UBE21 | P63279 | SUMO-conjugating enzyme UBC9 | UBC9 |
| C-UBC9-3 | UBC9 | UBE21 | P63279 | SUMO-conjugating enzyme UBC9 | UBC9 |
| C-UBC9-4 | UBC9 | UBE21 | P63279 | SUMO-conjugating enzyme UBC9 | UBC9 |
| C-UBE2C-1 | UBE2C | UBE2C | O00762 | Ubiquitin-conjugating enzyme E2 C | UBE2C |
| C-UBE2C-4 | UBE2C | UBE2C | O00762 | Ubiquitin-conjugating enzyme E2 C | UBE2C |
| C-UCHL5-1 | UCHL5 | UCHL5 | Q9Y5K5 | Ubiquitin carboxyl-terminal hydrolase isozyme L5 | UCHL5 |
| Her2 | Her2/ErbB2 | ERBB2 | P04626 | Receptor tyrosine-protein kinase erbB-2 | Her2/ErbB-2 |
| Erbitux | EGFR | EGFR | P00533 | Epidermal growth factor receptor | EGFR |
| I-CBPP22-2 | CBPP22 | CHP1 | Q99653 | Calcineurin B homologous protein 1 | CHP1 |
| I-CBPP22-3 | CBPP22 | CHP1 | Q99653 | Calcineurin B homologous protein 2 | CHP1 |
| I-CENTG1-1 | CENTG1 | AGAP-2 | Q99490 | Arf-GAP with GTPase, ANK repeat and PH domain-containing protein 2 | AGAP-2 |
| I-CENTG1-2 | CENTG1 | AGAP-2 | Q99490 | Arf-GAP with GTPase, ANK repeat and PH domain-containing protein 2 | AGAP-2 |
| I-CENTG1-4 | CENTG1 | AGAP-2 | Q99490 | Arf-GAP with GTPase, ANK repeat and PH domain-containing protein 2 | AGAP-2 |
| I-CENTG1-5 | CENTG1 | AGAP-2 | Q99490 | Arf-GAP with GTPase, ANK repeat and PH domain-containing protein 2 | AGAP-2 |
| I-MAPK9-2 | MAPK9 | MAPK9 | P45984 | Mitogen-activated protein kinase 9 | MAPK9 |
| I-MAPK9-3 | MAPK9 | MAPK9 | P45984 | Mitogen-activated protein kinase 9 | MAPK9 |
| I-MAPK9-4 | MAPK9 | MAPK9 | P45984 | Mitogen-activated protein kinase 9 | MAPK9 |
| I-MAPK9-5 | MAPK9 | MAPK9 | P45984 | Mitogen-activated protein kinase 9 | MAPK9 |
| I-MAPK9-6 | MAPK9 | MAPK9 | P45984 | Mitogen-activated protein kinase 9 | MAPK9 |
| I-MAPK9-7 | MAPK9 | MAPK9 | P45984 | Mitogen-activated protein kinase 9 | MAPK9 |
| I-PAK5-1 | PAK5 | PAK7 | Q9P286 | Serine/threonine-protein kinase PAK 7 | PAK7 |
| I-PAK5-2 | PAK5 | PAK7 | Q9P286 | Serine/threonine-protein kinase PAK 7 | PAK7 |
| I-PAK5-3 | PAK5 | PAK7 | Q9P286 | Serine/threonine-protein kinase PAK 7 | PAK7 |
| I-GEM-1 | GEM | GEM | P55040 | GTP-binding protein GEM | GEM |
| I-GEM-2 | GEM | GEM | P55040 | GTP-binding protein GEM | GEM |
| I-GNAI3-2 | GNAI3 | GNAI3 | P08754 | Guanine nucleotide-binding protein G(k) subunit alpha | GNAI3 |
| I-GNAI3-5 | GNAI3 | GNAI3 | P08754 | Guanine nucleotide-binding protein G(k) subunit alpha | GNAI3 |
| I-GNAI3-6 | GNAI3 | GNAI3 | P08754 | Guanine nucleotide-binding protein G(k) subunit alpha | GNAI3 |
| I-GNAI3-7 | GNAI3 | GNAI3 | P08754 | Guanine nucleotide-binding protein G(k) subunit alpha | GNAI3 |
| I-MAP2K6-2 | MAP2K6 | MAP2K6 | P52564 | Dual specificity mitogen-activated protein kinase kinase 6 | MAPKK 6 |
| I-MAP2K6-3 | MAP2K6 | MAP2K6 | P52564 | Dual specificity mitogen-activated protein kinase kinase 6 | MAPKK 6 |
| I-MAP2K6-4 | MAP2K6 | MAP2K6 | P52564 | Dual specificity mitogen-activated protein kinase kinase 6 | MAPKK 6 |
| I-MAP2K6-7 | MAP2K6 | MAP2K6 | P52564 | Dual specificity mitogen-activated protein kinase kinase 6 | MAPKK 6 |
| I-MAP2K2-1 | MAP2K2 | MAP2K2 | P36507 | Dual specificity mitogen-activated protein kinase kinase 2 | MAPKK 2 |
| I-MAP2K2-5 | MAP2K2 | MAP2K2 | P36507 | Dual specificity mitogen-activated protein kinase kinase 2 | MAPKK 2 |
| I-MAP2K2-8 | MAP2K2 | MAP2K2 | P36507 | Dual specificity mitogen-activated protein kinase kinase 2 | MAPKK 2 |
| I-KRASB-1 | KRASB | KRAS | P01116 | GTPase KRas | KRAS |
| I-PTPRO-3 | PTPRO | PTPRO | Q16827 | Receptor-type tyrosine-protein phosphatase O | R-PTP-O |
| I-PTPRO-4 | PTPRO | PTPRO | Q16827 | Receptor-type tyrosine-protein phosphatase O | R-PTP-O |
| I-PTPRO-9 | PTPRO | PTPRO | Q16827 | Receptor-type tyrosine-protein phosphatase O | R-PTP-O |
| I-PTPRO-10 | PTPRO | PTPRO | Q16827 | Receptor-type tyrosine-protein phosphatase O | R-PTP-O |
| I-PARP6B-2 | PARP6B | PARD6B | Q9BYG5 | Partitioning defective 6 homolog beta | PAR-6B |
| I-PARP6B-6 | PARP6B | PARD6B | Q9BYG5 | Partitioning defective 6 homolog beta | PAR-6B |
| I-PRD14-1 | PRD14 | PRDM14 | Q9GZV8 | PR domain zinc finger protein 14 | PRD14 |
| I-PRD14-2 | PRD14 | PRDM14 | Q9GZV8 | PR domain zinc finger protein 14 | PRD14 |
| I-PRD14-5 | PRD14 | PRDM14 | Q9GZV8 | PR domain zinc finger protein 14 | PRD14 |
| I-PRD14-7 | PRD14 | PRDM14 | Q9GZV8 | PR domain zinc finger protein 14 | PRD14 |
| I-PRD14-8 | PRD14 | PRDM14 | Q9GZV8 | PR domain zinc finger protein 14 | PRD14 |
| I-TOPB1-1 | TOPB1 | TOPBP1 | Q92547 | DNA topoisomerase 2-binding protein 1 | TopBP1 |
| I-TOPB1-2 | TOPB1 | TOPBP1 | Q92547 | DNA topoisomerase 2-binding protein 1 | TopBP1 |
| I-UBP7-3 | UBP7 | USP7 | Q93009 | Ubiquitin carboxyl-terminal hydrolase 7 | UBP7 |
| I-UBP7-4 | UBP7 | USP7 | Q93009 | Ubiquitin carboxyl-terminal hydrolase 7 | UBP7 |
| I-UBP7-6 | UBP7 | USP7 | Q93009 | Ubiquitin carboxyl-terminal hydrolase 7 | UBP7 |
| I-UBP7-10 | UBP7 | USP7 | Q93009 | Ubiquitin carboxyl-terminal hydrolase 7 | UBP7 |
| I-PARP1-8 | PARP1 | PARP1 | P09874 | Poly [ADP-ribose] polymerase 1 | PARP-1 |
| I-GRIP2-1 | GRIP2-1 | GRIP2 | Q9C0E4 | Glutamate receptor-interacting protein 2 | GRIP-2 |
| I-GRIP2-2 | GRIP2-1 | GRIP2 | Q9C0E4 | Glutamate receptor-interacting protein 2 | GRIP-2 |
| I-GRIP2-3 | GRIP2-1 | GRIP2 | Q9C0E4 | Glutamate receptor-interacting protein 2 | GRIP-2 |
| I-GRIP2-4 | GRIP2-1 | GRIP2 | Q9C0E4 | Glutamate receptor-interacting protein 2 | GRIP-2 |
| I-GRIP2-5 | GRIP2-1 | GRIP2 | Q9C0E4 | Glutamate receptor-interacting protein 2 | GRIP-2 |
| I-GRIP2-9 | GRIP2-1 | GRIP2 | Q9C0E4 | Glutamate receptor-interacting protein 2 | GRIP-2 |
| I-GRIP2-10 | GRIP2-1 | GRIP2 | Q9C0E4 | Glutamate receptor-interacting protein 2 | GRIP-2 |
| I-GRIP2-12 | GRIP2-1 | GRIP2 | Q9C0E4 | Glutamate receptor-interacting protein 2 | GRIP-2 |
| I-GRIP2-14 | GRIP2-1 | GRIP2 | Q9C0E4 | Glutamate receptor-interacting protein 2 | GRIP-2 |
| I-GRIP2-18 | GRIP2-1 | GRIP2 | Q9C0E4 | Glutamate receptor-interacting protein 2 | GRIP-2 |
| I-MD2L1-2 | MD2L1 | MAD2L1 | Q13257 | Mitotic spindle assembly checkpoint protein MAD2A | HsMAD2 |
| I-MD2L1-3 | MD2L1 | MAD2L1 | Q13257 | Mitotic spindle assembly checkpoint protein MAD2A | HsMAD2 |
| I-MD2L1-7 | MD2L1 | MAD2L1 | Q13257 | Mitotic spindle assembly checkpoint protein MAD2A | HsMAD2 |
| I-NDC80-2 | NDC80 | NDC80 | O14777 | Kinetochore protein NDC80 homolog | HsHec1 |
| I-NDC80-3 | NDC80 | NDC80 | O14777 | Kinetochore protein NDC80 homolog | HsHec1 |
| I-NDC80-4 | NDC80 | NDC80 | O14777 | Kinetochore protein NDC80 homolog | HsHec1 |
| I-SPDLY-1 | SPDLY-1 | SPDL1 | Q96EA4 | Protein Spindly | hSpindly |
| I-SPDLY-2 | SPDLY-1 | SPDL1 | Q96EA4 | Protein Spindly | hSpindly |
| I-PTPRK-1 | PTPRK | PTPRK | Q15262 | Receptor-type tyrosine-protein phosphatase kappa | R-PTP-kappa |
| I-PTPRK-2 | PTPRK | PTPRK | Q15262 | Receptor-type tyrosine-protein phosphatase kappa | R-PTP-kappa |
| I-PTPRK-4 | PTPRK | PTPRK | Q15262 | Receptor-type tyrosine-protein phosphatase kappa | R-PTP-kappa |
| I-PTPRK-6 | PTPRK | PTPRK | Q15262 | Receptor-type tyrosine-protein phosphatase kappa | R-PTP-kappa |
| I-PTPRK-8 | PTPRK | PTPRK | Q15262 | Receptor-type tyrosine-protein phosphatase kappa | R-PTP-kappa |
| I-PTPRK-9 | PTPRK | PTPRK | Q15262 | Receptor-type tyrosine-protein phosphatase kappa | R-PTP-kappa |
| I-PTPRK-15 | PTPRK | PTPRK | Q15262 | Receptor-type tyrosine-protein phosphatase kappa | R-PTP-kappa |
| I-PTPRK-16 | PTPRK | PTPRK | Q15262 | Receptor-type tyrosine-protein phosphatase kappa | R-PTP-kappa |
| I-PTPRT-2 | PTPRT | PTPRT | O14522 | Receptor-type tyrosine-protein phosphatase T | R-PTP-T |
| I-PTPRT -5 | PTPRT | PTPRT | O14522 | Receptor-type tyrosine-protein phosphatase T | R-PTP-T |
| I-PTPRT -8 | PTPRT | PTPRT | O14522 | Receptor-type tyrosine-protein phosphatase T | R-PTP-T |
| I-PGAM5-1 | PGAM5 | PGAM5 | Q96HS1 | Serine/threonine-protein phosphatase PGAM5, mitochondrial | PGAM5 |
| I-PGAM5-2 | PGAM5 | PGAM5 | Q96HS1 | Serine/threonine-protein phosphatase PGAM5, mitochondrial | PGAM5 |
| I-PGAM5-3 | PGAM5 | PGAM5 | Q96HS1 | Serine/threonine-protein phosphatase PGAM5, mitochondrial | PGAM5 |
| I-PGAM5-4 | PGAM5 | PGAM5 | Q96HS1 | Serine/threonine-protein phosphatase PGAM5, mitochondrial | PGAM5 |
| I-PTPRJ-1 | PTPRJ | PTPRJ | Q12913 | Receptor-type tyrosine-protein phosphatase eta | R-PTP-eta |
| I-PTPRJ-2 | PTPRJ | PTPRJ | Q12913 | Receptor-type tyrosine-protein phosphatase eta | R-PTP-eta |
| I-PTPRJ-3 | PTPRJ | PTPRJ | Q12913 | Receptor-type tyrosine-protein phosphatase eta | R-PTP-eta |
| I-PTPRJ-5 | PTPRJ | PTPRJ | Q12913 | Receptor-type tyrosine-protein phosphatase eta | R-PTP-eta |
| I-PTPRJ-7 | PTPRJ | PTPRJ | Q12913 | Receptor-type tyrosine-protein phosphatase eta | R-PTP-eta |
| I-PTPRJ-8 | PTPRJ | PTPRJ | Q12913 | Receptor-type tyrosine-protein phosphatase eta | R-PTP-eta |
| I-PTPRJ-15 | PTPRJ | PTPRJ | Q12913 | Receptor-type tyrosine-protein phosphatase eta | R-PTP-eta |
| I-PTPRJ-17 | PTPRJ | PTPRJ | Q12913 | Receptor-type tyrosine-protein phosphatase eta | R-PTP-eta |
| N-ANM5-3 | ANM5 | PRMT5 | O14744 | Protein arginine N-methyltransferase 5 | SKB1Hs |
| N-ANM5-4 | ANM5 | PRMT5 | O14744 | Protein arginine N-methyltransferase 5 | SKB1Hs |
| N-ANM5-6 | ANM5 | PRMT5 | O14744 | Protein arginine N-methyltransferase 5 | SKB1Hs |
| N-ANM5-9 | ANM5 | PRMT5 | O14744 | Protein arginine N-methyltransferase 5 | SKB1Hs |
| N-ANM5-10 | ANM5 | PRMT5 | O14744 | Protein arginine N-methyltransferase 5 | SKB1Hs |
| N-APLF.1 | APLF | APLF | Q8IW19 | Aprataxin and PNK-like factor | APLF |
| N-APLF.2 | APLF | APLF | Q8IW19 | Aprataxin and PNK-like factor | APLF |
| N-APLF.3 | APLF | APLF | Q8IW19 | Aprataxin and PNK-like factor | APLF |
| N-APLF.4 | APLF | APLF | Q8IW19 | Aprataxin and PNK-like factor | APLF |
| N-ARHGC-7 | ARHGC-1 | ARHGEF12 | Q9NZN5 | Rho guanine nucleotide exchange factor 12 | |
| N-BIRC2-2 | BIRC2 | BIRC2 | Q13490 | Baculoviral IAP repeat-containing protein 2 | IAP-2 |
| N-BIRC2-4 | BIRC2 | BIRC2 | Q13490 | Baculoviral IAP repeat-containing protein 2 | IAP-2 |
| N-BIRC2-9 | BIRC2 | BIRC2 | Q13490 | Baculoviral IAP repeat-containing protein 2 | IAP-2 |
| N-BIRC2-12 | BIRC2 | BIRC2 | Q13490 | Baculoviral IAP repeat-containing protein 2 | IAP-2 |
| N-DCNL1-2 | DCNL1 | DCUN1D1 | Q96GG9 | DCN1-like protein 1 | |
| N-DCNL1-3 | DCNL1 | DCUN1D1 | Q96GG9 | DCN1-like protein 1 | |
| N-DCNL1-8 | DCNL1 | DCUN1D1 | Q96GG9 | DCN1-like protein 1 | |
| N-DCNL1-9 | DCNL1 | DCUN1D1 | Q96GG9 | DCN1-like protein 1 | |
| N-DLG1-2 | DLG1-1 | DLG1 | Q12959 | Disks large homolog 1 | SAP-97 |
| N-DLG1-5 | DLG1-1 | DLG1 | Q12959 | Disks large homolog 1 | SAP-97 |
| N-DLG1-8 | DLG1-1 | DLG1 | Q12959 | Disks large homolog 1 | SAP-97 |
| N-DLG1-10 | DLG1-1 | DLG1 | Q12959 | Disks large homolog 1 | SAP-97 |
| N-DLG2-2 | DLG2-1 | DLG2 | Q15700 | Disks large homolog 2 | Chapsyn-110 |
| N-DLG2-5 | DLG2-1 | DLG2 | Q15700 | Disks large homolog 2 | Chapsyn-110 |
| N-DLG2-9 | DLG2-1 | DLG2 | Q15700 | Disks large homolog 2 | Chapsyn-110 |
| N-DLG2-13 | DLG2-1 | DLG2 | Q15700 | Disks large homolog 2 | Chapsyn-110 |
| N-DLG2-15 | DLG2-1 | DLG2 | Q15700 | Disks large homolog 2 | Chapsyn-110 |
| N-DPOLM-1 | DPOLM | POLM | Q9NP87 | DNA-directed DNA/RNA polymerase mu | Pol Mu |
| N-DPOLM-2 | DPOLM | POLM | Q9NP87 | DNA-directed DNA/RNA polymerase mu | Pol Mu |
| N-DPOLM-3 | DPOLM | POLM | Q9NP87 | DNA-directed DNA/RNA polymerase mu | Pol Mu |
| N-DPOLM-4 | DPOLM | POLM | Q9NP87 | DNA-directed DNA/RNA polymerase mu | Pol Mu |
| N-DPOLM-5 | DPOLM | POLM | Q9NP87 | DNA-directed DNA/RNA polymerase mu | Pol Mu |
| N-DLG4-3 | DLG4-2 | DLG4 | P78352-2 | Disks large homolog 4 | PSD-95 |
| N-DLG4-5 | DLG4-2 | DLG4 | P78352-2 | Disks large homolog 4 | PSD-95 |
| N-DLG4-7 | DLG4-2 | DLG4 | P78352-2 | Disks large homolog 4 | PSD-95 |
| N-DLG4-8 | DLG4-2 | DLG4 | P78352-2 | Disks large homolog 4 | PSD-95 |
| N-DLG4-10 | DLG4-2 | DLG4 | P78352-2 | Disks large homolog 4 | PSD-95 |
| N-GORS2-3 | GORS2-1 | GORASP2 | Q9H8Y8 | Golgi reassembly-stacking protein 2 | GOLPH6 |
| N-GORS2-5 | GORS2-1 | GORASP2 | Q9H8Y8 | Golgi reassembly-stacking protein 2 | GOLPH6 |
| N-GORS2-11 | GORS2-1 | GORASP2 | Q9H8Y8 | Golgi reassembly-stacking protein 2 | GOLPH6 |
| N-GORS2-13 | GORS2-1 | GORASP2 | Q9H8Y8 | Golgi reassembly-stacking protein 2 | GOLPH6 |
| N-GORS2-16 | GORS2-1 | GORASP2 | Q9H8Y8 | Golgi reassembly-stacking protein 2 | GOLPH6 |
| N-GORS2-20 | GORS2-1 | GORASP2 | Q9H8Y8 | Golgi reassembly-stacking protein 2 | GOLPH6 |
| N-INADL-2 | INADL-1 | INADL | Q8NI35 | InaD-like protein | hINADL |
| N-INADL-5 | INADL-1 | INADL | Q8NI35 | InaD-like protein | hINADL |
| N-INADL-9 | INADL-1 | INADL | Q8NI35 | InaD-like protein | hINADL |
| N-KCC2B-5 | KCC2B-3 | CAMK2B | Q13554-3 | Calcium/calmodulin-dependent protein kinase type II subunit beta | CaM kinase II subunit beta |
| N-KCC2B-2 | KCC2B-3 | CAMK2B | Q13554-3 | Calcium/calmodulin-dependent protein kinase type II subunit beta | CaM kinase II subunit beta |
| N-KCC2B-3 | KCC2B-3 | CAMK2B | Q13554-3 | Calcium/calmodulin-dependent protein kinase type II subunit beta | CaM kinase II subunit beta |
| N-ITCH-2 | ITCH-2 | ITCH | Q96J02-2 | E3 ubiquitin-protein ligase Itchy homolog | Itch |
| N-ITCH-3 | ITCH-2 | ITCH | Q96J02-2 | E3 ubiquitin-protein ligase Itchy homolog | Itch |
| N-ITCH-5 | ITCH-2 | ITCH | Q96J02-2 | E3 ubiquitin-protein ligase Itchy homolog | Itch |
| N-ITCH-10 | ITCH-2 | ITCH | Q96J02-2 | E3 ubiquitin-protein ligase Itchy homolog | Itch |
| N-ITCH-14 | ITCH-2 | ITCH | Q96J02-2 | E3 ubiquitin-protein ligase Itchy homolog | Itch |
| N-KCC4-1 | KCC4 | CAMK4 | Q16566 | Calcium/calmodulin-dependent protein kinase type IV | CaMK IV |
| N-KCC4-8 | KCC4 | CAMK4 | Q16566 | Calcium/calmodulin-dependent protein kinase type IV | CaMK IV |
| N-KCC4-10 | KCC4 | CAMK4 | Q16566 | Calcium/calmodulin-dependent protein kinase type IV | CaMK IV |
| N-KCC4-11 | KCC4 | CAMK4 | Q16566 | Calcium/calmodulin-dependent protein kinase type IV | CaMK IV |
| N-KCC4-5 | KCC4 | CAMK4 | Q16566 | Calcium/calmodulin-dependent protein kinase type IV | CaMK IV |
| N-KCC4-4 | KCC4 | CAMK4 | Q16566 | Calcium/calmodulin-dependent protein kinase type IV | CaMK IV |
| N-KKCC1-1 | KKCC1-1 | CAMKK1 | Q8N5S9-1 | Calcium/calmodulin-dependent protein kinase kinase 1 | CaM-KK 1 |
| N-KKCC1-2 | KKCC1-1 | CAMKK1 | Q8N5S9-1 | Calcium/calmodulin-dependent protein kinase kinase 1 | CaM-KK 1 |
| N-KKCC1-3 | KKCC1-1 | CAMKK1 | Q8N5S9-1 | Calcium/calmodulin-dependent protein kinase kinase 1 | CaM-KK 1 |
| N-KKCC1-4 | KKCC1-1 | CAMKK1 | Q8N5S9-1 | Calcium/calmodulin-dependent protein kinase kinase 1 | CaM-KK 1 |
| N-KKCC1-5 | KKCC1-1 | CAMKK1 | Q8N5S9-1 | Calcium/calmodulin-dependent protein kinase kinase 1 | CaM-KK 1 |
| N-KKCC1-7 | KKCC1-1 | CAMKK1 | Q8N5S9-1 | Calcium/calmodulin-dependent protein kinase kinase 1 | CaM-KK 1 |
| N-LIN7A-2 | LIN7A | LIN7A | O14910 | Protein lin-7 homolog A | Lin-7A |
| N-LIN7A-3 | LIN7A | LIN7A | O14910 | Protein lin-7 homolog A | Lin-7A |
| N-SNTA1-2 | SNTA1 | SNTA1 | Q13424 | Alpha-1-syntrophin | TACIP1 |
| N-SNTA1-3 | SNTA1 | SNTA1 | Q13424 | Alpha-1-syntrophin | TACIP1 |
| N-MAGI1-1 | MAGI1-1 | MAGI1 | Q96QZ7 | Membrane-associated guanylate kinase, WW and PDZ domain-containing protein 1 | AIP-3 |
| N-MAGI1-3 | MAGI1-1 | MAGI1 | Q96QZ7 | Membrane-associated guanylate kinase, WW and PDZ domain-containing protein 1 | AIP-3 |
| N-MARK1-1 | MARK1-1 | MARK1 | Q9POL2-1 | Serine/threonine-protein kinase MARK1 | Par-1c |
| N-MARK1-2 | MARK1-1 | MARK1 | Q9POL2-1 | Serine/threonine-protein kinase MARK1 | Par-1c |
| N-MARK2-1 | MARK2-1 | MARK2 | Q7KZI7-1 | Serine/threonine-protein kinase MARK2 | EMK-1 |
| N-MARK2-2 | MARK2-1 | MARK2 | Q7KZI7-1 | Serine/threonine-protein kinase MARK2 | EMK-1 |
| N-MARK2-3 | MARK2-1 | MARK2 | Q7KZI7-1 | Serine/threonine-protein kinase MARK2 | EMK-1 |
| N-MARK2-4 | MARK2-1 | MARK2 | Q7KZI7-1 | Serine/threonine-protein kinase MARK2 | EMK-1 |
| N-MARK2-5 | MARK2-1 | MARK2 | Q7KZI7-1 | Serine/threonine-protein kinase MARK2 | EMK-1 |
| N-OTU6B-6 | OTU6B | OTUD6B | Q8N6M0 | OTU domain-containing protein 6B | |
| N-OTU6B-9 | OTU6B | OTUD6B | Q8N6M0 | OTU domain-containing protein 6B | |
| N-OTU6B-10 | OTU6B | OTUD6B | Q8N6M0 | OTU domain-containing protein 6B | |
| N-OTU6B-15 | OTU6B | OTUD6B | Q8N6M0 | OTU domain-containing protein 6B | |
| N-OTU6B-16 | OTU6B | OTUD6B | Q8N6M0 | OTU domain-containing protein 6B | |
| N-NOS1-2 | NOS1-1 | NOS1 | P29475 | Nitric oxide synthase, brain | N-NOS |
| N-NOS1-4 | NOS1-1 | NOS1 | P29475 | Nitric oxide synthase, brain | N-NOS |
| N-NOS1-5 | NOS1-1 | NOS1 | P29475 | Nitric oxide synthase, brain | N-NOS |
| N-NOS1-7 | NOS1-1 | NOS1 | P29475 | Nitric oxide synthase, brain | N-NOS |
| N-OTUB1-1 | OTUB1-1 | OTUB1 | Q96FW1-1 | Ubiquitin thioesterase OTUB1 | hOTU1 |
| N-OTUB1-3 | OTUB1-1 | OTUB1 | Q96FW1-1 | Ubiquitin thioesterase OTUB1 | hOTU1 |
| N-OTUB1-4 | OTUB1-1 | OTUB1 | Q96FW1-1 | Ubiquitin thioesterase OTUB1 | hOTU1 |
| N-OTUB1-5 | OTUB1-1 | OTUB1 | Q96FW1-1 | Ubiquitin thioesterase OTUB1 | hOTU1 |
| N-OTUB1-6 | OTUB1-1 | OTUB1 | Q96FW1-1 | Ubiquitin thioesterase OTUB1 | hOTU1 |
| N-OTUB2-1 | OTUB2-1 | OTUB2 | Q96DC9-1 | Ubiquitin thioesterase OTUB2 | |
| N-OTUB2-2 | OTUB2-1 | OTUB2 | Q96DC9-1 | Ubiquitin thioesterase OTUB2 | |
| N-OTUB2-3 | OTUB2-1 | OTUB2 | Q96DC9-1 | Ubiquitin thioesterase OTUB2 | |
| N-OTUB2-4 | OTUB2-1 | OTUB2 | Q96DC9-1 | Ubiquitin thioesterase OTUB2 | |
| N-PAK4-4 | PAK4-1 | PAK4 | O96013-1 | Serine/threonine-protein kinase PAK 4 | PAK-4 |
| N-PAK4-5 | PAK4-1 | PAK4 | O96013-1 | Serine/threonine-protein kinase PAK 4 | PAK-4 |
| N-PAK4-8 | PAK4-1 | PAK4 | O96013-1 | Serine/threonine-protein kinase PAK 4 | PAK-4 |
| N-PRDM8-1 | PRDM8-1 | PRDM8 | Q9NQV8-1 | PR domain zinc finger protein 8 | |
| N-PRDM8-2 | PRDM8-1 | PRDM8 | Q9NQV8-1 | PR domain zinc finger protein 8 | |
| N-PRDM8-4 | PRDM8-1 | PRDM8 | Q9NQV8-1 | PR domain zinc finger protein 8 | |
| N-PRDM8-6 | PRDM8-1 | PRDM8 | Q9NQV8-1 | PR domain zinc finger protein 8 | |
| N-PTN13-1 | PTN13-1 | PTPN13 | Q12923-1 | Tyrosine-protein phosphatase non-receptor type 13 | FAP-1 |
| N-PTN13-2 | PTN13-1 | PTPN13 | Q12923-1 | Tyrosine-protein phosphatase non-receptor type 13 | FAP-1 |
| N-PTN13-4 | PTN13-1 | PTPN13 | Q12923-1 | Tyrosine-protein phosphatase non-receptor type 13 | FAP-1 |
| N-PTN13-6 | PTN13-1 | PTPN13 | Q12923-1 | Tyrosine-protein phosphatase non-receptor type 13 | FAP-1 |
| N-PTN13-8 | PTN13-1 | PTPN13 | Q12923-1 | Tyrosine-protein phosphatase non-receptor type 13 | FAP-1 |
| L-CHEK2-1 | CHEK2 | CHEK2 | O96017 | Serine/threonine-protein kinase Chk2 | Hucds1 |
| L-CHEK2-2 | CHEK2 | CHEK2 | O96017 | Serine/threonine-protein kinase Chk2 | Hucds1 |
| L-CHEK2-3 | CHEK2 | CHEK2 | O96017 | Serine/threonine-protein kinase Chk2 | Hucds1 |
| L-CSNK1E-1 | CSNK1E | CSNK1E | P49674 | Casein kinase I isoform epsilon | CKI-epsilon |
| L-CSNK1E-3 | CSNK1E | CSNK1E | P49674 | Casein kinase I isoform epsilon | CKI-epsilon |
| L-CSNK1E-5 | CSNK1E | CSNK1E | P49674 | Casein kinase I isoform epsilon | CKI-epsilon |
| L-CSNK1E-6 | CSNK1E | CSNK1E | P49674 | Casein kinase I isoform epsilon | CKI-epsilon |
| L-CSNK1E-9 | CSNK1E | CSNK1E | P49674 | Casein kinase I isoform epsilon | CKI-epsilon |
| L-DUSP7-1 | DUSP7 | DUSP7 | Q16829 | Dual specificity protein phosphatase 7 | |
| L-DUSP7-2 | DUSP7 | DUSP7 | Q16829 | Dual specificity protein phosphatase 7 | |
| L-FER-2 | FER | FER | P16591 | Tyrosine-protein kinase Fer | |
| L-FER-5 | FER | FER | P16591 | Tyrosine-protein kinase Fer | |
| L-FER-7 | FER | FER | P16591 | Tyrosine-protein kinase Fer | |
| L-FER-8 | FER | FER | P16591 | Tyrosine-protein kinase Fer | |
| L-FER-9 | FER | FER | P16591 | Tyrosine-protein kinase Fer | |
| L-GPRK5-1 | GPRK5 | GRK5 | P34947 | G protein-coupled receptor kinase 5 | |
| L-PRKCZ-1 | PRKCZ | PRKCZ | Q05513 | Protein kinase C zeta type | |
| L-PRKCZ-2 | PRKCZ | PRKCZ | Q05513 | Protein kinase C zeta type | |
| L-PRKCZ-3 | PRKCZ | PRKCZ | Q05513 | Protein kinase C zeta type | |
| L-PRKCZ-4 | PRKCZ | PRKCZ | Q05513 | Protein kinase C zeta type | |
| L-PRKG2-1 | PRKG2 | PRKG2 | Q13237 | cGMP-dependent protein kinase 2 | cGK 2 |
| L-PRKG2-3 | PRKG2 | PRKG2 | Q13237 | cGMP-dependent protein kinase 2 | cGK 2 |
| L-PTPRD-1 | PTPRD | PTPRD | P23468 | Receptor-type tyrosine-protein phosphatase delta | R-PTP-delt |
| L-PTPRD-2 | PTPRD | PTPRD | P23468 | Receptor-type tyrosine-protein phosphatase delta | R-PTP-delt |
| L-PTPRD-4 | PTPRD | PTPRD | P23468 | Receptor-type tyrosine-protein phosphatase delta | R-PTP-delt |
| L-PTPRD-5 | PTPRD | PTPRD | P23468 | Receptor-type tyrosine-protein phosphatase delta | R-PTP-delt |
| L-PTPRN2-2 | PTPRN2 | PTPRN2 | Q92932 | Receptor-type tyrosine-protein phosphatase N2 | R-PTP-N2 |
| L-PTPRN2-4 | PTPRN2 | PTPRN2 | Q92932 | Receptor-type tyrosine-protein phosphatase N2 | R-PTP-N2 |
| L-PTPRN2-7 | PTPRN2 | PTPRN2 | Q92932 | Receptor-type tyrosine-protein phosphatase N2 | R-PTP-N2 |
| L-PTPRN2-9 | PTPRN2 | PTPRN2 | Q92932 | Receptor-type tyrosine-protein phosphatase N2 | R-PTP-N2 |
| L-PTPRN2-14 | PTPRN2 | PTPRN2 | Q92932 | Receptor-type tyrosine-protein phosphatase N2 | R-PTP-N2 |
| L-PTPRN2-15 | PTPRN2 | PTPRN2 | Q92932 | Receptor-type tyrosine-protein phosphatase N2 | R-PTP-N2 |
| L-SHC1-1 | SHC1 | SHC1 | P29353 | SHC-transforming protein 1 | SH2 domain protein C1 |
| L-SHC1-3 | SHC1 | SHC1 | P29353 | SHC-transforming protein 1 | SH2 domain protein C1 |
| L-STAP1-1 | STAP1 | STAP1 | Q9ULZ2 | Signal-transducing adaptor protein 1 | STAP-1 |
| L-STAP1-2 | STAP1 | STAP1 | Q9ULZ2 | Signal-transducing adaptor protein 1 | STAP-1 |
| L-STAP1-4 | STAP1 | STAP1 | Q9ULZ2 | Signal-transducing adaptor protein 1 | STAP-1 |
| L-STAP1-6 | STAP1 | STAP1 | Q9ULZ2 | Signal-transducing adaptor protein 1 | STAP-1 |
| L-STAP1-7 | STAP1 | STAP1 | Q9ULZ2 | Signal-transducing adaptor protein 1 | STAP-1 |
| L-STAP1-8 | STAP1 | STAP1 | Q9ULZ2 | Signal-transducing adaptor protein 1 | STAP-1 |
| P-AGR3-1 | AGR3 | | Q8TD06 | | |
| P-AGR3-3 | AGR3 | | Q8TD06 | | |
| P-AGR3-5 | AGR3 | | Q8TD06 | | |
| P-AIBP-2 | AIBP | | Q8NCW5 | | |
| P-AIBP-3 | AIBP | | Q8NCW5 | | |
| P-AIBP-8 | AIBP | | Q8NCW5 | | |
| P-AIBP-9 | AIBP | | Q8NCW5 | | |
| P-AIBP-12 | AIBP | | Q8NCW5 | | |
| P-CB39L-2 | CB39L | | Q9H9S4 | | |
| P-CB39L-4 | CB39L | | Q9H9S4 | | |
| P-CB39L-16 | CB39L | | Q9H9S4 | | |
| P-CB39L-26 | CB39L | | Q9H9S4 | | |
| P-CB39L-31 | CB39L | | Q9H9S4 | | |
| P-CDN1B-3 | CDN1B | | P46527 | | |
| P-CDN1B-4 | CDN1B | | P46527 | | |
| P-CDN1B-5 | CDN1B | | P46527 | | |
| P-CDN1B-7 | CDN1B | | P46527 | | |
| P-CDN1B-12 | CDN1B | | P46527 | | |
| P-CYTB-4 | CYTB | | P04080 | | |
| P-CYTB-6 | CYTB | | P04080 | | |
| P-CYTB-8 | CYTB | | P04080 | | |
| P-CYTB-10 | CYTB | | P04080 | | |
| P-CYTB-13 | CYTB | | P04080 | | |
| P-DUS11-1 | DUS11-1 | | O75319-1 | | |
| P-FANCJ-2 | FANCJ-1 | | Q9BX63 | | |
| P-FANCJ-7 | FANCJ-1 | | Q9BX63 | | |
| P-FANCJ-8 | FANCJ-1 | | Q9BX63 | | |
| P-FANCJ-14 | FANCJ-1 | | Q9BX63 | | |
| P-FANCJ-22 | FANCJ-1 | | Q9BX63 | | |
| P-GNAI1-3 | GNAI1 | | P63096 | | |
| P-GNAI1-7 | GNAI1 | | P63096 | | |
| P-GNAI1-8 | GNAI1 | | P63096 | | |
| P-GNAI1-11 | GNAI1 | | P63096 | | |
| P-GNAI1-13 | GNAI1 | | P63096 | | |
| P-G3P-2 | G3P | | P04406 | | |
| P-G3P-4 | G3P | | P04406 | | |
| P-G3P-6 | G3P | | P04406 | | |
| P-GNAS2-2 | GNAS2-1 | | P63092 | | |
| P-GNAS2-4 | GNAS2-1 | | P63092 | | |
| P-GNAS2-7 | GNAS2-1 | | P63092 | | |
| P-GNAS2-9 | GNAS2-1 | | P63092 | | |
| P-GNAS2-10 | GNAS2-1 | | P63092 | | |
| P-GNAI2-3 | GNAI2-1 | | P04899 | | |
| P-GNAI2-6 | GNAI2-1 | | P04899 | | |
| P-GNAI2-14 | GNAI2-1 | | P04899 | | |
| P-GNAI2-22 | GNAI2-1 | | P04899 | | |
| P-GNAI2-25 | GNAI2-1 | | P04899 | | |
| P-MPP7-2 | MPP7 | | Q5T2T1 | | |
| P-MPP7-9 | MPP7 | | Q5T2T1 | | |
| P-PPP5-5 | PPP5 | | P53041 | | |
| P-PPP5-6 | PPP5 | | P53041 | | |
| P-PPP5-7 | PPP5 | | P53041 | | |
| P-PTN2-4 | PTN2-2 | | P17706-2 | | |
| P-PTN2-8 | PTN2-2 | | P17706-2 | | |
| P-PTN2-10 | PTN2-2 | | P17706-2 | | |
| P-PTN2-11 | PTN2-2 | | P17706-2 | | |
| P-PTN2-13 | PTN2-2 | | P17706-2 | | |
| P-SSU72-5 | SSU72-1 | | Q9N P77 | | |
| P-ST17B-1 | ST17B | | O94768 | | |
| P-ST17B-5 | ST17B | | O94768 | | |
| P-ST17B-9 | ST17B | | O94768 | | |
| P-ST17B-10 | ST17B | | O94768 | | |
| P-ST17B-12 | ST17B | | O94768 | | |
| P-TRIB1-5 | TRIB1 | | Q96RU8 | | |
| P-BCR-1 | BCR | | P11274 | | |
| P-BCR-3 | BCR | | P11274 | | |
| P-BCR-4 | BCR | | P11274 | | |
| P-BCR-5 | BCR | | P11274 | | |
| P-BRD2-3 | BRD2-1 | | P25440-1 | | |
| P-BRD2-4 | BRD2-1 | | P25440-1 | | |
| P-BRD2-5 | BRD2-1 | | P25440-1 | | |
| P-BRD2-10 | BRD2-1 | | P25440-1 | | |
| P-BRD2-12 | BRD2-1 | | P25440-1 | | |
| P-C102B-3 | C102B-1 | | Q68D86 | | |
| P-C102B-4 | C102B-1 | | Q68D86 | | |
| P-C102B-6 | C102B-1 | | Q68D86 | | |
| P-C102B-16 | C102B-1 | | Q68D86 | | |
| P-C102B-23 | C102B-1 | | Q68D86 | | |
| O-TP53B-1-1 | TP53B-1 | | Q12888 | | |
| O-TP53B-1-3 | TP53B-1 | | Q12888 | | |
| O-TP53B-1-5 | TP53B-1 | | Q12888 | | |
| O-TP53B-1-6 | TP53B-1 | | Q12888 | | |
| O-TP53B-1-8 | TP53B-1 | | Q12888 | | |
| O-UB2E3-3 | UB2E3 | | Q969T4 | | |
| O-UB2E3-4 | UB2E3 | | Q969T4 | | |
| O-UB2E3-7 | UB2E3 | | Q969T4 | | |
| O-TX1B3-2 | TX1B3 | | O14907 | | |
| O-TX1B3-3 | TX1B3 | | O14907 | | |
| O-TX1B3-4 | TX1B3 | | O14907 | | |
| O-TX1B3-5 | TX1B3 | | O14907 | | |
| O-UBE2A-4 | UBE2A | | P49459 | | |
| O-UBE2A-6 | UBE2A | | P49459 | | |
| O-UBE2A-7 | UBE2A | | P49459 | | |
| O-UB2D3-1-1 | UB2D3-1 | | P61077-1 | | |
| O-UB2D3-1-2 | UB2D3-1 | | P61077-1 | | |
| O-UB2D3-1-3 | UB2D3-1 | | P61077-1 | | |
| O-UBE2W1-2 | UBE2W1 | | Q96B02-1 | | |
| O-GNAI1-1 | GNAI1 | | P63096 | | |
| O-GNAI1-2 | GNAI1 | | P63096 | | |
| O-GNAI1-3 | GNAI1 | | P63096 | | |
| O-GNAI1-6 | GNAI1 | | P63096 | | |
| O-GNAI1-7 | GNAI1 | | P63096 | | |
| O-STABP-5 | STABP | | O95630 | | |
| O-STABP-6 | STABP | | O95630 | | |
| O-STABP-7 | STABP | | O95630 | | |
| O-STABP-8 | STABP | | O95630 | | |
| O-STABP-10 | STABP | | O95630 | | |
| O-ZBT32-2 | ZBT32 | | Q9Y2Y4 | | |
| O-ZBT32-3 | ZBT32 | | Q9Y2Y4 | | |
| O-ZBT32-4 | ZBT32 | | Q9Y2Y4 | | |
| O-ZBT32-6 | ZBT32 | | Q9Y2Y4 | | |
| O-UBE2B-1 | UBE2B | | P63146 | | |
| O-UBE2B-2 | UBE2B | | P63146 | | |
| O-UBE2N-1 | UBE2N | | P61088 | | |
| O-UBE2N-2 | UBE2N | | P61088 | | |
| O-UB2L6-1-1 | UB2L6-1 | | O14933-1 | | |
| O-UB2D2-1 | UB2D2 | | P62837 | | |
| O-ZO3-1 | Z03 | | O95049 | | |
| O-UB2D4-1 | UB2D4 | | Q9Y2X8 | | |
| O-UB2D4-2 | UB2D4 | | Q9Y2X8 | | |
| O-UB2D4-3 | UB2D4 | | Q9Y2X8 | | |
| O-UB2G2-1 | UB2G2 | | P60604 | | |
| O-UB2G2-2 | UB2G2 | | P60604 | | |
| O-UEVLD-1-1 | UEVLD-1 | | Q8IX04-1 | | |
| O-UEVLD-1-2 | UEVLD-1 | | Q8IX04-1 | | |
| Q-BCAR3A-9 | BCAR3A | | O75815 | | |
| Q-BCAR3A-12 | BCAR3A | | O75815 | | |
| Q-BCAR3A-17 | BCAR3A | | O75815 | | |
| Q-BCAR3A-18 | BCAR3A | | O75815 | | |
| Q-CBLBA-4 | CBLBA | | Q13191 | | |
| Q-CBLBA-8 | CBLBA | | Q13191 | | |
| Q-CBLBA-14 | CBLBA | | Q13191 | | |
| Q-CBLBA-18 | CBLBA | | Q13191 | | |
| Q-GRAP2A-2 | GRAP2A | | O75791 | | |
| Q-GRAP2A-3 | GRAP2A | | O75791 | | |
| Q-GRAP2A-5 | GRAP2A | | O75791 | | |
| Q-INPPL1A-4 | INPPL1A | | O15357 | | |
| Q-INPPL1A-6 | INPPL1A | | O15357 | | |
| Q-INPPL1A-8 | INPPL1A | | O15357 | | |
| Q-INPPL1A-13 | INPPL1A | | O15357 | | |
| Q-PARP16A-9 | PARP16A | | Q8N5Y8 | | |
| Q-PARP16A-11 | PARP16A | | Q8N5Y8 | | |
| Q-PARP16A-12 | PARP16A | | Q8N5Y8 | | |
| Q-PARP16A-13 | PARP16A | | Q8N5Y8 | | |
| Q-PLCG2A-1 | PLCG2A | | P16885 | | |
| Q-PLCG2A-2 | PLCG2A | | P16885 | | |
| Q-PLCG2A-4 | PLCG2A | | P16885 | | |
| Q-PARP4A-5 | PARP4A | | Q9UKK3 | | |
| Q-PARP4A-8 | PARP4A | | Q9UKK3 | | |
| Q-PARP4A-9 | PARP4A | | Q9UKK3 | | |
| Q-PARP4A-11 | PARP4A | | Q9UKK3 | | |
| Q-RIN1A-3 | RIN1A | | Q13671 | | |
| Q-RIN2A-2 | RIN2A | | Q8WYP3 | | |
| Q-RIN2A-7 | RIN2A | | Q8WYP3 | | |
| Q-RIN2A-8 | RIN2A | | Q8WYP3 | | |
| Q-RIN2A-10 | RIN2A | | Q8WYP3 | | |
| Q-RIN2A-14 | RIN2A | | Q8WYP3 | | |
| Q-RIN2A-19 | RIN2A | | Q8WYP3 | | |
| Q-SH2D6A-1 | SH2D6A | | Q7Z4S9 | | |
| Q-SH2D6A-2 | SH2D6A | | Q7Z4S9 | | |
| Q-SH2D6A-6 | SH2D6A | | Q7Z4S9 | | |
| Q-ZAP70A-1 | ZAP70A | | P43403 | | |
| Q-ZAP70A-4 | ZAP70A | | P43403 | | |
| Q-ZAP70A-10 | ZAP70A | | P43403 | | |
| Q-STAT4A-6 | STAT4A | | Q14765 | | |
| Q-STAT4A-10 | STAT4A | | Q14765 | | |
| Q-STAT4A-12 | STAT4A | | Q14765 | | |
| Q-STAT4A-14 | STAT4A | | Q14765 | | |
| Q-STAT4A-16 | STAT4A | | Q14765 | | |
| Q-STAT4A-23 | STAT4A | | Q14765 | | |
| Q-TNS4A-6 | TNS4A | | Q8IZW8 | | |
| Q-TNS4A-8 | TNS4A | | Q8IZW8 | | |
| Q-TNS4A-14 | TNS4A | | Q8IZW8 | | |
| Q-TNS4A-19 | TNS4A | | Q8IZW8 | | |
| Q-TNS4A-24 | TNS4A | | Q8IZW8 | | |
| Q-TNS4A-25 | TNS4A | | Q8IZW8 | | |
| Q-TNS4A-30 | TNS4A | | Q8IZW8 | | |
| Q-TXKA-1 | TXKA | | P42681 | | |
| Q-TXKA-3 | TXKA | | P42681 | | |
| Q-TXKA-4 | TXKA | | P42681 | | |
| Q-RASA1A-6 | RASA1A | | P20936 | | |
| Q-RASA1A-7 | RASA1A | | P20936 | | |
| Q-RASA1A-11 | RASA1A | | P20936 | | |
| Q-RASA1A-16 | RASA1A | | P20936 | | |
| Q-BUB1-3 | BUB1 | | O43683 | | |
| Q-BUB1-4 | BUB1 | | O43683 | | |
| Q-BUB1-6 | BUB1 | | O43683 | | |
| Q-BUB1-18 | BUB1 | | O43683 | | |
| Q-CAMKK2B-1 | CAMKK2B | | Q96RR4 | | |
| Q-CAMKK2B-4 | CAMKK2B | | Q96RR4 | | |
| Q-CAMKK2B-8 | CAMKK2B | | Q96RR4 | | |
| Q-CAMKK2B-9 | CAMKK2B | | Q96RR4 | | |
| Q-DUSP10-3 | DUSP10 | | Q9Y6W6 | | |
| Q-DUSP10-4 | DUSP10 | | Q9Y6W6 | | |
| Q-DUSP10-5 | DUSP10 | | Q9Y6W6 | | |
| Q-DUSP10-7 | DUSP10 | | Q9Y6W6 | | |
| Q-PDK1-7 | PDK1 | | Q15118 | | |
| Q-DUSP18-2 | DUSP18 | | Q8NEJ0 | | |
| Q-DUSP18-4 | DUSP18 | | Q8NEJ0 | | |
| Q-DUSP18-7 | DUSP18 | | Q8NEJ0 | | |
| Q-MAPK13-4 | MAPK13 | | O15264 | | |
| Q-MAPK13-6 | MAPK13 | | O15264 | | |
| Q-MYLK-1 | MYLK | | Q15746 | | |
| Q-MYLK-5 | MYLK | | Q15746 | | |
| Q-PDK4-2 | PDK4 | | Q16654 | | |
| Q-PDK4-6 | PDK4 | | Q16654 | | |
| Q-PDK4-10 | PDK4 | | Q16654 | | |
| Q-PDK4-15 | PDK4 | | Q16654 | | |
| Q-PDK4-16 | PDK4 | | Q16654 | | |
| Q-PIM3-8 | PIM3 | | Q86V86 | | |
| Q-PIM3-10 | PIM3 | | Q86V86 | | |
| Q-PIM3-16 | PIM3 | | Q86V86 | | |
| Q-PTP4A2-3 | PTP4A2 | | Q12974 | | |
| Q-PTP4A2-5 | PTP4A2 | | Q12974 | | |
| Q-RNGTT-2 | RNGTT | | O60942 | | |
| Q-RNGTT-8 | RNGTT | | O60942 | | |
| Q-RNGTT-9 | RNGTT | | O60942 | | |
| Q-RPS6KA5-4 | RPS6KA5 | | O75582 | | |
| Q-RPS6KA5-7 | RPS6KA5 | | O75582 | | |
| S-STK3A-1 | STK3 | | Q13188 | | |
| S-STK3A-2 | STK3 | | Q13188 | | |
| S-STK3A-6 | STK3 | | Q13188 | | |
| S-STK3A-12 | STK3 | | Q13188 | | |
| S-STK3A-15 | STK3 | | Q13188 | | |
| S-ALDH2-1 | ALDH2 | | P05091 | | |
| S-ALDH2-4 | ALDH2 | | P05091 | | |
| S-ALDH2-5 | ALDH2 | | P05091 | | |
| S-STK4A-1 | STK4 | | Q13043 | | |
| S-STK4A-2 | STK4 | | Q13043 | | |
| S-TECA-2 | TEC | | P42680 | | |
| S-TECA-3 | TEC | | P42680 | | |
| S-TECA-4 | TEC | | P42680 | | |
| S-STK17AA-6 | STK17A | | Q9UEE5 | | |
| S-STK17AA-7 | STK17A | | Q9UEE5 | | |
| S-STK17AA-10 | STK17A | | Q9UEE5 | | |
| S-STK38LA-5 | STK38L | | Q9Y2H1 | | |
| S-STK38LA-6 | STK38L | | Q9Y2H1 | | |
| S-STK38LA-14 | STK38L | | Q9Y2H1 | | |
| S-VRK1A-2 | VRK1 | | Q99986 | | |
| S-VRK1A-3 | VRK1 | | Q99986 | | |
| S-VRK1A-4 | VRK1 | | Q99986 | | |
| S-RPS6KA6A-2 | RPS6KA6 | | Q9UK32 | | |
| S-RPS6KA6A-4 | RPS6KA6 | | Q9UK32 | | |
| S-RPS6KA6A-10 | RPS6KA6 | | Q9UK32 | | |
| S-RPS6KA6A-12 | RPS6KA6 | | Q9UK32 | | |
| S-TOPKA-7 | TOPK | | Q96KB5 | | |
| S-TOPKA-8 | TOPK | | Q96KB5 | | |
| S-TOPKA-9 | TOPK | | Q96KB5 | | |
| S-TOPKA-16 | TOPK | | Q96KB5 | | |
| S-TOPKA-18 | TOPK | | Q96KB5 | | |
| S-TOPKA-20 | TOPK | | Q96KB5 | | |
| S-CITA-1 | CIT | | O14578 | | |
| S-CITA-5 | CIT | | O14578 | | |
| S-CITA-11 | CIT | | O14578 | | |
| S-CSN2A1A-2 | CSNK2A1 | | P68400 | | |
| S-CSN2A1A-4 | CSNK2A1 | | P68400 | | |
| S-CSN2A1A-9 | CSNK2A1 | | P68400 | | |
| S-CSN2A1A-12 | CSNK2A1 | | P68400 | | |
| S-CSN2A1A-14 | CSNK2A1 | | P68400 | | |
| S-CSN2A1A-15 | CSNK2A1 | | P68400 | | |
| S-CSN2A2A-1 | CSNK2A2 | | P19784 | | |
| S-CSN2A2A-2 | CSNK2A2 | | P19784 | | |
| S-CSN2A2A-5 | CSNK2A2 | | P19784 | | |
| S-CSN2A2A-10 | CSNK2A2 | | P19784 | | |
| S-CSN2A2A-15 | CSNK2A2 | | P19784 | | |
| S-STK39A-2 | STK39 | | Q9UEW8 | | |
| S-STK39A-9 | STK39 | | Q9UEW8 | | |
| S-STK39A-13 | STK39 | | Q9UEW8 | | |
| S-IRS1-4 | IRS1 | | P35568 | | |
| S-IRS1-7 | IRS1 | | P35568 | | |
| S-BRCA1-5 | BRCA1-1 | | P38398-1 | | |
| S-BRCA1-9 | BRCA1-1 | | P38398-1 | | |
| S-BRCA1-12 | BRCA1-1 | | P38398-1 | | |
| S-BRCA1-13 | BRCA1-1 | | P38398-1 | | |
| S-BRCA1-16 | BRCA1-1 | | P38398-1 | | |
| S-BRCA1-18 | BRCA1-1 | | P38398-1 | | |
| S-MMP3-4 | MMP3 | | P08254 | | |
| S-MMP3-6 | MMP3 | | P08254 | | |
| S-MMP3-10 | MMP3 | | P08254 | | |
| S-MMP3-11 | MMP3 | | P08254 | | |
| S-MMP3-22 | MMP3 | | P08254 | | |
| S-MTG8-1 | MTG8-1 | | Q06455 | | |
| S-MTG8-2 | MTG8-1 | | Q06455 | | |
| S-MTG8-7 | MTG8-1 | | Q06455 | | |
| S-MTG8-9 | MTG8-1 | | Q06455 | | |
| S-MTG8-11 | MTG8-1 | | Q06455 | | |
| S-MTG8-12 | MTG8-1 | | Q06455 | | |
| S-PROF1-3 | PROF1 | | P07737 | | |
| S-PROF1-4 | PROF1 | | P07737 | | |
| S-PROF1-6 | PROF1 | | P07737 | | |
| S-PROF1-11 | PROF1 | | P07737 | | |
| S-PROF1-15 | PROF1 | | P07737 | | |
| S-PEBB-2 | PEBB-1 | | Q13951 | | |
| S-PEBB-4 | PEBB-1 | | Q13951 | | |
| S-PEBB-12 | PEBB-1 | | Q13951 | | |
| S-PEBB-14 | PEBB-1 | | Q13951 | | |
| S-TXLNA-4 | TXLNA | | P40222 | | |
| S-TXLNA-10 | TXLNA | | P40222 | | |
| S-TXLNA-13 | TXLNA | | P40222 | | |
| S-TXLNA-14 | TXLNA | | P40222 | | |
| S-TXLNA-17 | TXLNA | | P40222 | | |
| S-ALDR-2 | ALDR | | P15121 | | |
| S-ALDR-12 | ALDR | | P15121 | | |
| S-ALDR-14 | ALDR | | P15121 | | |
| S-ALDR-16 | ALDR | | P15121 | | |
| S-RNF8-1 | RNF8-1 | | O76064-1 | | |
| S-RNF8-3 | RNF8-1 | | O76064-1 | | |
| S-RNF8-6 | RNF8-1 | | O76064-1 | | |
| S-RNF8-12 | RNF8-1 | | O76064-1 | | |
| S-SIRTS1-2 | SIRT5-1 | | Q9NXA8-1 | | |
| S-SIRTS1-5 | SIRT5-1 | | Q9NXA8-1 | | |
| S-SIRTS1-11 | SIRT5-1 | | Q9NXA8-1 | | |
| S-SIRTS1-18 | SIRT5-1 | | Q9NXA8-1 | | |
| S-SIRTS1-22 | SIRT5-1 | | Q9NXA8-1 | | |
| T-MDA5A-2 | IFIH1 | | Q9BYX4 | | |
| T-MDA5A-7 | IFIH1 | | Q9BYX4 | | |
| T-MDA5A-15 | IFIH1 | | Q9BYX4 | | |
| T-MDA5A-16 | IFIH1 | | Q9BYX4 | | |
| T-MDA5A-17 | IFIH1 | | Q9BYX4 | | |
| T-SH3BP2A-13 | SH3BP2 | | P78314 | | |
| T-PARP2A-3 | PARP2 | | Q9UGN5 | | |
| T-PTPN6A-1 | PTPN6 | | P29350 | | |
| T-PTPN6A-5 | PTPN6 | | P29350 | | |
| T-PTPN6A-6 | PTPN6 | | P29350 | | |
| T-PTPN6A-8 | PTPN6 | | P29350 | | |
| T-PTPN6A-9 | PTPN6 | | P29350 | | |
| T-PTPN6A-11 | PTPN6 | | P29350 | | |
| T-PTPN6A-15 | PTPN6 | | P29350 | | |
| T-PTPN6A-16 | PTPN6 | | P29350 | | |
| T-FESA-2 | FES | | P07332 | | |
| T-FESA-3 | FES | | P07332 | | |
| T-FESA-11 | FES | | P07332 | | |
| T-FESA-13 | FES | | P07332 | | |
| T-FESA-14 | FES | | P07332 | | |
| T-FESA-16 | FES | | P07332 | | |
| T-FESA-19 | FES | | P07332 | | |
| T-RIN3A-1 | RIN3 | | Q8TB24 | | |
| T-RIN3A-2 | RIN3 | | Q8TB24 | | |
| T-RIN3A-4 | RIN3 | | Q8TB24 | | |
| T-RIN3A-6 | RIN3 | | Q8TB24 | | |
| T-RIN3A-7 | RIN3 | | Q8TB24 | | |
| T-RIN3A-10 | RIN3 | | Q8TB24 | | |
| T-ULK4A-1 | ULK4 | | Q96C45 | | |
| T-ULK4A-5 | ULK4 | | Q96C45 | | |
| T-ULK4A-7 | ULK4 | | Q96C45 | | |
| T-ITKA-4 | ITK | | Q08881 | | |
| T-ITKA-8 | ITK | | Q08881 | | |
| T-ITKA-10 | ITK | | Q08881 | | |
| T-ITKA-12 | ITK | | Q08881 | | |
| T-ITKA-13 | ITK | | Q08881 | | |
| T-ZC3HAV1A-3 | ZC3HAV1 | | Q7Z2W4 | | |
| T-ZC3HAV1A-9 | ZC3HAV1 | | Q7Z2W4 | | |
| T-ZC3HAV1A-10 | ZC3HAV1 | | Q7Z2W4 | | |
| T-SH2D1AA-2 | SH2D1A | | O60880 | | |
| T-TNS3A-6 | TNS3 | | Q68CZ2 | | |
| T-TNS3A-11 | TNS3 | | Q68CZ2 | | |
| T-CSKA-9 | CSK | | P41240 | | |
| T-CSKA-11 | CSK | | P41240 | | |
| T-CSKA-14 | CSK | | P41240 | | |
| T-CSKA-16 | CSK | | P41240 | | |
| T-VAV2A-1 | VAV2 | | P52735 | | |
| T-VAV2A-4 | VAV2 | | P52735 | | |
| T-VAV2A-7 | VAV2 | | P52735 | | |
| T-VAV2A-13 | VAV2 | | P52735 | | |
| T-PTPN11A-16 | PTPN11 | | Q06124 | | |
| T-SH2D1BA-5 | SH2D1B | | O14796 | | |
| T-SH2D1BA-6 | SH2D1B | | O14796 | | |
| T-SH2D4AA-7 | SH2D4A | | Q9H788 | | |
| T-SH2D4AA-10 | SH2D4A | | Q9H788 | | |
| T-SH2D4AA-11 | SH2D4A | | Q9H788 | | |
| T-SH2D4AA-16 | SH2D4A | | Q9H788 | | |
| T-SRMSA-2 | SRMS | | Q9H3Y6 | | |
| T-SRMSA-3 | SRMS | | Q9H3Y6 | | |
| T-SRMSA-6 | SRMS | | Q9H3Y6 | | |
| T-PI K3C3A-1 | PIK3C3 | | Q8NEB9 | | |
| T-PI K3C3A-5 | PIK3C3 | | Q8NEB9 | | |
| T-PI K3C3A-16 | PIK3C3 | | Q8NEB9 | | |
| T-PARP12A-5 | PARP12 | | Q9H0J9 | | |
| T-PARP12A-10 | PARP12 | | Q9H0J9 | | |
| T-PARP12A-16 | PARP12 | | Q9H0J9 | | |
| T-PARP12A-17 | PARP12 | | Q9H0J9 | | |
| T-CBLCA-10 | CBLC | | Q9ULV8 | | |
| T-CBLCA-13 | CBLC | | Q9ULV8 | | |
| T-DDX58A-3 | DDX58 | | O95786 | | |
| T-DDX58A-5 | DDX58 | | O95786 | | |
| T-DDX58A-6 | DDX58 | | O95786 | | |
| T-DDX58A-9 | DDX58 | | O95786 | | |
| T-DDX58A-12 | DDX58 | | O95786 | | |
| T-INPP5BA-4 | INPP5B | | P32019 | | |
| T-INPP5BA-5 | INPP5B | | P32019 | | |
| T-INPP5BA-13 | INPP5B | | P32019 | | |
| T-INPP5BA-15 | INPP5B | | P32019 | | |
| T-PIP5K2CA-2 | PIP5K2C | | Q8TBX8 | | |
| T-PIP5K2CA-6 | PIP5K2C | | Q8TBX8 | | |
| T-PIP5K2CA-8 | PIP5K2C | | Q8TBX8 | | |
| T-PIP5K2CA-24 | PIP5K2C | | Q8TBX8 | | |
| T-SYNJ2A-1 | SYNJ2 | | O15056 | | |
| T-SYNJ2A-3 | SYNJ2 | | O15056 | | |
| T-SYNJ2A-4 | SYNJ2 | | O15056 | | |
| T-SYNJ2A-6 | SYNJ2 | | O15056 | | |
| T-SYNJ2A-11 | SYNJ2 | | O15056 | | |
| T-SYNJ2A-14 | SYNJ2 | | O15056 | | |
| T-SYNJ2A-22 | SYNJ2 | | O15056 | | |
| T-SHFA-1 | SHF | | Q7M4L6 | | |
| T-DDX47A--5 | DDX47 | | Q9H0S4 | | |
| T-DDX47A--8 | DDX47 | | Q9H0S4 | | |
| T-DDX47A--18 | DDX47 | | Q9H0S4 | | |
| T-ATF1-17 | ATF1 | | P18846 | | |

**Table 6: ROC-AUCs of selected biomarker combinations**

| ***Name in patent table*** | ***Short name*** | 0.6 | 0.53 | 0.52 | 0.62 | 0.58 | 0.62 | 0.64 | 0.62 | 0.69 | 0.65 | 0.67 | 0.66 | 0.69 | 0.66 | 0.64 | 0.62 | 0.72 |
|---|---|---|---|---|---|---|---|---|---|---|---|---|---|---|---|---|---|---|
| PRD14 | PRD14 | 1 | 1 | 1 | 1 | 1 | 1 | 1 | 1 | 1 | 1 | 1 | 1 | 1 | 1 | 1 | 1 | 1 |
| HsHec1 | HsHec1 | | 2 | 2 | 2 | 2 | 2 | 2 | 2 | 2 | 2 | 2 | 2 | 2 | 2 | 2 | 2 | 2 |
| hSpindly | hSpindly | | | 3 | 3 | 3 | 3 | 3 | 3 | 3 | 3 | 3 | 3 | 3 | 3 | 3 | 3 | 3 |
| GNAI3 | GNAI3 | | | | 4 | 4 | 4 | 4 | 4 | 4 | 4 | 4 | 4 | 4 | 4 | 4 | 4 | 4 |
| GRP2 | GRIP-2 | | | | | 5 | 5 | 5 | 5 | 5 | 5 | 5 | 5 | 5 | 5 | 5 | 5 | 5 |
| HsMAD2 | HsMAD2 | | | | | | 6 | 6 | 6 | 6 | 6 | 6 | 6 | 6 | 6 | 6 | 6 | 6 |
| KIA_G4 | TBC1D9 | | | | | | | 7 | 7 | 7 | 7 | 7 | 7 | 7 | 7 | 7 | 7 | 7 |
| MAPK 6 | MAPKK6 | | | | | | | 8 | 8 | 8 | 8 | 8 | 8 | 8 | 8 | 8 | 8 | 8 |
| MAPK 9 | MAPK9 | | | | | | | | | 9 | 9 | 9 | 9 | 9 | 9 | 9 | 9 | 9 |
| MAPK8 | MAPK8 | | | | | | | | | | 10 | 10 | 10 | 10 | 10 | 10 | 10 | 10 |
| oxy | ORP-3 | | | | | | | | | | | 11 | 11 | 11 | 11 | 11 | 11 | 11 |
| P3-15 | MUC1 | | | | | | | | | | | | 12 | 12 | 12 | 12 | 12 | 12 |
| PTK6 | PTK6 | | | | | | | | | | | | | 13 | 13 | 13 | 13 | 13 |
| PTPN1 | PTPN1 | | | | | | | | | | | | | | 14 | 14 | 14 | 14 |
| PTPRJ | R-PTP-eta | | | | | | | | | | | | | | | 15 | 15 | 15 |
| R-PTP-O | R-PTP-O | | | | | | | | | | | | | | | | 16 | 16 |
| PGAM5 | Q96HS1 | | | | | | | | | | | | | | | | | 17 |

## Claims

1. A method for determining the location of a tumour in the pancreas of an individual with pancreatic cancer, the method comprising or consisting of the steps of:
a) providing a sample to be tested from the individual;
b) determining a biomarker signature of the test sample by measuring the expression in the test sample of two or more biomarkers selected from the group defined in Table A (i), (ii) or (iii);
wherein the two or more biomarkers selected from the group defined in Table A (i), (ii) or (iii) include PR domain zinc finger protein 14 (PRD14) and kinetochore protein NDC80 homolog (HsHec1); and
wherein the expression in the test sample of the two or more biomarkers selected from the group defined in Table A (i), (ii) or (iii) is indicative of the location of the tumour in the pancreas of the individual.

2. The method according to any one of the preceding claims further comprising or consisting of the steps of:
c) providing a control sample from an individual afflicted with pancreatic cancer located in and/or originating from the head of the pancreas; and
d) determining a biomarker signature of the control sample by measuring the expression in the control sample of the two or more biomarkers measured in step (b);
wherein the location of pancreatic cancer in the test sample is identified as being located in and/or originating from head of the pancreas in the event that the expression in the test sample of the two or more biomarkers measured in step (b) corresponds to the expression in the control sample of the two or more biomarkers measured in step (d); and
wherein the location of pancreatic cancer in the test sample is identified as being located in and/or originating from the body and/or tail of the pancreas in the event that the expression in the test sample of the two or more biomarkers measured in step (b) is different from the expression in the control sample of the two or more biomarkers measured in step (d).

3. The method according to any one of the preceding claims further comprising or consisting of the steps of:
e) providing a control sample from an individual afflicted with pancreatic cancer located in and/or originating from the body and/or tail of the pancreas; and
f) determining a biomarker signature of the control sample by measuring the expression in the control sample of the two or more biomarkers measured in step (b);
wherein the location of pancreatic cancer in the test sample is identified as being located in and/or originating from the body and/or tail of the pancreas in the event that the expression in the test sample of the two or more biomarkers measured in step (b) corresponds to the expression in the control sample of the two or more biomarkers measured in step (f); and
wherein the location of pancreatic cancer in the test sample is identified as being located in and/or originating from the head of the pancreas in the event that the expression in the test sample of the two or more biomarkers measured in step (b) is different from the expression in the control sample of the two or more biomarkers measured in step (f).

4. The method according to any one of the preceding claims wherein step (b) comprises or consists of measuring the expression of one or more of the biomarkers listed in Table A, for example, at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123 or 124 of the biomarkers listed in Table A.

5. The method according to any one of the preceding claims, wherein step (b) comprises or consists of
(i) measuring the expression of 1 or more of the biomarkers listed in Table (A)(ii), for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 or 46 of the biomarkers listed in Table A(ii),
(ii) measuring the expression of 1 or more biomarkers from the biomarkers listed in Table A(iii), for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 of the biomarkers listed in Table A(iii), and/or
(iii) measuring the expression of 1 or more biomarkers from the biomarkers listed in Table A(iv), for example at least 2, 3, 4, 5, 6, 7, 8, 9, 10, 11, 12, 13, 14, 15, 16, 17, 18, 19, 20, 21, 22, 23, 24, 25 or 26 of the biomarkers listed in Table A(iv).

6. The method according to any one of the preceding claims wherein step (b) comprises or consists of measuring the expression in the test sample of all of the biomarkers defined in Table A.

7. The method according to any one of the preceding claims wherein the pancreatic cancer is an adenocarcinoma, for example, pancreatic ductal adenocarcinoma.

8. The method according to any one of the preceding claims wherein step (b), (d), and/or step (f) is performed using antibodies or antigen-binding fragments thereof capable of binding to the two or more biomarkers.

9. The method according to Claim 8 wherein the antibodies or antigen-binding fragments thereof are immobilised on a surface.

10. The method according to any one of the preceding claims wherein step (b), (d), and/or step (f) is performed using an array.

11. The method according to any one of the preceding claims wherein the method comprises:
(i) labelling biomarkers present in the sample with biotin;
(ii) contacting the biotin-labelled proteins with an array comprising a plurality of scFv immobilised at discrete locations on its surface, the scFv having specificity for two or more of the proteins in Table A;
(iii) contacting the immobilised scFv with a streptavidin conjugate comprising a fluorescent dye; and
(iv) detecting the presence of the dye at discrete locations on the array surface
wherein the expression of the dye on the array surface is indicative of the expression of a biomarker from Table A in the sample.

12. The method according to any one of the preceding claims wherein, step (b), (d), and/or step (f) comprises measuring the expression of a nucleic acid molecule encoding the one or more biomarkers.

13. The method according to any one of the preceding claims wherein, the sample provided in step (b) is selected from the group consisting of unfractionated blood, plasma, serum, tissue fluid, pancreatic tissue, pancreatic juice, bile and urine.

14. The method according to any one of the preceding claims wherein the predicative accuracy of the method, as determined by an ROC AUC value, is at least 0.70.

15. An array for determining the location of a tumour in the pancreas of an individual with pancreatic cancer, the array comprising antibodies or antigen-binding fragments thereof capable of binding to two or more biomarkers selected from the group defined in Table A (i), (ii) or (iii)
wherein the two or more biomarkers selected from the group defined in Table A (i), (ii) or (iii) include PR domain zinc finger protein 14 (PRD14) and kinetochore protein NDC80 homolog (HsHec1).

## Patentansprüche

1. Verfahren zum Bestimmen der Stelle eines Tumors in der Bauchspeicheldrüse eines Individuums mit Bauchspeicheldrüsenkrebs, wobei das Verfahren die folgenden Schritte umfasst oder aus diesen besteht:
a) Bereitstellen einer zu untersuchenden Probe von dem Individuum;
b) Bestimmen einer Biomarkersignatur der Untersuchungsprobe durch Messen der Expression in der Untersuchungsprobe von zwei oder mehr Biomarkern, die aus der in Tabelle A (i), (ii) oder (iii) definierten Gruppe ausgewählt sind;
wobei die zwei oder mehr Biomarker, die aus der in Tabelle A (i), (ii) oder (iii) definierten Gruppe ausgewählt sind, PR-Domänenzinkfingerprotein 14 (PRD14) und Kinetochorprotein-NDC80-Homolog (HsHec1) einschließen; und
wobei die Expression in der Untersuchungsprobe der zwei oder mehr Biomarker, die aus der in Tabelle A (i), (ii) oder (iii) definierten Gruppe ausgewählt sind, die Stelle des Tumors in der Bauchspeicheldrüse des Individuums anzeigt.

2. Verfahren nach einem der vorhergehenden Ansprüche, das ferner die folgenden Schritte umfasst oder aus diesen besteht:
c) Bereitstellen einer Kontrollprobe von einem Individuum, die an Bauchspeicheldrüsenkrebs leidet, der in dem Kopf der Bauchspeicheldrüse gelegen ist und/oder von diesem ausgeht; und
b) Bestimmen einer Biomarkersignatur der Kontrollprobe durch Messen der Expression in der Kontrollprobe der zwei oder mehr in Schritt (b) gemessenen Biomarker;
wobei die Stelle des Bauchspeicheldrüsenkrebses in der Untersuchungsprobe als in dem Kopf der Bauchspeicheldrüse gelegen und/oder von diesem ausgehend identifiziert wird, falls die in Schritt (b) gemessene Expression in der Untersuchungsprobe der zwei oder mehr Biomarker der Expression in der Kontrollprobe der zwei oder mehr in Schritt (d) gemessenen Biomarker entspricht; und
wobei die Stelle des Bauchspeicheldrüsenkrebses in der Untersuchungsprobe als in dem Körper und/oder dem Schwanz der Bauchspeicheldrüse gelegen und/oder von diesem ausgehend identifiziert wird, falls die in Schritt (b) gemessene Expression in der Untersuchungsprobe der zwei oder mehr Biomarker sich von der Expression in der Kontrollprobe der zwei oder mehr in Schritt (d) gemessenen Biomarker unterscheidet.

3. Verfahren nach einem der vorhergehenden Ansprüche, das ferner die folgenden Schritte umfasst oder aus diesen besteht:
e) Bereitstellen einer Kontrollprobe von einem Individuum, das an Bauchspeicheldrüsenkrebs leidet, der in dem Körper und/oder dem Schwanz der Bauchspeicheldrüse gelegen ist und/oder von diesem ausgeht; und
f) Bestimmen einer Biomarkersignatur der Kontrollprobe durch Messen der Expression in der Kontrollprobe der zwei oder mehr in Schritt (b) gemessenen Biomarker;
wobei die Stelle des Bauchspeicheldrüsenkrebses in der Untersuchungsprobe als in dem Körper und/oder dem Schwanz der Bauchspeicheldrüse gelegen und/oder von diesem ausgehend identifiziert wird, falls die in Schritt (b) gemessene Expression in der Untersuchungsprobe der zwei oder mehr Biomarker der Expression in der Kontrollprobe der zwei oder mehr in Schritt (f) gemessenen Biomarker entspricht; und
wobei die Stelle des Bauchspeicheldrüsenkrebses in der Untersuchungsprobe als in dem Kopf der Bauchspeicheldrüse gelegen und/oder von diesem ausgehend identifiziert wird, falls die in Schritt (b) gemessene Expression in der Untersuchungsprobe der zwei oder mehr Biomarker sich von der Expression in der Kontrollprobe der zwei oder mehr in Schritt (f) gemessenen Biomarker unterscheidet.

4. Verfahren nach einem der vorhergehenden Ansprüche, wobei Schritt (b) ein Messen der Expression eines oder mehrerer der in Tabelle A aufgelisteten Biomarker umfasst, oder daraus besteht, zum Beispiel wenigstens 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123 oder 124 der in Tabelle A aufgelisteten Biomarker.

5. Verfahren nach einem der vorhergehenden Ansprüche, wobei Schritt (b) Folgendes umfasst oder daraus besteht:
(i) Messen der Expression von 1 oder mehreren der in Tabelle (A)(ii) aufgelisteten Biomarker, zum Beispiel wenigstens 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 oder 46 der in Tabelle A(ii) aufgelisteten Biomarker,
(ii) Messen der Expression von 1 oder mehreren Biomarkern aus den in Tabelle A(iii) aufgelisteten Biomarkern, zum Beispiel wenigstens 2, 3, 4, 5, 6, 7, 8, 9, 10, 11, 12, 13, 14, 15, 16, 17, 18,
19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 oder 50 der in Tabelle A(iii) aufgelisteten Biomarker und/oder
(iii) Messen der Expression von 1 oder mehreren Biomarkern aus den in Tabelle A(iv) aufgelisteten Biomarkern, zum Beispiel wenigstens 2, 3, 4, 5, 6, 7, 8, 9, 10, 11, 12, 13, 14, 15, 16, 17, 18, 19, 20, 21, 22, 23, 24, 25 oder 26 der in Tabelle A(iv) aufgelisteten Biomarker.

6. Verfahren nach einem der vorhergehenden Ansprüche, wobei Schritt (b) das Messen der Expression in der Untersuchungsprobe all der in Tabelle A definierten Biomarker umfasst oder daraus besteht.

7. Verfahren nach einem der vorhergehenden Ansprüche, wobei der Bauchspeicheldrüsenkrebs ein Adenokarzinom ist, zum Beispiel duktales Bauchspeicheldrüsenadenokarzinom.

8. Verfahren nach einem der vorhergehenden Ansprüche, wobei Schritt (b), (d) und/oder Schritt (f) unter Verwendung von Antikörpern oder antigenbindenden Fragmenten davon durchgeführt wird, die zur Bindung an die zwei oder mehr Biomarker fähig sind.

9. Verfahren nach Anspruch 8, wobei die Antikörper oder antigenbindenden Fragmente davon auf einer Oberfläche immobilisiert sind.

10. Verfahren nach einem der vorhergehenden Ansprüche, wobei Schritt (b), (d), und/oder Schritt (f) unter Verwendung eines Arrays durchgeführt wird.

11. Verfahren nach einem der vorhergehenden Ansprüche, wobei das Verfahren Folgendes umfasst:
(i) Markieren von in der Probe vorhandenen Biomarkern mit Biotin;
(ii) Inberührungbringen der Biotin-markierten Proteine mit einem Array, umfassend mehrere scFv, die an separaten Stellen auf ihrer Oberfläche immobilisiert sind, wobei das scFv eine Spezifität für zwei oder mehr der Proteine in Tabelle A aufweist;
(iii) Inberührungbringen des immobilisierten scFv mit einem Streptavidinkonjugat, das einen fluoreszierenden Farbstoff umfasst; und
(iv) Nachweisen des Vorhandenseins des Farbstoffs an separaten Stellen auf der Arrayoberfläche
wobei die Expression des Farbstoffs auf der Arrayoberfläche die Expression eines Biomarkers aus Tabelle A in der Probe anzeigt.

12. Verfahren nach einem der vorhergehenden Ansprüche, wobei Schritt (b), (d) und/oder Schritt (f) das Messen der Expression eines Nukleinsäuremoleküls, das für den einen oder die mehreren Biomarker codiert, umfasst.

13. Verfahren nach einem der vorhergehenden Ansprüche, wobei die in Schritt (b) bereitgestellte Probe aus der Gruppe ausgewählt ist, die aus unfraktioniertem Blut, Plasma, Serum, Gewebefluid, Bauchspeicheldrüsengewebe, Bauchspeicheldrüsensaft, Galle und Urin besteht.

14. Verfahren nach einem der vorhergehenden Ansprüche, wobei die Vorhersagegenauigkeit des Verfahrens, wie durch einen ROC-AUC-Wert bestimmt, wenigstens 0,70 beträgt.

15. Array zum Bestimmen der Stelle eines Tumors in der Bauchspeicheldrüse eines Individuums mit Bauchspeicheldrüsenkrebs, wobei das Array Antikörper oder antigenbindende Fragmente davon umfasst, die zur Bindung an zwei oder mehr Biomarker fähig sind, die aus der in Tabelle A (i), (ii) oder (iii) definierten Gruppe ausgewählt sind
wobei die zwei oder mehr Biomarker, die aus der in Tabelle A (i), (ii) oder (iii) definierten Gruppe ausgewählt sind, PR-Domänenzinkfingerprotein 14 (PRD14) und Kinetochorprotein-NDC80-Homolog (HsHec1) einschließen.

## Revendications

1. Procédé de localisation d'une tumeur dans le pancréas d'un individu atteint d'un cancer du pancréas, le procédé comprenant ou comportant les étapes consistant à :
a) fournir un échantillon à tester provenant de l'individu ;
b) déterminer une signature de biomarqueur de l'échantillon de test en mesurant l'expression dans l'échantillon de test d'au moins deux biomarqueurs choisis dans le groupe défini dans le tableau A(i), (ii) ou (iii) ;
les au moins deux biomarqueurs sélectionnés dans le groupe défini dans le tableau A(i), (ii) ou (iii) comprenant la protéine en doigt de zinc 14 du domaine PR (PRD14) et l'homologue de la protéine du kinétochore NDC80 (HsHec1) ; et
l'expression dans l'échantillon de test des au moins deux biomarqueurs choisis dans le groupe défini dans le tableau A(i), (ii) ou (iii) indiquant la position de la tumeur dans le pancréas de l'individu.

2. Procédé selon l'une quelconque des revendications précédentes, comprenant ou comportant en outre les étapes consistant à :
c) fournir un échantillon témoin provenant d'un individu atteint d'un cancer du pancréas situé dans la tête du pancréas et/ou provenant de celle-ci ; et à
d) déterminer une signature de biomarqueur de l'échantillon témoin en mesurant l'expression dans l'échantillon témoin des au moins deux biomarqueurs mesurés à l'étape (b) ;
la position du cancer du pancréas dans l'échantillon de test étant identifiée comme étant située dans la tête du pancréas et/ou provenant de celle-ci dans le cas où l'expression dans l'échantillon de test des au moins deux biomarqueurs mesurés à l'étape (b) correspond à l'expression dans l'échantillon témoin des au moins deux biomarqueurs mesurés à l'étape (d) ; et
la position du cancer du pancréas dans l'échantillon de test étant identifiée comme étant située dans le corps et/ou la queue du pancréas et/ou provenant de celui-ci/celle-ci dans le cas où l'expression dans l'échantillon de test des au moins deux biomarqueurs mesurés à l'étape (b) est différente de l'expression dans l'échantillon témoin des au moins deux biomarqueurs mesurés à l'étape (d).

3. Procédé selon l'une quelconque des revendications précédentes, comprenant ou comportant en outre les étapes consistant à :
e) fournir un échantillon témoin provenant d'un individu atteint d'un cancer du pancréas situé dans le corps et/ou la queue du pancréas et/ou provenant de celui-ci/celle-ci ; et à
f) déterminer une signature de biomarqueur de l'échantillon témoin en mesurant l'expression dans l'échantillon témoin des au moins deux biomarqueurs mesurés à l'étape (b) ;
la position du cancer du pancréas dans l'échantillon de test étant identifiée comme étant située dans le corps et/ou la queue du pancréas et/ou provenant de celui-ci/celle-ci dans le cas où l'expression dans l'échantillon de test des au moins deux biomarqueurs mesurés à l'étape (b) correspond à l'expression dans l'échantillon témoin des au moins deux biomarqueurs mesurés à l'étape (f) ; et
la position du cancer du pancréas dans l'échantillon de test étant identifiée comme étant située dans la tête du pancréas et/ou provenant de celle-ci dans le cas où l'expression dans l'échantillon de test des au moins deux biomarqueurs mesurés à l'étape (b) est différente de l'expression dans l'échantillon témoin des au moins deux biomarqueurs mesurés à l'étape (f).

4. Procédé selon l'une quelconque des revendications précédentes, dans lequel l'étape (b) comprend ou consiste en la mesure de l'expression d'un ou de plusieurs des biomarqueurs énumérés dans le tableau A, par exemple au moins 3, 4, 5, 6, 7, 8, 9, 10, 11,12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123 ou 124 des biomarqueurs énumérés dans le tableau A.

5. Procédé selon l'une quelconque des revendications précédentes, dans lequel l'étape (b) comprend ou consiste en la
(i) mesure de l'expression d'un ou de plusieurs des biomarqueurs énumérés dans le tableau A(ii), par exemple au moins 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 ou 46 des biomarqueurs énumérés dans le tableau A(ii),
(ii) la mesure de l'expression d'un ou de plusieurs biomarqueurs parmi les biomarqueurs énumérés dans le tableau A(iii), par exemple au moins 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 ou 50 des biomarqueurs énumérés dans le tableau A(iii), et/ou
(iii) la mesure de l'expression d'un ou de plusieurs biomarqueurs parmi les biomarqueurs énumérés dans le tableau A(iv), par exemple au moins 2, 3, 4, 5, 6, 7, 8, 9, 10, 11, 12, 13, 14, 15, 16, 17, 18, 19, 20, 21, 22, 23, 24, 25 ou 26 des biomarqueurs énumérés au tableau A(iv).

6. Procédé selon l'une quelconque des revendications précédentes, dans lequel l'étape (b) comprend ou consiste en la mesure de l'expression dans l'échantillon de test de tous les biomarqueurs définis dans le tableau A.

7. Procédé selon l'une quelconque des revendications précédentes, dans lequel le cancer du pancréas est un adénocarcinome, par exemple l'adénocarcinome canalaire du pancréas.

8. Procédé selon l'une quelconque des revendications précédentes, dans lequel l'étape (b), (d) et/ou l'étape (f) est effectuée en utilisant des anticorps ou leurs fragments de liaison d'antigène capables de se lier aux au moins deux biomarqueurs.

9. Procédé selon la revendication 8, dans lequel les anticorps ou leurs fragments de liaison d'antigène sont immobilisés sur une surface.

10. Procédé selon l'une quelconque des revendications précédentes, dans lequel l'étape (b), (d) et/ou (f) est effectuée au moyen d'un réseau.

11. Procédé selon l'une quelconque des revendications précédentes, le procédé consistant à :
(i) marquer, avec de la biotine, les biomarqueurs présents dans l'échantillon ;
(ii) mettre les protéines marquées à la biotine en contact avec un réseau comprenant une pluralité de scFv immobilisés à des positions discrètes de sa surface, le scFv ayant une spécificité pour au moins deux des protéines du tableau A ;
(iii) mettre le scFv immobilisé en contact avec un conjugué de streptavidine comprenant un colorant fluorescent ; et à
(iv) détecter la présence du colorant à des positions discrètes de la surface du réseau,
l'expression du colorant sur la surface de réseau indiquant l'expression d'un biomarqueur du tableau A dans l'échantillon.

12. Procédé selon l'une quelconque des revendications précédentes, dans lequel l'étape (b), (d) et/ou l'étape (f) consiste en la mesure de l'expression d'une molécule d'acide nucléique codant pour l'au moins un biomarqueur.

13. Procédé selon l'une quelconque des revendications précédentes, dans lequel l'échantillon fourni à l'étape (b) est choisi dans le groupe constitué par du sang non fractionné, du plasma, du sérum, un fluide tissulaire, le tissu pancréatique, le suc pancréatique, la bile et l'urine.

14. Procédé selon l'une quelconque des revendications précédentes, dans lequel la précision prédictive du procédé, telle que déterminée par une valeur ROC AUC, est d'au moins 0,70.

15. Réseau permettant de localiser une tumeur dans le pancréas d'un individu atteint d'un cancer du pancréas, le réseau comprenant des anticorps ou leurs fragments de liaison d'antigène capables de se lier à au moins deux biomarqueurs choisis dans le groupe défini dans le tableau A(i), (ii) ou (iii),
les au moins deux biomarqueurs sélectionnés dans le groupe défini dans le tableau A(i), (ii) ou (iii) comprenant la protéine en doigt de zinc 14 du domaine PR (PRD14) et l'homologue de la protéine du kinétochore NDC80 (HsHec1).
